# EUROPEAN PATENT SPECIFICATION

(11) **EP 3 353 214 B1**
(45) Date of publication and mention of the grant of the patent: **18.08.2021**
(21) Application number: 16775537.0
(22) Date of filing: 21.09.2016
(51) Int. Cl.: C07K 16/40, A61K 39/395, A61P 25/28, G01N 33/53

(54) **AGENTS INHIBITING KALLIKREIN-8 FOR USE IN THE PREVENTION OR TREATMENT OF ALZHEIMER'S DISEASE**
WIRKSTOFFE ZUR HEMMUNG VON KALLIKREIN-8 ZUR VERWENDUNG BEI DER VORBEUGUNG UND BEHANDLUNG VON MORBUS ALZHEIMER
AGENTS D'INHIBITION KALLIKREIN-8 POUR UTILISATION DANS LA PRÉVENTION OU LE TRAITEMENT DE LA MALADIE D'ALZHEIMER

(30) Priority: 25.09.2015 EP 15002769; 22.12.2015 EP 15003657
(43) Date of publication of application: 01.08.2018
(73) Proprietor: Universität Duisburg-Essen, 45141 Essen (DE)
(72) Inventor: HERRING, Arne, 48147 Münster (DE); KEYVANI, Kathy, 45134 Essen (DE)
(74) Representative: Lahrtz, Fritz
(86) International application number: PCT/EP2016/072381
(87) International publication number: WO 2017/050803

(56) References cited:
- WO-A1-2009/097141
- WO-A2-2005/022164
- US-A1- 2005 106 586
- MOMOTA YOSHIHARU ET AL: "Blockade of neuropsin, a serine protease, ameliorates kindling epilepsy", EUROPEAN JOURNAL OF NEUROSCIENCE, vol. 10, no. 2, February 1998 (1998-02), pages 760-764, XP002755848, ISSN: 0953-816X
- SHIMIZU-OKABE C ET AL: "EXPRESSION OF THE KALLIKREIN GENE FAMILY IN NORMAL AND ALZHEIMER'S DISEASE BRAIN", NEUROREPORT, LIPPINCOTT WILLIAMS & WILKINS, UK, vol. 12, no. 12, 28 August 2001 (2001-08-28), pages 2747-2751, XP008040774, ISSN: 0959-4965, DOI: 10.1097/00001756-200108280-00031 cited in the application

## Description

The present invention relates to an antibody or functionally active part thereof comprising at least one antigen binding fragment of the antibody which specifically binds to Kallikrein-8 and inhibits the proteolytic activity of Kallikrein-8 for use in the treatment or prevention of Alzheimer's disease (AD) as well as to compounds for use and uses relating thereto.

Kallikrein-8 (KLK8, also known as neuropsin) is a synaptic plasticity-modulating extracellular serine protease with trypsin-related specificity (Tamura, H., Kawata, M., Hamaguchi, S., Ishikawa, Y., and Shiosaka, S. 2012. Processing of neuregulin-1 by neuropsin regulates GABAergic neuron to control neural plasticity of the mouse hippocampus. J Neurosci 32:12657-12672). In the amygdala, KLK8 cleaves the ephrin receptor B2 (EPHB2), inducing the expression of FK506 binding protein-5 (FKBP5), which in turn regulates glucocorticoid receptor sensitivity and provokes anxiety (Attwood, B.K., Bourgognon, J.M., Patel, S., Mucha, M., Schiavon, E., Skrzypiec, A.E., Young, K.W., Shiosaka, S., Korostynski, M., Piechota, M., et al. 2011. Neuropsin cleaves EphB2 in the amygdala to control anxiety. Nature 473:372-375), whereas FKBP5 inhibition has anxiolytic effects (Hartmann, J., Wagner, K.V., Gaali, S., Kirschner, A., Kozany, C., Ruhter, G., Dedic, N., Hausl, A.S., Hoeijmakers, L., Westerholz, S., et al. 2015. Pharmacological Inhibition of the Psychiatric Risk Factor FKBP51 Has Anxiolytic Properties. J Neurosci 35:9007-9016). EPHB2 signalling via trans-cellular communication with ephrin ligands (EFNs) is of particular relevance for neuronal plasticity, as it coordinates axonal guidance (Srivastava, N., Robichaux, M.A., Chenaux, G., Henkemeyer, M., and Cowan, C.W. 2013. EphB2 receptor forward signaling controls cortical growth cone collapse via Nck and Pak. Mol Cell Neurosci 52:106-116) and controls synaptogenesis (Kayser, M.S., Nolt, M.J., and Dalva, M.B. 2008. EphB receptors couple dendritic filopodia motility to synapse formation. Neuron 59:56-69). Additionally, EPHB2 is also known to induce angiogenesis (Adams, R.H., Wilkinson, G.A., Weiss, C., Diella, F., Gale, N.W., Deutsch, U., Risau, W., and Klein, R. 1999. Roles of ephrinB ligands and EphB receptors in cardiovascular development: demarcation of arterial/venous domains, vascular morphogenesis, and sprouting angiogenesis. Genes Dev 13:295-306) and autophagy (Chukkapalli, S., Amessou, M., Dilly, A.K., Dekhil, H., Zhao, J., Liu, Q., Bejna, A., Thomas, R.D., Bandyopadhyay, S., Bismar, T.A., et al. 2014. Role of the EphB2 receptor in autophagy, apoptosis and invasion in human breast cancer cells. Exp Cell Res 320:233-246; Kandouz, M., Haidara, K., Zhao, J., Brisson, M.L., and Batist, G. 2010. The EphB2 tumor suppressor induces autophagic cell death via concomitant activation of the ERK1/2 and PI3K pathways. Cell Cycle 9:398-407) under neoplastic conditions.

All the aforementioned behavioural, molecular and structural processes, i.e. anxiety and cognition, neuronal (D'Amelio, M., and Rossini, P.M. 2012. Brain excitability and connectivity of neuronal assemblies in Alzheimer's disease: from animal models to human findings. Prog Neurobiol 99:42-60; Ruan, L., Lau, B.W., Wang, J., Huang, L., Zhuge, Q., Wang, B., Jin, K., and So, K.F. 2014. Neurogenesis in neurological and psychiatric diseases and brain injury: from bench to bedside. Prog Neurobiol 115:116-137) and vascular (Zlokovic, B.V. 2011. Neurovascular pathways to neurodegeneration in Alzheimer's disease and other disorders. Nat Rev Neurosci 12:723-738; Paris, D., Ganey, N., Banasiak, M., Laporte, V., Patel, N., Mullan, M., Murphy, S.F., Yee, G.T., Bachmeier, C., Ganey, C., et al. 2010. Impaired orthotopic glioma growth and vascularization in transgenic mouse models of Alzheimer's disease. J Neurosci 30:11251-11258) plasticity, as well as autophagy (Ghavami, S., Shojaei, S., Yeganeh, B., Ande, S.R., Jangamreddy, J.R., Mehrpour, M., Christoffersson, J., Chaabane, W., Moghadam, A.R., Kashani, H.H., et al. 2014. Autophagy and apoptosis dysfunction in neurodegenerative disorders. Prog Neurobiol 112:24-49; D.S., Stavrides, P., Mohan, P.S., Kaushik, S., Kumar, A., Ohno, M., Schmidt, S.D., Wesson, D., Bandyopadhyay, U., Jiang, Y., et al. 2011. Reversal of autophagy dysfunction in the TgCRND8 mouse model of Alzheimer's disease ameliorates amyloid pathologies and memory deficits. Brain 134:258-277) are also pathologically altered in AD patients and transgenic mice with AD-like pathology. We, therefore hypothesized that KLK8/EPHB2 signalling could also be involved in the pathogenesis of AD, although there is no proven evidence for this hypothesis in the literature. Up-regulation of KLK8 mRNA (Shimizu-Okabe, C., Yousef, G.M., Diamandis, E.P., Yoshida, S., Shiosaka, S., and Fahnestock, M. 2001. Expression of the kallikrein gene family in normal and Alzheimer's disease brain. Neuroreport 12:2747-2751) in AD-affected human hippocampus has been reported once in patients and down-regulation of EPHB2 protein in human and murine hippocampus has been also published a few times (Qu, M., Jiang, J., Liu, X.P., Tian, Q., Chen, L.M., Yin, G., Liu, D., Wang, J.Z., and Zhu, L.Q. 2013. Reduction and the intracellular translocation of EphB2 in Tg2576 mice and the effects of beta-amyloid. Neuropathol Appl Neurobiol 39:612-622; Simon, A.M., de Maturana, R.L., Ricobaraza, A., Escribano, L., Schiapparelli, L., Cuadrado-Tejedor, M., Perez-Mediavilla, A., Avila, J., Del Rio, J., and Frechilla, D. 2009. Early changes in hippocampal Eph receptors precede the onset of memory decline in mouse models of Alzheimer's disease. J Alzheimers Dis 17:773-786). There exist also a single publication showing that Lentivirus-mediated EPHB2 up-regulation improves cognition (Cisse, M., Halabisky, B., Harris, J., Devidze, N., Dubal, D.B., Sun, B., Orr, A., Lotz, G., Kim, D.H., Hamto, P., et al. 2011. Reversing EphB2 depletion rescues cognitive functions in Alzheimer model. Nature 469:47-52) and ligand-triggered EPHB2 activation diminishes tau phosphorylation (Jiang, J., Wang, Z.H., Qu, M., Gao, D., Liu, X.P., Zhu, L.Q., and Wang, J.Z. 2015. Stimulation of EphB2 attenuates tau phosphorylation through Pl3K/Akt-mediated inactivation of glycogen synthase kinase-3beta. Sci Rep 5:11765) in transgenic mice. Further, WO 2005/022164 A2 speculates about a role of KLK8 in a huge variety of diseases.

Accordingly, only fragmentary, inconclusive and/or merely descriptive gene expression results are present in this field.

Until now there is no cure for Alzheimer's disease and no reliable pre-mortem diagnostic assays or biomarkers with satisfying sensitivity and/or specificity for the diagnosis of AD or its precursor stages.

We demonstrate here an AD-related KLK8 increase and EPHB2 depletion in both murine and human hippocampus. We show a drastic rise in KLK8 mRNA and protein levels in different brain regions, surprisingly long before any "clinical" signs of disease appear and even prior to Aβ pathology onset. Of outmost significance for this invention, we now show for the first time that four weeks of KLK8 inhibition, by intraventricular delivery of an anti-KLK8 antibody after disease onset, is sufficient to mitigate multiple features of Alzheimer's pathology in transgenic mice. Yet, a compound which inhibits Kallikrein-8 has a great and hitherto unknown potential to prevent or treat Alzheimer's disease and/or its precursor stages, such as mild cognitive impairment (MCI). Moreover, our results indicate for the first time that KLK-8 as such could be used in the diagnosis, prediction and stratification of patients in Alzheimer's disease, including its precursor stages.

Accordingly, in one embodiment, the present invention relates to an antibody or functionally active part thereof comprising at least one antigen binding fragment of the antibody which specifically binds to Kallikrein-8 and inhibits the proteolytic activity of Kallikrein-8 for use in the treatment or prevention of Alzheimer's disease.

Disclosed is an agent, which inhibits Kallikrein-8 for use in the treatment or prevention of Alzheimer's disease, and/or in the treatment or prevention of precursor stages of Alzheimer's disease.

"Kallikrein 8" or "KLK8" or "neuropsin" is a protein which is a synaptic plasticity-modulating extracellular serine protease with trypsin-related specificity. Kallikrein 8 protein according to the invention is preferably a mammalian protein, such as a murine, rat, dog, sheep, monkey, horse or human Kallikrein 8 protein, more preferably a human Kallikrein 8 protein. The sequence of the enzymatically inactive pre-pro-form of the human Kallikrein 8 protein is shown as SEQ ID No: 3. The sequence of the enzymatically inactive pre-pro-form of the murine Kallikrein 8 protein is shown as SEQ ID No: 2. For diagnostic purposes of the present invention, the detected Kallikrein 8 protein may be the proteolytically active form of Kallikrein 8 and/or the enzymatically inactive pre-pro and/or pro-form of Kallikrein 8. For the therapeutic, in particular prevention or treatment purposes, the agent which inhibits Kallikrein-8 is preferably an agent which (1) specifically binds to Kallikrein-8 and inhibits the proteolytic activity of Kallikrein-8, or an agent which (2) is capable of reducing the level of Kallikrein-8 in the brain of a patient. In case of (1), Kallikrein 8 is preferably the proteolytically active form of Kallikrein 8. In case of (2), it may be the proteolytically active form of Kallikrein 8 and/or the enzymatically inactive pre-pro and/or pro-form of Kallikrein 8. The pro-form without signal peptide starts of Gly24 of human KLK8 pursuant to SEQ ID No: 3. The mature, proteolytically active form of KLK8 starts of Val 33 of human KLK8 pursuant to SEQ ID No: 3. Preferably, the substrate motif recognized by proteolytically active Kallikrein 8 is YGRY (SEQ ID No: 1).

The agent for use is an agent which inhibits Kallikrein-8. Such agent may be of any chemical nature and is capable of inhibiting Kallikrein-8.

In one disclosure, the agent is capable of reducing the level of Kallikrein 8 in the brain of a human, in particular a patient, more preferably an AD patient or a patient having a precursor stage of AD, such as MCI. Such reduction may be achieved by mediating reduced level of transcription, reduced level of translation and/or enhanced level of protein degradation.

The antibody or functionally active part thereof as agent for use of the invention inhibits the proteolytic activity of Kallikrein-8. Methods for determining proteolytic activity in vitro, in particular proteolytic activity for at least one substrate of KLK8, more preferably wherein the substrate is selected from EPHB2, steroid 5 alpha-reductase 1, casein, fibronectin, collagen type IV, fibrinogen, kininogen, neuregulin-1, CAM-L1, single-chain tPA, PAR2, pro-KLK1 and pro-KLK11, can be determined by methods known in the art and as described in the Examples for EPHB2. In a more preferred embodiment, the proteolytic activity of Kallikrein-8 is inhibited by the agent in vitro by at least 50%, 90%, 91%, 92%, 93%, 94%, 95%, 96%, 97%, 98% or 99%. In a preferred embodiment, the substrate is EPHB2. In a further preferred embodiment, the agent inhibits one or more proteases in addition to Kallikrein-8, or the agent specifically inhibit the proteolytic activity of Kallikrein-8. An agent is understood to specifically inhibit the proteolytic activity of Kallikrein-8 if the proteolytic activity of proteases other than Kallikrein-8 is inhibited by the agent less than 50%, 20%, 10%, 5% or 1% as compared to the inhibition of proteolytic activity of Kallikrein-8 by the agent. In a further more preferred embodiment, the agent specifically binds to Kallikrein-8. An agent is understood to specifically bind to Kallikrein-8 if the affinity to other proteins, including a Kallikrein other than Kallikrein-8 is less than 50%, 20%, 10%, 5% or 1% as compared to the affinity to Kallikrein-8. Affinity may be determined by methods known to the skilled person such as surface plasmon resonance spectroscopy methods. An agent which specifically binds to Kallikrein-8 and inhibits the proteolytic activity of Kallikrein-8 is expected to specifically inhibit the proteolytic activity of Kallikrein-8.

The agent for use according to the invention specifically binds to Kallikrein-8 and inhibits the proteolytic activity of Kallikrein-8.

"Alzheimer's disease" or "AD" is a chronic neurodegenerative disease of the central nervous system associated with progressive memory loss resulting in dementia. Two pathological characteristics are observed in AD patients at autopsy: extracellular plaques and intracellular tangles in the hippocampus, cerebral cortex, and other areas of the brain essential for cognitive function. Plaques are formed mostly from the deposition of amyloid beta ("Aβ"), a peptide derived from amyloid precursor protein ("APP"). Filamentous tangles are formed from paired helical filaments composed of neurofilament and hyperphosphorylated tau protein, a microtubule-associated protein. There are different stages of AD. Preferably, clinical AD stages are typically differentiated by persons skilled in the art pursuant to the CDR classification or the FAST classification, more preferably pursuant to the CDR classification.

The CDR classification (Clinical Dementia Rating) is described in http://madrc.mgh.harvard.edu/clinical-dementia-rating-cdr-scale. Following stages are differentiated according to the CDR classification:
0 = Normal
0.5 = Very Mild Dementia, corresponding to MCI
1 = Mild Dementia, corresponding to mild AD
2 = Moderate Dementia, corresponding to moderate AD
3 = Severe Dementia, corresponding to severe AD.

The FAST (Functional Assessment Staging Test) is described in http://www.mccare.com/pdf/fast.pdf. In this classification, 7 stages are differentiated. Stage 3 relates to MCI. Stages 4 to 7 relate to AD of increasing severity.

An individual who is in a "pre-clinical stage of Alzheimer's disease" is understood to belong to a high risk group for developing Alzheimer's disease in the future. In particular, Alzheimer's disease occurred one or more times in the family of such individual and/or the individual is known to have at least one mutation which is known to be associated with Alzheimer's disease, such as at least one mutation in the presenilin-1 or presenilin-2 gene or the APP gene known to be associated with Alzheimer's disease. In the pre-clinical stage of Alzheimer's disease, an individual does not show clinical symptoms of Alzheimer's disease.

The main clinical feature and symptom of AD is a progressive cognitive decline, and accordingly, cognitive impairment, such as memory loss. Symptoms of Alzheimer's disease include cognitive impairment, including language impairment, deficits in visual function, memory loss, and impairment of short-term memory, increased anxiety, sleeping disorder, personality changes, such as progressive passivity or marked agitation, decreased expressions of affection, depression and psychosis. The memory dysfunction involves impairment of learning new information which is often characterized as short-term memory loss. In the early and moderate stages of the illness, recall of remote well-learned material may appear to be preserved, but new information cannot be adequately incorporated into memory. Disorientation to time is closely related to memory disturbance. Language impairments are also a prominent symptom of AD. These are often manifest first as word finding difficulty in spontaneous speech. The language of the AD patient is often vague, lacking in specifics and may have increased automatic phrases and cliches. Difficulty in naming everyday objects is often prominent. Complex deficits in visual function are present in many AD patients, as are other focal cognitive deficits such as apraxia, acalculia and left-right disorientation. Impairments of judgment and problems solving are frequently seen. Non-cognitive or behavioral symptoms are also common in AD and may account for an event larger proportion of caregiver burden or stress than the cognitive dysfunction. Personality changes are commonly reported and range from progressive passivity to marked agitation. Patients may exhibit changes such as decreased expressions of affection. Depressive symptoms are present in up to 40%. A similar rate for anxiety has also been recognized. Psychosis occurs in 25%. In some cases, personality changes may predate cognitive abnormality.

Accordingly, a "symptom of Alzheimer's disease" is understood to include cognitive impairment, including language impairment, deficits in visual function, memory loss, and impairment of short-term memory, increased anxiety, sleeping disorder, personality changes, such as progressive passivity or marked agitation, decreased expressions of affection, depression and psychosis. A symptom of a precursor of Alzheimer's disease is mild cognitive impairment (MCI) and optionally increased anxiety. "Mild cognitive impairment" or "MCI" is understood by a skilled person as grey area between intact cognitive functioning and clinical dementia as defined in the "Diagnostic and Statistical Manual for Mental Disorders" (DSM), in particular as defined in version DSM-5.

About 70% of patients exhibiting mild cognitive impairment (MCI) later develop Alzheimer's disease. The remaining patients develop different forms of dementia, such as vascular dementia.

In the examples, an anti-KLK-8 antibody was successfully used in vivo in a murine animal model with AD-related pathology for mitigating symptoms of AD, which specifically binds to Kallikrein-8 and inhibits the proteolytic activity of Kallikrein-8. Accordingly, agents which specifically bind to Kallikrein-8 and inhibit the proteolytic activity of Kallikrein-8, are particularly suitable for treating and preventing AD or precursor stages thereof.

Therefore, the agent for use according to the invention specifically binds to Kallikrein-8 and inhibits the proteolytic activity of Kallikrein-8.

In a further disclosure, the agent for use is capable of reducing the level of Kallikrein-8 in the brain of a patient.

It is disclosed that the agent for use specifically binds to Kallikrein-8 and inhibits the proteolytic activity of Kallikrein-8, wherein the agent is a Kallikrein-8 enzyme inhibitor, preferably selected from a small molecule, a ribozyme, a peptide and a protein.

A Kallikrein-8 enzyme inhibitor may inhibit enzymatic, in particular proteolytic activity by binding to the active site, or a site different from the active site. In the latter case, inhibition may occur by e.g. changing the 3-dimensional structure of KLK8 and/or by steric hindrance.

Various classes of agents are known to be suitable to act as protease inhibitors, such as small molecules, in particular molecules having a molecular weight of between 50 Da and 1000 Da or 100 Da and 500 Da, a ribozyme, a peptide and a protein. For example, suitable inhibitory peptides may be derived from and/or encompass the substrate motif pursuant to SEQ ID No. 1, e.g. such inhibitor may be a non-cleavable substrate derivative, which may be a peptide or protein.

A Kallikrein-8 enzyme inhibitor may be endogenous or exogenous.

Known endogenous, natural KLK8 inhibitors are for example serine proteinase inhibitor-3 (SPI3), murinoglobulin I (MUG I), phosphatidylethanolamine-binding protein (PEBP), a2-antiplasmin, protein C inhibitor, proteinase inhibitor 6 (PI6) and Zn²⁺ ions. These endogenous KLK8 inhibitors block several Kallikrein proteins.

Known exogenous, non-natural KLK8 inhibitors are Leupeptin/antipain, Chymostatin, TLCK/PPACK (tosyl-lysyl chloromethyl ketone/D-phenylalanyl-L-prolyl-L-arginyl chloromethyl ketone. These exogenous KLK8 inhibitors block several Kallikrein proteins.

An overview of Kallikrein inhibitors in given in Goettig P. et al. (2010, Biochimie, 92(11): 1546-1567).

In a further disclosure, the agent for use which inhibits Kallikrein 8, preferably the agent for use which specifically binds to Kallikrein-8 and inhibits the proteolytic activity of Kallikrein-8 is a protein, more preferably an antibody or functionally active part thereof or an antibody mimetic. The agent for use of the invention is an antibody or functionally active part thereof comprising at least one antigen binding fragment of the antibody which specifically binds to Kallikrein-8 and inhibits the proteolytic activity of Kallikrein-8. Preferably the agent is selected from a monoclonal antibody, chimeric antibody, human antibody, humanized antibody, Fab, a Fab', a F(ab')2, a Fv, a disulfide-linked Fv, a scFv, a (scFv)2, a bivalent antibody, a bispecific antibody, a multispecific antibody, a diabody, a triabody, a tetrabody and a minibody. It is disclosed that the agent is monoclonal antibody MabB5 or functionally active part thereof, or an agent, in particular selected from an antibody or functionally active part thereof and an antibody mimetic, binding to the same epitope as MabB5.

Methods for determining epitopes are known in the art and comprise e.g. epitope mapping e.g. using protein microarrays, and with the ELISPOT or ELISA techniques. Epitopes of proteins typically comprise several amino acids, in case of linear epitopes typically a stretch of 5 to 15 amino acids.

In the example, Mab5, which is well known in the prior art and is described in the examples, was surprisingly successfully used for mitigating Alzheimer's disease symptoms in an established *in vivo* mouse model for Alzheimer's disease. Mab 5 is for example described in Momota, Y., Yoshida, S., Ito, J., Shibata, M., Kato, K., Sakurai, K., Matsumoto, K., and Shiosaka, S. (1998, Blockade of neuropsin, a serine protease, ameliorates kindling epilepsy. Eur J Neurosci 10:760-764).

As an antibody or functionally active part thereof typically exhibit high specificity for a given target, such agents represent particularly preferred agent for use according to the invention. Further, it could be shown in the examples that a monoclonal antibody was transported efficiently to the brain to mitigate the AD symptoms anxiety and cognitive impairment in the AD mouse model.

Naturally occurring antibodies are globular plasma proteins (-150 kDa (http://en.wikipedia.org/wiki/Dalton_unit)) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM. In the present invention, examples of suitable formats include the format of naturally occurring antibodies including antibody isotypes known as IgA, IgD, IgE, IgG and IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two beta sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals. Other types of light chains, such as the i chain, are found in lower vertebrates like Chondrichthyes and Teleostei.

In addition to naturally occurring antibodies, artificial antibody formats including antibody fragments have been developed. Some of them are described in the following.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

Accordingly, the term "antibody", as used herein, means any polypeptide which has structural similarity to a naturally occurring antibody and is capable of specific binding to the respective target, wherein the binding specificity is determined by the CDRs. Hence, "antibody" is intended to relate to an immunoglobulin-derived structure with binding to the respective target including, but not limited to, a full length or whole antibody, an antigen binding fragment (a fragment derived, physically or conceptually, from an antibody structure), a derivative of any of the foregoing, a chimeric molecule, a fusion of any of the foregoing with another polypeptide, or any alternative structure/composition which selectively binds to the respective target. The antibody or functionally active parts thereof may be any polypeptide which comprises at least one antigen binding fragment. Antigen binding fragments consist of at least the variable domain of the heavy chain and the variable domain of the light chain, arranged in a manner that both domains together are able to bind to the specific antigen. The "respective target" is the Kallikrein 8 protein.

"Full length" or "complete" antibodies refer to proteins that comprise two heavy (H) and two light (L) chains inter-connected by disulfide bonds which comprise: (1) in terms of the heavy chains, a variable region and a heavy chain constant region which comprises three domains, CH1, CH2 and CH3; and (2) in terms of the light chains, a light chain variable region and a light chain constant region which comprises one domain, CL. With regard to the term "complete antibody", any antibody is meant that has a typical overall domain structure of a naturally occurring antibody (i.e. comprising a heavy chain of three or four constant domains and a light chain of one constant domain as well as the respective variable domains), even though each domain may comprise further modifications, such as mutations, deletions, or insertions, which do not change the overall domain structure.

"Functionally active parts of antibodies" or "antibody fragments" also contain at least one antigen binding fragment as defined above, and exhibit essentially the same function and binding specificity as the complete antibody of which the functionally active part (or fragment) is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

As the first generation of full sized antibodies presented some problems, many of the second generation antibodies comprise only fragments of the antibody. Variable domains (Fvs) are the smallest fragments with an intact antigen-binding domain consisting of one VL and one VH. Such fragments, with only the binding domains, can be generated by enzymatic approaches or expression of the relevant gene fragments, e.g. in bacterial and eukaryotic cells. Different approaches can be used, e.g. either the Fv fragment alone or 'Fab'-fragments comprising one of the upper arms of the "Y" that includes the Fv plus the first constant domains. These fragments are usually stabilized by introducing a polypeptide link between the two chains which results in the production of a single chain Fv (scFv). Alternatively, disulfide-linked Fv (dsFv) fragments may be used. The binding domains of fragments can be combined with any constant domain in order to produce full length antibodies or can be fused with other proteins and polypeptides.

A recombinant antibody fragment is the single-chain Fv (scFv) fragment, which is a preferred functionally active part of an antibody for use according to the invention. In general, it has a high affinity for its antigen and can be expressed in a variety of hosts. These and other properties make scFv fragments not only applicable in medicine, but also of potential for biotechnological applications. As detailed above, in the scFv fragment the VH and VL domains are joined with a hydrophilic and flexible peptide linker, which improves expression and folding efficiency. Usually linkers of about 15 amino acids are used, of which the (Gly4Ser)3 linker has been used most frequently. scFv molecules might be easily proteolytically degraded, depending on the linker used. With the development of genetic engineering techniques these limitations could be practically overcome by research focussed on improvement of function and stability. An example is the generation of disulfide-stabilized (or disulfide-linked) Fv fragments where the VH-VL dimer is stabilized by an interchain disulfide bond. Cysteines are introduced at the interface between the VL and VH domains, forming a disulfide bridge, which holds the two domains together.

Dissociation of scFvs results in monomeric scFvs, which can be complexed into dimers (diabodies), trimers (triabodies) or larger aggregates such as TandAbs and Flexibodies, which also represent functionally active parts of an antibody for use according to the invention.

Antibodies with two binding domains can be created either through the binding of two scFv with a simple polypeptide link (scFv)2 or through the dimerization of two monomers (diabodies). The simplest designs are diabodies that have two functional antigen-binding domains that can be either the same, similar (bivalent diabodies) or have specificity for distinct antigens (bispecific diabodies).

Also, antibody formats comprising four variable domains of heavy chains and four variable domains of light chains have been developed. Examples of these include tetravalent bispecific antibodies (TandAbs and Flexibodies, Affimed Therapeutics AG, Heidelberg. Germany). In contrast to a bispecific diabody, a bispecific TandAb is a homodimer consisting of only one polypeptide. Because of the two different chains, a diabody can build three different dimers of which only one is functional. Therefore, it is simpler and cheaper to produce and purify this homogeneous product. Moreover, the TandAb usually shows better binding properties (possessing twice the number of binding sites) and increased stability in vivo. Flexibodies are a combination of scFv with a diabody multimer motif resulting in a multivalent molecule with a high degree of flexibility for joining two molecules which are quite distant from each other on the cell surface. If more than two functional antigen-binding domains are present and if they have specificity for distinct antigens, the antibody is multispecific.

In summary, specific immunoglobulin types which represent antibodies or functionally active parts thereof include but are not limited to the following antibody: a Fab (monovalent fragment with variable light (VL), variable heavy (VH), constant light (CL) and constant heavy 1 (CHI) domains), a F(ab')2 (bivalent fragment comprising two Fab fragments linked by a disulfide bridge or alternative at the hinge region), a Fv (VL and VH domains), a scFv (a single chain Fv where VL and VH are joined by a linker, e.g., a peptide linker), a bispecific antibody molecule (an antibody molecule with specificity as described herein linked to a second functional moiety having a different binding specificity than the antibody, including, without limitation, another peptide or protein such as an antibody, or receptor ligand), a bispecific single chain Fv dimer, a diabody, a triabody, a tetrabody, a minibody (a scFv joined to a CH3).

Certain antibody molecules or functionally active parts thereof including, but not limited to, Fv, scFv, diabody molecules or domain antibodies (Domantis) may be stabilized by incorporating disulfide bridges to line the VH and VL domains. Bispecific antibodies may be produced using conventional technologies, specific methods of which include production chemically, or from hybrid hybridomas) and other technologies including, but not limited to, the BiTETM technology (molecules possessing antigen binding regions of different specificity with a peptide linker) and knobs-into-holes engineering.

Accordingly, an antibody molecule or functionally active part thereof may be a Fab, a Fab', a F(ab')2, a Fv, a disulfide-linked Fv, a scFv, a (scFv)2, a bivalent antibody, a bispecific antibody, a multispecific antibody, a diabody, a triabody, a tetrabody or a minibody.

In another preferred embodiment, the antibody is a monoclonal antibody, a chimeric antibody or a humanised antibody. Monoclonal antibodies are monospecific antibodies that are identical because they are produced by one type of immune cell that are all clones of a single parent cell. A chimeric antibody is an antibody in which at least one region of an immunoglobulin of one species is fused to another region of an immunoglobulin of another species by genetic engineering in order to reduce its immunogenicity. For example murine VL and VH regions may be fused to the remaining part of a human immunoglobulin. A particular type of chimeric antibodies are humanised antibodies. Humanised antibodies are produced by merging the DNA that encodes the CDRs of a non-human antibody with human antibody-producing DNA. The resulting DNA construct can then be used to express and produce antibodies that are usually not as immunogenic as the non-human parenteral antibody or as a chimeric antibody, since merely the CDRs are non-human.

As detailed above in the context with the antibody for use of the present invention, each heavy chain of a naturally occurring antibody has two regions, the constant region and the variable region. There are five types of mammalian immunoglobulin heavy chain: γ, δ, α, µ and ε, which define classes of immunoglobulins IgM, IgD, IgG, IgA and IgE, respectively.

There are here four IgG subclasses (IgG1, 2, 3 and 4) in humans, named in order of their abundance in serum (IgG1 being the most abundant). Even though there is about 95 % similarity between their Fc regions of the IgG subclasses, the structure of the hinge regions are relatively different. This region, between the Fab arms (Fragment antigen binding) and the two carboxy-terminal domains CH2 and CH3 of both heavy chains, determines the flexibility of the molecule. The upper hinge (towards the amino-terminal) segment allows variability of the angle between the Fab arms (Fab-Fab flexibility) as well as rotational flexibility of each individual Fab. The flexibility of the lower hinge region (towards the carboxy-terminal) directly determines the position of the Fab-arms relative to the Fc region (Fab-Fc flexibility). Hinge-dependent Fab-Fab and Fab-Fc flexibility may be important in triggering further effector functions such as complement activation and Fc receptor binding. Accordingly, the structure of the hinge regions gives each of the four IgG classes their unique biological profile.

The length and flexibility of the hinge region varies among the IgG subclasses. The hinge region of IgG1 encompasses amino acids 216-231 and since it is freely flexible, the Fab fragments can rotate about their axes of symmetry and move within a sphere centered at the first of two inter-heavy chain disulfide bridges. IgG2 has a shorter hinge than IgG1, with 12 amino acid residues and four disulfide bridges. The hinge region of IgG2 lacks a glycine residue, it is relatively short and contains a rigid poly-proline double helix, stabilised by extra inter-heavy chain disulfide bridges. These properties restrict the flexibility of the IgG2 molecule. IgG3 differs from the other subclasses by its unique extended hinge region (about four times as long as the IgG1 hinge), containing 62 amino acids (including 21 prolines and 11 cysteines), forming an inflexible poly-proline double helix. In IgG3 the Fab fragments are relatively far away from the Fc fragment, giving the molecule a greater flexibility. The elongated hinge in IgG3 is also responsible for its higher molecular weight compared to the other subclasses. The hinge region of IgG4 is shorter than that of IgG1 and its flexibility is intermediate between that of IgG1 and IgG2.

As mentioned above, it is possible to reduce the amount of Kallikrein-8 in the brain of a patient. "Reducing the amount of Kallikrein-8 in the brain of a patient" is understood as that the amount of Kallikrein-8 in at least one region of the brain is reduced. As further explained above, the reduction in amount or level of Kallikrein 8 in vivo in the brain of a patient may be achieved by reducing mRNA transcription, translation of the mRNA or by enhanced degradation of the protein in vivo, e.g. by proteolytic degradation. Agents known in the art which are suitable for reducing the amount of Kallikrein 8 are for example antisense oligonucleotides and interfering oligonucleotides. Further, natural or artificially engineered proteases may be used, which are capable of cleaving Kallikrein 8.

Accordingly, it is disclosed that the agent for use reduces the expression rate of Kallikrein-8, preferably wherein said agent knocks down the Kallikrein-8 expression, in particular wherein knocking down is reducing the transcription rate of the Kallikrein-8 gene, reducing the translation rate of the Kallikrein-8 messenger ribonucleic acid (mRNA), and/or reducing the transcript level of Kallikrein-8. In one disclosure, the agent for use is an interfering oligonucleotide.

According to a disclosure, the agent for use is an oligonucleotide or an oligonucleotide analogue, in particular an oligonucleotide or an oligonucleotide analogue selected from the group consisting of:
(a) an antisense oligonucleotide, in particular an antisense deoxyribonucleic acid (asDNA), an antisense ribonucleic acid (asRNA);
(b) an antisense oligonucleotide analogue, in particular an antisense 2'-O-methoxyethyl (2'MOE) oligonucleotide, an antisense morpholino, an antisense peptide nucleic acid (PNA), an antisense glycol nucleic acid (GNA), an antisense locked nucleic acid (LNA) or an antisense threose nucleic acid (TNA);
(c) an interfering oligonucleotide, more preferably small interfering ribonucleic acid (siRNA), short hairpin ribonucleic acid (shRNA) or micro ribonucleic acid (microRNA), in particular siRNA of from 18 to 24 bases in length;
(d) an oligonucleotide modifying the splicing of pre-mRNA, in particular wherein said oligonucleotide is single stranded deoxyribonucleic acid (ssDNA) or single stranded ribonucleic acid (ssRNA);
(e) an oligonucleotide analogue modifying the splicing of pre-mRNA, in particular wherein said oligonucleotide is a 2'MOE, morpholino, PNA, GNA, LNA or TNA; and
(f) an oligonucleotide encoding for one or more of the aforementioned (a)-(e), optionally wherein said oligonucleotide is embedded in a vector or virus in particular a self-complementary adeno associated viruses (scAAV).

As used in this context of the present invention, the term "antisense oligonucleotide" may be understood in the broadest sense as generally understood in the art. Therefore, an antisense oligonucleotide may be any single-stranded oligonucleotide complementary to the Kallikrein-8 mRNA and may, therefore also be designated as "Kallikrein-8 mRNA-interfering complementary oligonucleotides". It will be understood that an oligonucleotide may also comprise one or more modifications such as, e.g., one or more sulfur chemistry modification(s)/sulfatation (e.g. phosphorothioates), methylation, alkylation, oxidation, lipidation, phosphorylation, glycosylation, oxidation, reduction, deamidation and/or partial intramolecular cyclization. Particularly preferably, an oligonucleotide comprises one or more nucleotide analogues shown in detail below.

Further, additionally or alternatively, the oligonucleotide may optionally comprise one or more non-nucleotide moiety/moieties and/or one or more non-natural nucleotide moiety/moieties. In particular, the termini of the oligonucleotide may, optionally, be capped by any means known in the art, such as, e.g., by sulfatation, amidation, acetylation, methylation, acylation, by one or more non-nucleotide moiety/moieties and/or by one or more non-natural nucleotide moiety/moieties. Optionally, the oligonucleotide may also be conjugated to any one of biotin, heme, eicosanoid(s), steroid(s), peptide(s) and/or small molecule(s). Preferably, such modified forms of oligonucleotide are those more stable against degradation in comparison with unmodified oligonucleotides.

An antisense oligonucleotide may be introduced into at least one cell of a patient to inhibit translation of Kallikrein-8 by base pairing to the mRNA encoding it and physically/sterically obstructing the translation machinery regarding Kallikrein-8. Most typically, an antisense oligonucleotide may bind to the Kallikrein-8 polypeptide-encoding region. However, an antisense oligonucleotide may also be complementary to an untranslated region (UTR) of the Kallikrein-8 mRNA, in particular such located at the 3' end, or 5'UTR, in particular overlapping with the start codon (ATG) region. Typically but not necessarily, such region will be in a range of not more than 40 base pairs (bp), more preferably not more than 30 bp, even more preferably not more than 20 bp from the Kallikrein-8 polypeptide-encoding region. Particularly preferably, an antisense oligonucleotide in the sense of the present invention is antisense RNA (asRNA). In this context, it may be noted that the 3'UTR of Kallikrein-8 is comparably long and comprises several regulatory elements, which might be inhibited.

An antisense oligonucleotide analogue acts in a way comparable with the action of an antisense oligonucleotide as laid out above. The only difference is that an antisense oligonucleotide analogue may typically be more stable against metabolic degradation. Therefore, the bonds between the moieties of the oligomers (monomers), thus, the nucleotide moiety analogues, of the antisense oligonucleotide analogue will typically be cleaved slower than the bonds between the corresponding nucleotide moieties, of the corresponding antisense oligonucleotide. Further, the rate of backbone and/or base modifications (e.g., acetylation, glycosylation) may preferably be lower in the antisense oligonucleotide analogues.

An antisense 2'-O-methoxyethyl (2'MOE) oligonucleotide may be any oligonucleotide analogue comprising at least one 2'-O-methoxyethyl nucleotide analogues, preferably an oligonucleotide analogue wherein at least 10 % of the nucleotide moieties are 2'-O-methoxyethyl nucleotide analogues, more preferably an oligonucleotide analogue wherein at least 20 % of the nucleotide moieties are 2'-O-methoxyethyl nucleotide analogues, even more preferably an oligonucleotide analogue wherein at least 50 % of the nucleotide moieties are 2'-O-methoxyethyl nucleotide analogues, even more preferably an oligonucleotide analogue wherein at least 80 % of the nucleotide moieties are 2'-O-methoxyethyl nucleotide analogues, even more preferably an oligonucleotide analogue wherein at least 90 % of the nucleotide moieties are 2'-O-methoxyethyl nucleotide analogues, in particular even more preferably an oligonucleotide analogue wherein essentially all nucleotide moieties are 2'-O-methoxyethyl nucleotide analogues. This analogue nears an RNA-like structure.

A morpholino may also be designated as "phosphorodiamidate morpholino oligonucleotide" or "PMO" and may typically interact with a comparably small region of the complementary pre-mRNA or mRNA of from approximately 15 to approximately 30 bases in length. In a morpholino, the bases are bound to morpholine rings instead of ribose moieties in RNA and deoxyribose moieties in DNA, respectively. Accordingly, in a morpholino, the moieties are linked through phosphorodiamidate groups instead of phosphates.

As used throughout the present invention, the terms "peptide nucleic acid" and "PNA" may be understood in the broadest sense as any oligonucleotide analogue comprising repeating N-(2-aminoethyl)-glycine moieties linked by peptide bonds. Various purine and pyrimidine bases may be linked to the backbone by a methylene bridge (-CH₂-) and a carbonyl group (-(C=O)-).

As used throughout the present invention, the terms "glycol nucleic acid" and "GNA" may be understood in the broadest sense as any oligonucleotide analogue comprising 2,3-dihydroxypropylnucleoside analogues and repeating glycol moieties linked by phosphodiester bonds. Typically, in GNA, the Watson-Crick base pairing is comparably stable leading to comparably high melting temperatures of GNAs.

As used throughout the present invention, the terms "locked nucleic acid" and "LNA" may be understood in the broadest sense as any oligonucleotide analogue wherein the ribose moiety is modified with an extra bridge connecting the 2' oxygen and 4' carbon. LNA may also be designated as "inaccessible RNA". This bridge "locks" the ribose in the 3'-endo (North) conformation, which is often found in the A-form duplexes. Typically, the locked ribose conformation enhances base stacking and backbone pre-organization leading to an increased melting temperature.

As used throughout the present invention, the terms "threose nucleic acid" and "TNA" may be understood in the broadest sense as any oligonucleotide analogue comprising a backbone comprising repeating threose sugars linked together by phosphodiester bonds. TNA may form the helical geometry similar to A-form RNA.

In the above inhibitors, nucleotide moiety analogues may be conjugated to DNA and/or RNA moieties in a single molecule then comprising one or more nucleotide moiety/moieties and one or more DNA and/or RNA moiety/moieties whenever desired. Furthermore, additionally or alternatively, such molecule or the above oligonucleotide analogues may be hybridized with one or more DNA and/or RNA oligonucleotide(s) whenever desired.

As used in this context of the present invention, the term "interfering oligonucleotide" may be understood in the broadest sense as generally understood in the art. Accordingly, most typically, the interfering oligonucleotide may be a double-stranded oligonucleotide molecule of from approximately 20 bp to approximately 25 bp in length. Particularly preferably, an interfering oligonucleotide in the sense of the present invention is interfering RNA (iRNA), in particular small interfering RNA (siRNA) suitable for the well-known technology of RNA interference (RNAi), also known as "post-transcriptional gene silencing" (PTGS), specifically interfering with the expression of Kallikrein-8 encoded by a gene having a complementary nucleotide sequence. Such RNA may also be short shRNA and micro RNA. As used herein, the terms "small interfering RNA", "short interfering RNA" and "silencing RNA" may be understood interchangeably in the broadest sense as generally understood in the art. Accordingly, most typically, the siRNA may be a double-stranded RNA (dsRNA) molecule of from approximately 20 bp to approximately 25 bp in length.

As used herein, the term "precursor messenger RNA" (pre-mRNA) may be understood in the broadest sense as any immature single strand of messenger ribonucleic acid (mRNA). Typically, pre-mRNA is synthesized from a template of genomic DNA by transcription and comprises the bulk of heterogeneous nuclear RNA (hnRNA). Once pre-mRNA has been completely processed, it is typically designated as "mature messenger RNA" (mature mRNA).

The person skilled in the art will immediately know that pre-mRNA may be processed further by splicing. Such splicing processes are well-known in detail by any person skilled in the art. Therefore, "splicing" in the context of the present invention may be understood in the broadest sense as a process of modifying nascent pre-mRNA that may take place after or concurrently with its generation by transcription of the genomic DNA. By splicing, introns may be removed whereas the exons may preferably remain in the mature mRNA. In many cases this may be needed before the mRNA can be used to produce a correct polypeptide strand by mean of translation of the mRNA. For many eukaryotic introns, splicing is performed in a series of reactions typically catalyzed by the spliceosome, a complex of small nuclear ribonucleoproteins (snRNPs), but the person skilled in the art will also know self-splicing introns wherein splicing may typically be performed directly in the nucleus. Any splicing process may, in principle, be modified by oligonucleotides according to the present disclosure.

The oligonucleotide encoding for one or more of the aforementioned (a)-(e) may be any genetic material as known in the art and exemplified above.

The person skilled in the art will notice that, optionally,
(i) two or more antisense oligonucleotides;
(ii) more than one interfering oligonucleotide(s);
(iii) more than one interfering oligonucleotide analogue(s);
(iv) more than one oligonucleotides modifying pre-mRNA splicing; or
(v) more than one oligonucleotide analogues modifying pre-mRNA, may be combined with another.

Moreover, the person skilled in the art will also notice that, optionally, one or more antisense oligonucleotide(s) may be combined with:
(i) one or more antisense oligonucleotide analogue(s);
(ii) one or more antisense oligonucleotide(s); and/or
(iii) one or more oligonucleotide(s) modifying pre-mRNA splicing.

Moreover, the person skilled in the art will also notice that, optionally, one or more antisense oligonucleotide(s) may be combined with:
(i) one or more oligonucleotide(s) modifying pre-mRNA splicing; and/or
(ii) one or more oligonucleotide analogue(s) modifying pre-mRNA splicing.

Moreover, the person skilled in the art will also notice that, optionally, one or more interfering oligonucleotide(s) may be combined with one or more oligonucleotide analogue(s) modifying pre-mRNA splicing.

Moreover, also one or more antisense oligonucleotide(s) and one or more antisense oligonucleotide analogue(s) may be combined with:
(i) one or more oligonucleotide(s) modifying pre-mRNA-splicing; or
(ii) one or more oligonucleotide analogue(s) modifying pre-mRNA-splicing.

Moreover, also one or more interfering oligonucleotide(s), one or more antisense oligonucleotide(s) and one or more oligonucleotide(s) modifying pre-mRNA splicing may be combined with another. Moreover, also one or more interfering oligonucleotide(s), one or more antisense oligonucleotide analogues(s) and one or more oligonucleotide(s) modifying pre-mRNA splicing may be combined with another. Moreover, also one or more interfering oligonucleotide(s), one or more antisense oligonucleotide(s) and one or more oligonucleotide analogues(s) modifying pre-mRNA splicing may be combined with another. Moreover, also one or more interfering oligonucleotide(s), one or more antisense oligonucleotide analogues(s) and one or more oligonucleotide analogues(s) modifying pre-mRNA splicing may be combined with another.

Furthermore, also four or even all of the above listed oligonucleotide and oligonucleotide analogue groups (a) to (f) may be combined with another.

The oligonucleotides may be administered to cells of a patient and/or patients by any means known in the art. The person skilled in the art will know methods suitable for administering such molecules to cells and patients.

Exemplarily, an oligonucleotide or an analogue thereof may be administered to cells by means of electroporation (e.g., single pulse or multi-pulse electroporation (e.g., nucleofection), one or more amphiphilic lipid(s), one or more cell-penetrating peptide(s) (e.g., the chariot peptide, a polyarginine (e.g., R7, R8, R9, R10, R11 or R12), the HIV tat peptide, a lactoferrin-derived peptide, or an antimicrobial peptide, a nucleic targeting sequence), one or more liposome(s), one or more micelle(s), one or more episome(s), one or more polymersome(s), one or more microbead(s), one or more nanobead(s), one or more amphiphilic polymer(s), one or more positively charged polymer(s) (e.g., polyethylene imine (PEI)), one or more virus(es) (e.g., self complementary adeno-associated viruses (scAAV), an altered herpes simplex virus (HSV)), one or more viroid(s), and/or gene gun technology (e.g., by using gold beads).

Exemplarily, an oligonucleotide or an analogue thereof may be administered to a patient by means of one or more amphiphilic lipid(s), one or more cell-penetrating peptide(s), one or more liposome(s), one or more micelle(s), one or more polymersome(s), one or more microbead(s), one or more nanobead(s), one or more amphiphilic polymer(s), one or more positively charged polymer(s), one or more virus(es) and/or one or more viroid(s)

The person skilled in the art will know how to administer the oligonucleotides and analogues thereof. Administration to a patient may be systemically and/or locally.

Preferably, administration to a patient may include injecting the oligonucleotide(s) and/or analogue(s) thereof or the nasally uptake of these in order to circumvent the first pass effect.

Such oligonucleotide may be any one suitable for the purpose of the present disclosure, i.e., serving as an inhibitor of Kallikrein-8.

In a disclosure, the inhibitor is an oligonucleotide having a sequence identity of at least 80 %, 90%, 91%, 92%, 93%, 94%, 95%, 96%, 97%, 98%, 99%, or a sequence identity of 100% to any of SEQ ID Nos 8 to 13, preferably 9 to 13, more preferably the coding regions of any of SEQ ID Nos 9 to 13.

The disclosure relates to an agent for use which is capable of reducing the amount of Kallikrein-8 in the brain of a patient, wherein the agent is selected from a group consisting of a small molecule, a ribozyme, one or more nucleic acid(s), one or more oligonucleotide(s), a peptide and a protein, preferably wherein the agent is selected from (a) one or more interfering oligonucleotide(s) and (b) a protein which is capable of proteolytic cleavage of Kallikrein-8.

As shown in the examples, it is possible to deliver a therapeutically effective amount of a monoclonal antibody to the brain. In the example, this was achieved by intraventricular delivery. Such administration is also a preferred way of administration in humans.

To minimize adverse effects following long-term intraventricular administration such as inflammation, the agent such as an antibody should be modified. A convenient way of delivery is intravenous injection. In order to ensure sufficient passage across the blood-brain-barrier, the agent for use according to the invention is in one preferred embodiment bound to a compound which is able to transport the agent across the blood-brain barrier. The agent may be bound to the compound covalently or non-covalently, and/or in from of a fusion protein, conjugate, complex, liposome or nanoparticle. The agent may be bound to the compound in form of a fusion protein. In such embodiment, the compound which is able to transport the agent across the blood-brain barrier is itself a protein or peptide.

For example, a monovalent molecular shuttle to increase brain penetration and potency of therapeutic antibodies was described recently (Niewoehner et al., 2013, http://dx.doi.org/10.1016/j.neuron.2013.10.061). A fusion of an agent for use according to the invention with this molecular shuttle, which is an anti-Transferrin receptor antibody (anti-TfR antibody), enables intravenous administration of an agent for use of the invention, and therefore a long-term treatment with minimized adverse side-effects.

Accordingly, the agent for use of the invention is in a preferred embodiment bound to a compound which is able to transport the agent across the blood-brain barrier. More preferably, the agent is bound to the compound covalently or non-covalently, and/or in from of a fusion protein, conjugate, complex, liposome or nanoparticle. Even more preferably, the agent is bound to an antibody which specifically binds to the Transferrin receptor (Tfr) or a functionally active part thereof or an antibody mimetic thereof. In particular, the antibody which specifically binds to the Transferrin receptor (Tfr) or a functionally active part thereof or an antibody mimetic thereof is the antibody as described in Niewoehner et al above and/or does not interfere with the binding of TfR to Transferrin.

An antibody mimetic is understood as an organic compound, preferably a protein or peptide, that, like antibodies, can specifically bind antigens, but that is not structurally related to antibodies. Preferably, the antibody mimetic is selected from an affibody, affitin, affimer, affilin, alphabody, anticalin, avimer, DARPin, fynomer, Kunitz domain peptide and monobody, which are known in the art.

Accordingly, the present disclosure relates to an agent which inhibits Kallikrein-8, wherein the agent is bound to a compound which is able to transport the agent across the blood-brain barrier,
preferably wherein the agent is bound to the compound covalently or non-covalently, and/or in from of a fusion protein, conjugate, complex, liposome or nanoparticle,
more preferably wherein the agent is bound to an antibody which specifically binds to the Transferrin receptor (Tfr) or a functionally active part thereof or an antibody mimetic thereof,
even more preferably wherein the agent is a protein, preferably an antibody or functionally active part thereof or an antibody mimetic, such as an anti-KLK8 antibody or functionally active part thereof or an antibody mimetic thereof.

It is disclosed that the agent is bound to an antibody which specifically binds to the Transferrin receptor (Tfr) or a functionally active part thereof or an antibody mimetic thereof. In particular, the antibody which specifically binds to the Transferrin receptor (Tfr) or a functionally active part thereof or an antibody mimetic thereof is the antibody as described in Niewoehner et al above and/or does not interfere with the binding of TfR to Transferrin.

As described above, other modes of administration known in the art are possible. In one preferred embodiment, the agent, which is optionally bound to a compound which is able to transport the agent across the blood-brain-barrier (BBB), is administered intravenously, nasally, orally, or by intraventricular delivery.

Intraventricular delivery can be achieved by intracerebroventricular (icv) devices, such as a catheter, or osmotic pumps for intraventricular drug infusion. Further the agent may be delivered by surgical implantation of devices that release the agent to brain tissue for variable time durations, such as sponges. Further, intranasal delivery is suitable for agents for use according to the present invention, in order to bypass the BBB. Formulations suitable for intravenous, nasal, oral, or intraventricular delivery or for delivery by implantation into the brain are known to a skilled person.

The duration of treatment, dosage and administration scheme will depend on the agent for use, the formulation and the patient to be treated or prevented.

It is possible to administer the agent repeatedly in single doses or continuously. A long-term treatment, either continuously, by repeated single doses, or by repeated continuous administrations, over 1 day, or 1 week of more, 1, 2, 3, 4, 5, or 6 months or more, 1 to 5 or 10 years or more or even a lifelong administration is possible.

Accordingly, in a further preferred embodiment, the agent for use is administered over a period of 1 day or 1 week of more, 1, 2, 3, 4, 5, or 6 months or more, 1 to 5 or 10 years or more.

For an agent, for example an agent being or comprising an antibody or physiologically active fragment thereof, a typical dosage may be in the range of 1 ng/kg bw or 1 µg/kg bw to 100 mg/kg bw per dose and/or per day.

A considerable up-regulation of KLK8 protein and kallikrein 8 mRNA in human and murine brain was surprisingly found at incipient stages of AD, long before the "clinical" signs of disease appear. Additionally, we could demonstrate increased KLK8 protein levels in cerebrospinal fluid (CSF) and blood serum of AD patients in comparison to age-matched healthy controls. Further, we surprisingly show that four weeks of KLK8 inhibition, by intraventricular delivery of an anti-KLK8 antibody after disease onset, is sufficient to mitigate multiple features of Alzheimer's pathology in transgenic CRND8 mice. Accordingly, the present agent for use in treatment or prevention is in particular suitable for administration to a patient which exhibits an increased level of Kallikrein-8 and/or kallikrein-8 mRNA. Such increased level of Kallikrein-8 or of kallikrein-8 mRNA, or of both Kallikrein 8 protein and kallikrein 8 mRNA may be observed in one or more tissues of interest, in particular in cerebrospinal fluid (CSF), in brain tissue, which is preferably a brain tissue biopsy, or in blood, serum or plasma. CSF and brain tissue are particularly preferred.

A level is considered to be increased in case the level is higher than a reference level from a healthy population, in particular by 10%, 20%, 50%, 100% or more higher than a reference value from a healthy population. The reference value may be determined as a median or mean value or as cut-off value for quantitative determinations. Also, semi-quantitative determinations are possible, such as by visual inspection such as in case of in situ hybridization based detection methods.

The level of kallikrein 8 mRNA may be determined by methods known in the art, in particular by semi-quantitative or quantitative RT-PCR.

The level of kallikrein 8 mRNA may be determined using suitable probes. Suitable probes or may be oligonucleotides or oligonucleotide derived molecules which are capable to specifically hybridize to Kallikrein 8 mRNA under stringent conditions.

In a preferred embodiment, Kallikrein 8 mRNA has a sequence of any of SEQ ID Nos 8 to 13, preferably 9 to 13, more preferably the coding regions of any of SEQ ID Nos 9 to 13.

In a preferred embodiment, probe comprises or consists of an oligonucleotide having a sequence identity of at least 80 %, 90%, 91%, 92%, 93%, 94%, 95%, 96%, 97%, 98%, 99%, or a sequence identity of 100% to any of SEQ ID Nos 8 to 13, preferably 9 to 13, more preferably the coding regions of any of SEQ ID Nos 9 to 13.

The probe length may vary. Typical probe lengths are 10, 15, 16, 17, 18, 19, 20, 21, 22, 23, 24, 25, 26, 27, 28, 29, 30 or more nucleotides, such as up to 50, 100, 150, or 200 nucleotides.

"Stringent conditions" refers to hybridization conditions under which the nucleic acid molecules that are capable of hybridizing to the Kallikrein 8 mRNA nucleic acid molecule or parts thereof do not cross hybridize to unrelated nucleic acid molecules. Stringent conditions are sequence-dependent and will be different in different circumstances. Appropriate stringent hybridization conditions for each nucleic acid sequence may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), loc. cit., see in particular the chapter "Hybridization Strategy" by Britten & Davidson, 3 to 15. Such conditions comprise, e.g. an overnight incubation at 65° C. in 4xSSC (600 mM NaCl, 60 mM sodium citrate) followed by washing at 65° C. in 0.1×SSC for one hour. Alternatively, hybridization conditions can comprise: an overnight incubation at 42° C. in a solution comprising 50% formamide, 5×SSC (750 mM NaCl, 75 mM sodium citrate), 50 mM sodium phosphate (pH 7.6), 5×Denhardt's solution, 10% dextran sulphate, and 20 µg/ml denatured, sheared salmon sperm DNA, followed by washing in e.g. 0.1-0.5×SSC at about 55-65° C. for about 5 to 20 min. Said conditions for hybridization are also known by a person skilled in the art as "highly stringent conditions for hybridization".

kallikrein 8 mRNA may be detected in situ, by methods known in the art, such as in situ hybridisation methods, such as FISH and chromogenic in situ hybridization (CISH). In these applications, the probe is typically bound to a detectable label itself or a binding partner of a bioaffine binding pair. In the latter case, the second partner of a bioaffine binding pair comprises a detectable label, such as a fluorescent or chromogenic label, depending on the application. Alternatively, the level of mRNA may be determined using amplification methods, such as PCR, optionally using a reverse transcription step.
Kallikrein 8 protein level may be determined using immunoassays, such as Western Blotting or ELISA methods known to a skilled person.

Accordingly, in a further preferred embodiment of the present invention, the patient exhibits an increased level of Kallikrein-8 and/or kallikrein-8 mRNA in brain tissue and/or in cerebrospinal fluid and/or in the blood.

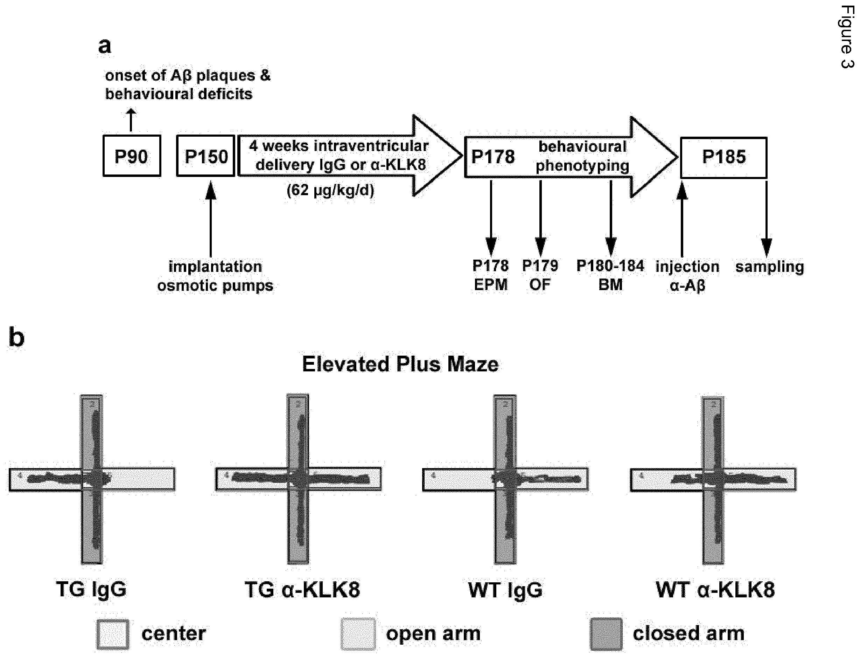

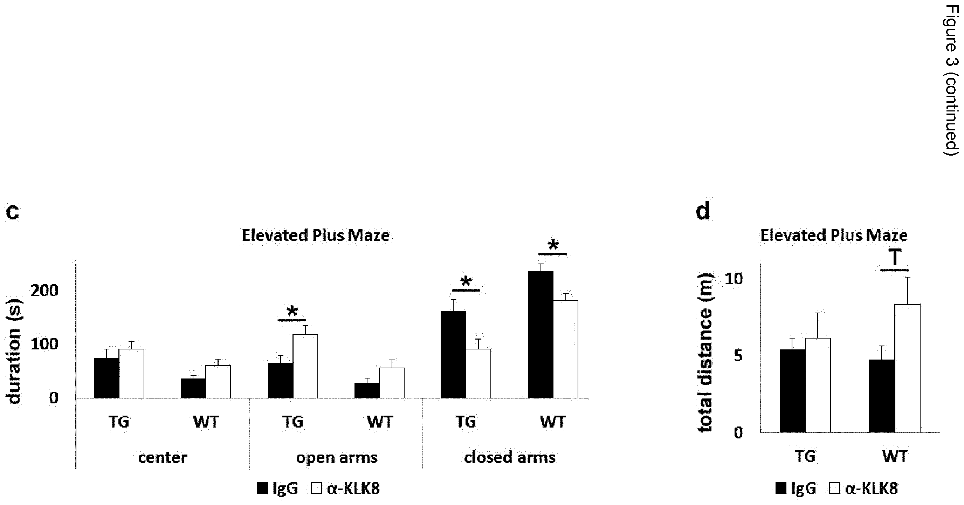

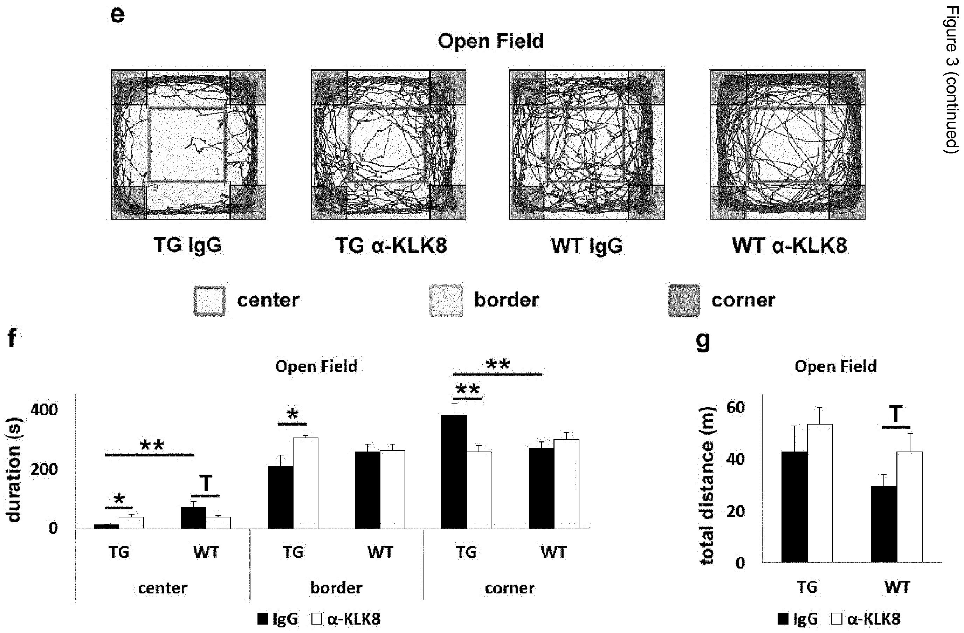

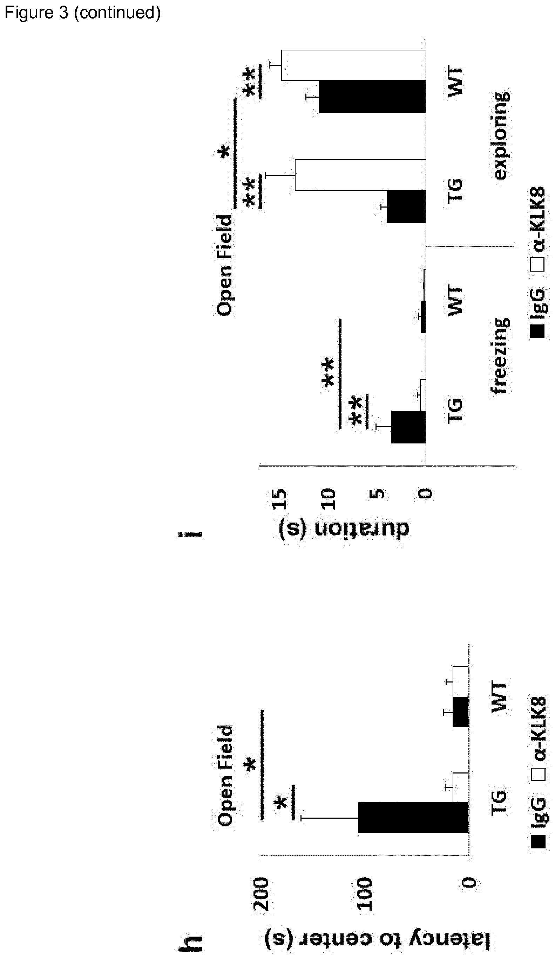

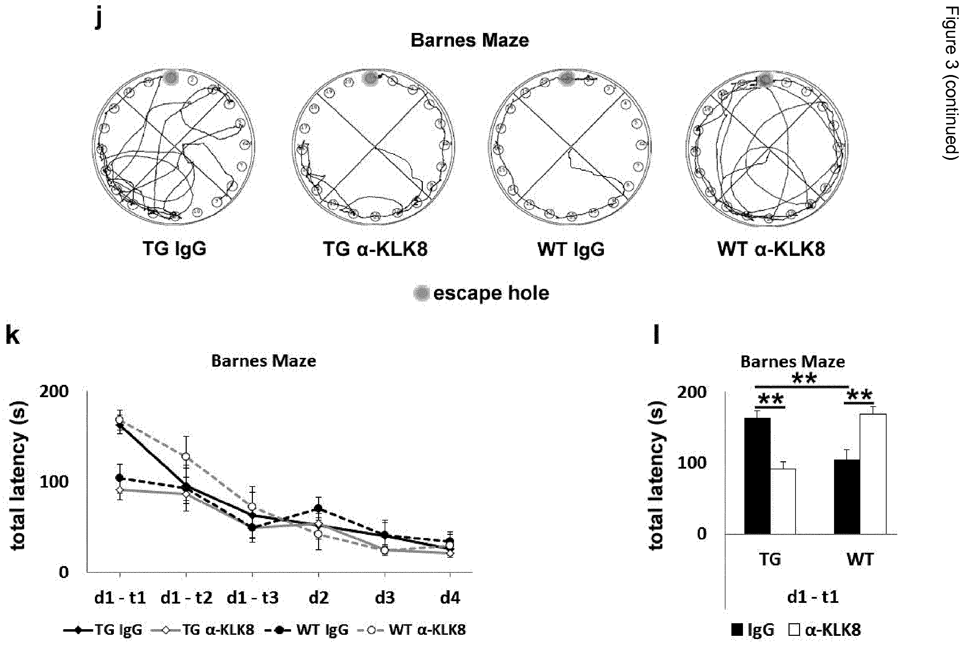

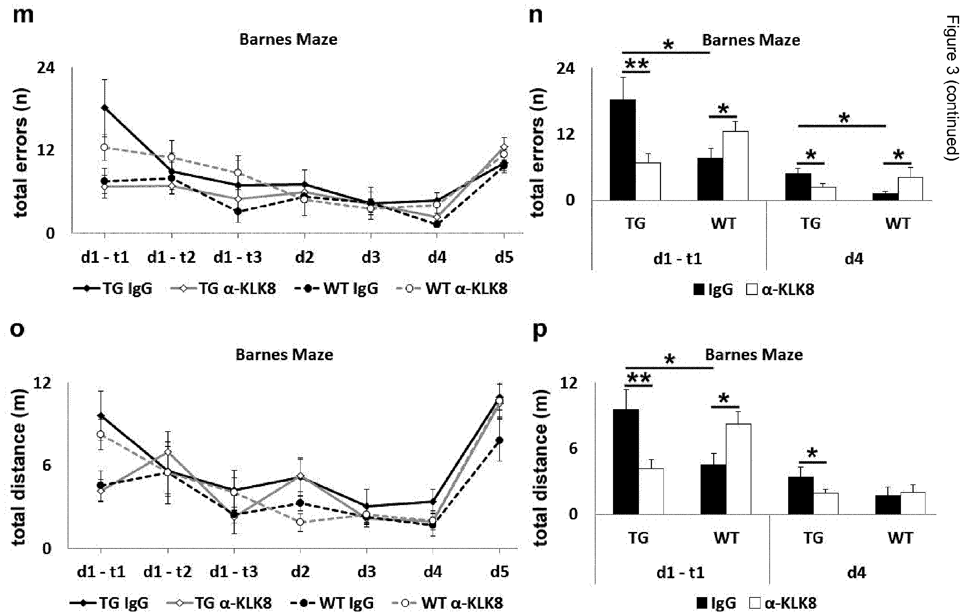

It was surprisingly shown in the Examples that antibody-mediated cerebral KLK8 inhibition has anxiolytic effects and increases exploratory behavior in a transgenic mouse model of AD, as anti-KLK8 antibody-treated animals spent more time in the open arms and less time in the closed arms of the EPM (Fig. 3b, c), spent more time in the center and border areas and less time in the corners of the OF arena (Fig. 3e, f), took less time to enter the center area for the first time (Fig. 3h), and showed reduced freezing and increased exploratory behavior (Fig. 3i), when compared to controls (IgG & saline). Evaluation of the BM further revealed that blockade of KLK8 improved spatial memory performance in AD-affected mice, as verum-treated transgenics had reduced latencies (Fig 3j-l), explored fewer wrong holes (Fig. 3m, n), and covered shorter distances (Fig. 3o, p) before escaping through the escape hole at trial 1 on test day 1 (24 h following 2 trials of habituation) as well as on test day 4.

Further, Kallikrein 8 overexpression could already be detected in 30 days old transgenic mice, at an age where AD pathology and cognitive impairment are still absent, thereby correlating to a precursor stage of AD. Accordingly, administration of an agent for use of the invention to a patient having or diagnosed to have a precursor stage of AD is expected to have a beneficial effect for treating or preventing the disease.

Therefore, the administration of an agent for use of the invention was shown to be suitable for treating or preventing the symptoms of Alzheimer's disease increased anxiety and cognitive impairment, as well as the corresponding symptoms of precursor stages of Alzheimer's disease, such as MCI and increased anxiety.

Therefore, in a further preferred embodiment, the disease symptoms increased anxiety and/or cognitive impairment are prevented or treated by the agent for use according to the invention.

The agent for use of the invention is preferably administered to a patient who has Alzheimer's disease and/or a precursor stage of Alzheimer's disease, such as MCI and/or a preclinical stage of Alzheimer's disease. Typically, such patient is diagnosed or was diagnosed at some time point to have Alzheimer's disease and/or a precursor stage of Alzheimer's disease or a preclinical stage of Alzheimer's disease. As noted above, a definite diagnosis of Alzheimer's disease can only be performed post mortem, by histopathological examination. Accordingly, a patient who has Alzheimer's disease and/or a precursor stage of Alzheimer's disease or a patient who is diagnosed to have Alzheimer's disease and/or a precursor stage of Alzheimer's disease is understood to relate to a patient who likely has Alzheimer's disease and/or a precursor stage of Alzheimer's disease or a patient who is diagnosed to likely have Alzheimer's disease and/or a precursor stage of Alzheimer's disease, by applying the current tests such as cognitive tests for Alzheimer's disease. Typically, a patient who has or is diagnosed to have a precursor stage of Alzheimer's disease typically exhibits mild cognitive impairment, as can be determined by tests known to a skilled person. Accordingly, a patient who has a preclinical stage of Alzheimer's disease or a patient who is diagnosed to have a preclinical stage of Alzheimer's is understood to relate to a patient who belongs to a high risk group for developing Alzheimer's disease in the future, by applying the current tests, such as occurrence of Alzheimer's disease in the family of the individual and/or determination of mutations known to be associated with Alzheimer's disease, such as mutations in the presenilin-1 or presenilin-2 gene or the APP gene known to be associated with Alzheimer's disease.

The data from the Examples demonstrate that anti-KLK8 treatment attenuates Alzheimer's disease-like pathology in mice when started at 150 days of age, corresponding to approximately 2-3 months after disease onset in TgCRND8 mice. Accordingly, it is extrapolated that human patients in CDR stages 0,5, 1, 2 or 3, encompassing very mild, mild, moderate and severe Alzheimer's disease, respectively, more preferably very mild, mild, and moderate Alzheimer's disease will in particular benefit from the administration of an agent for use of the invention.

Accordingly, in a further preferred embodiment, the agent for use according to the invention is administered to a patient who has Alzheimer's disease and/or a precursor stage of Alzheimer's disease or a preclinical stage of Alzheimer's disease, and/or is diagnosed to have Alzheimer's disease and/or a precursor stage of Alzheimer's disease or a preclinical stage of Alzheimer's disease.

Further, an elevation of Kallikrein 8 protein and kallikrein 8 mRNA levels was found in brain tissue of mice and humans suffering from AD-like-pathology or AD, respectively. Moreover, increased KLK8 protein levels in cerebrospinal fluid (CSF) and blood serum could be detected in AD patients in comparison to age-matched healthy controls. Accordingly, it is expected that patients who exhibit elevated levels of Kallikrein 8 protein and kallikrein 8 mRNA in a bodily sample, in particular in blood, CSF or brain tissue, are in particular responsive to treatment with an agent for use of the invention. It is therefore preferred to treat patients who are identified to be responsive to a treatment which inhibits Kallikrein 8.

Therefore, in a further preferred embodiment, the patient is identified to be responsive to treatment with an agent for use of the invention which inhibits Kallikrein-8, preferably wherein the patient is identified to have an increased level of Kallikrein 8 and/or kallikrein 8 mRNA in at least one bodily sample and/or in at least one bodily tissue. In a more preferred embodiment, the patient has Alzheimer's disease and/or a precursor stage of Alzheimer's disease or a preclinical stage of Alzheimer's disease at the start of treatment, and/or is diagnosed to have Alzheimer's disease and/or a precursor stage of Alzheimer's disease or a preclinical stage of Alzheimer's disease at the start of treatment.

As Alzheimer's disease is a chronic, progressive disease, the agent for use of the invention may be also be administered or continued to be administered in case the disease progresses to a different stage and/or is diagnosed to progress to a different stage (such as from CRD stage 1 to stage 2).

Therefore, in a yet more preferred embodiment of the present invention, the patient has Alzheimer's disease and/or a preclinical stage of Alzheimer's disease, in particular at the start of treatment, and/or is diagnosed to have Alzheimer's disease and/or a preclinical stage of Alzheimer's disease, in particular at the start of treatment.

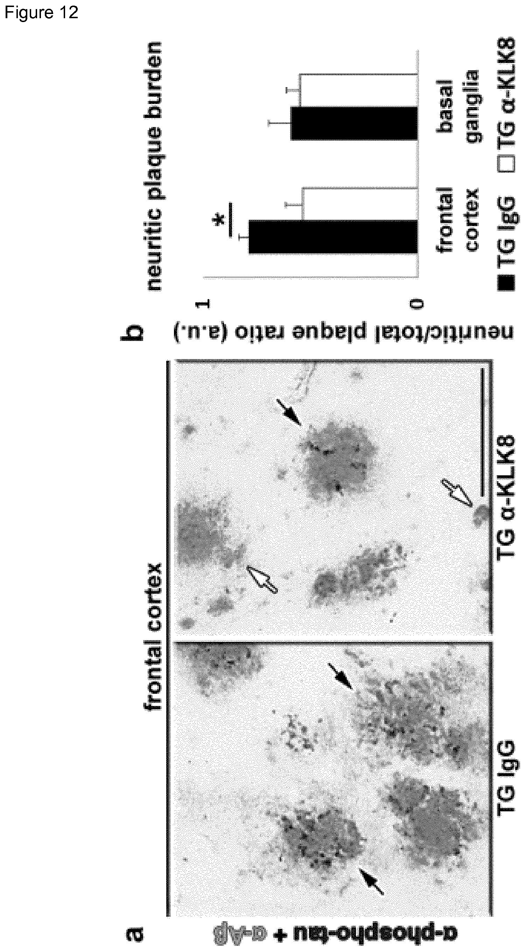

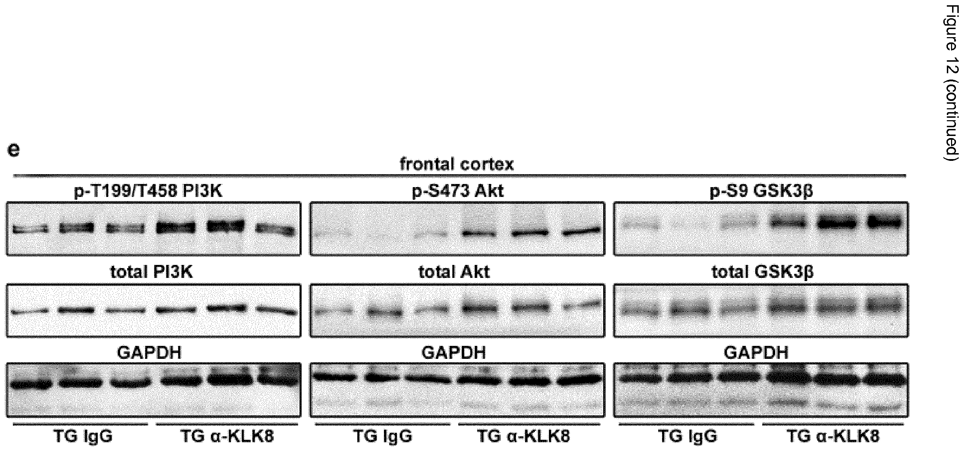

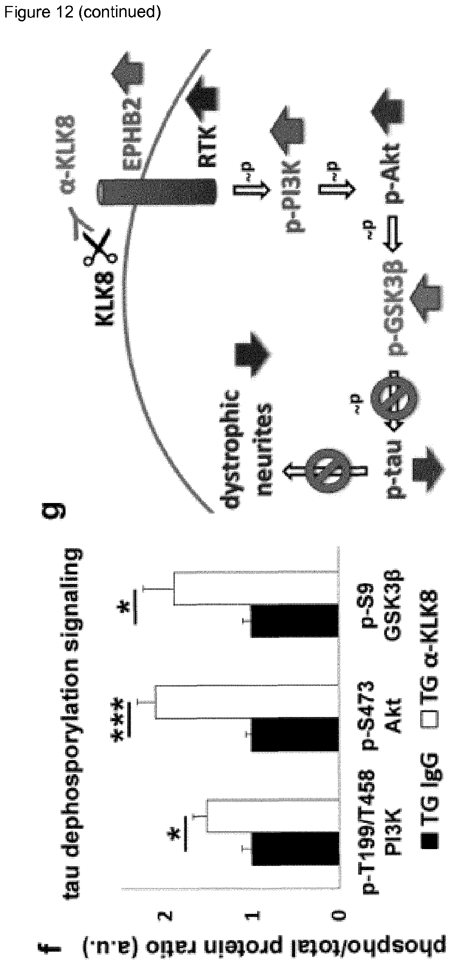

In addition to the mitigating of the disease symptoms studied, i.e. increased anxiety and cognitive impairment, it was surprisingly shown that administration of an anti-KLK8 antibody to a murine AD model also counteracts a variety of molecular features of Alzheimer's disease. Surprisingly, it was found that KLK8 inhibition and resulting EPHB2 protection diminishes AD-associated tau pathology: For example, it could be shown that Tau-hyperphosphorylation, which is typical for Alzheimer's disease decreases drastically by administration of the anti-KLK8 antibody. In particular, we could show that four weeks of anti-KLK8 antibody administration reduced the ratio of neuritic plaques in relationship to the number of total plaques and reduced tau phosphorylation at amino acids S202/T205, S396 and S212/214 in the frontal cortex of transgenic mice (Fig. 12a to d). Neuritic plaques are understood as plaques containing phospho-tau positive dystrophic neurites (Fig. 12a to d). The neuroprotective effect of anti-KLK8 therapy against tau hyperphosphorylation was mediated by activation of PI3K (indicated by increased phosphorylation of PI3K at T199/T458) as well as Akt (indicated by increased phosphorylation of Akt at S473), and thus down-stream inhibition of GSK3β (indicated by increased phosphorylation of GSK3β at S9) (Fig. 12e, f).

In particular, Tau hyperphosphorylation is decreased in a brain sample of a patient by 5%, 10%, 15%, 20%, 30%, 40%, 50% or more, as compared to a brain sample prior to administration. Tau phosphorylation is understood according to the present invention as phosphorylation of Tau at any of the phosphorylation sites known in the art. Accordingly, Tau hyperphosphorylation is understood according to the present invention as increased level of phosphorylation of Tau proteins at any of the phosphorylation sites known in the art, such as sites S202, T205, S396, S212 and/or S214.

In a further preferred embodiment, the proportion of neuritic plaques is decreased in a brain sample of a patient by 5%, 10%, 15%, 20% or more, as compared to a brain sample prior to administration. A method for determining such neuritic plaques is described in detail in the Examples. The proportion of neuritic plaques is preferably determined by stereological quantification of the neuritic to total plaque ratio, as shown in the Examples. In a further preferred embodiment, the proportion of neuritic plaques is decreased in the frontal cortex region of the brain.

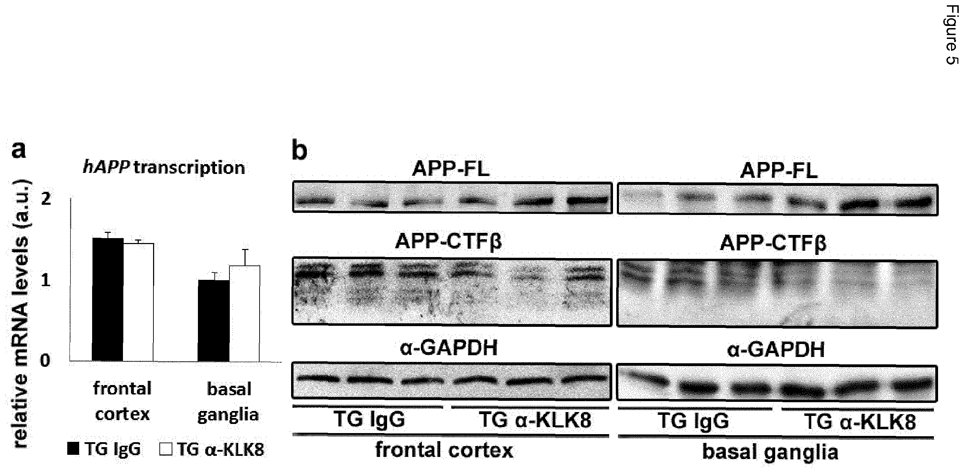

Further, four weeks of anti-KLK8 antibody administration increased Amyloid precursor Protein -full length (APP-FL) levels, decreased APP C-terminal fragments β (CTFβ) and β-Amyloid 42 (Aβ₄₂) peptide concentration, while β-Amyloid 40 (Aβ₄₀) and soluble APP-alpha (sAPPα) peptide levels remained unaffected (Fig. 5a-e). These results indicate that blockade of KLK8 impedes amyloidogenic APP processing. Further, stereological quantification revealed that in the basal ganglia, KLK8 blockade diminished the total volume and average size of diffuse Aβ plaques (which gradually evolve into core plaques). Accordingly, amyloidogenic APP processing, as indicated by an increase in APP-FL levels, a decrease in APP C-terminal fragments β (CTFβ), a decrease in Aβ₄₂ peptide concentration and/or a decrease in the total volume and/or average size of diffuse Aβ plaques can be reduced in a brain sample of a patient, in particular reduced by 5%, 10%, 15%, 20%, 30% or more in a brain sample of a patient, as compared to a brain sample prior to agent administration. Further, the Aβ load can be reduced by 5%, 10%, 15%, 20%, 30% or more in a brain sample of a patient as compared to a brain sample prior to agent administration. "Aβ load" is understood as the amount of soluble or insoluble Aβ₄₂ and/or Aβ₄₀ in the brain and/or in the cerebrospinal fluid of patients. Aβ load can be determined as shown in the Examples.

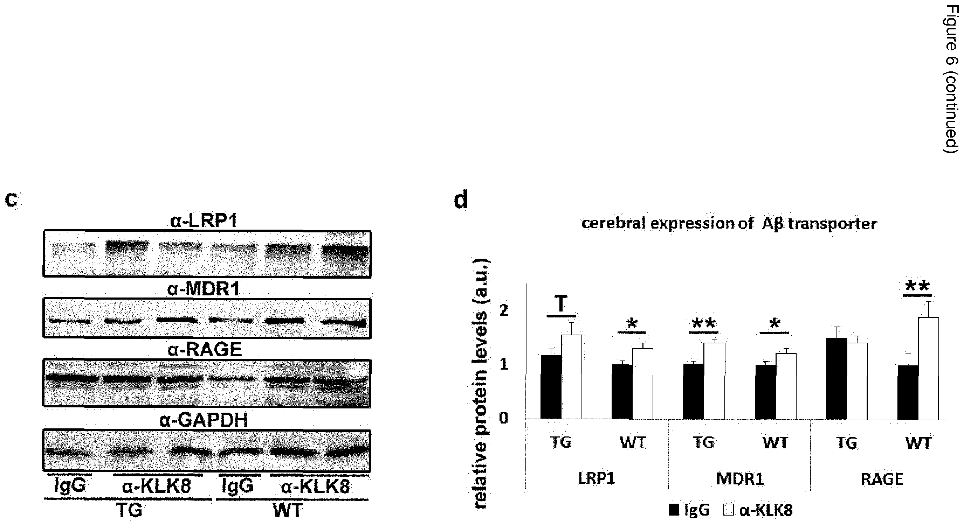

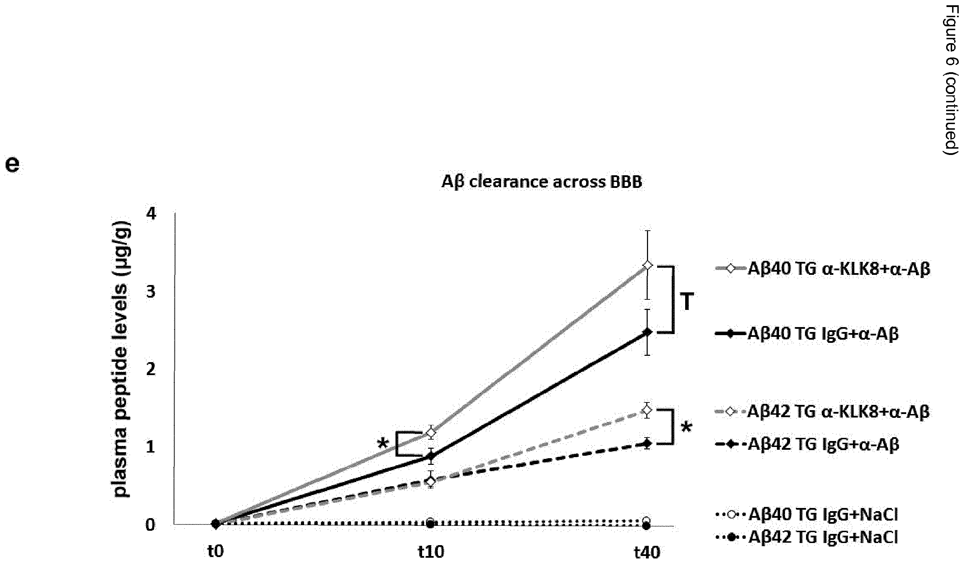

Further, it could be shown that KLK8 inhibition improves neurovascular function. Improvement of neurovascular function preferably characterized by an increase in LRP1 and/or MDR1 protein levels in a brain tissue of a patient as compared to a brain sample prior to administration, and/or by an increase of Aβ₄₀ and/or Aβ₄₂ clearance across the brain - blood-barrier (BBB) in a patient, as compared to clearance levels prior to administration of the agent. The levels can be determined by methods known to a skilled person, in particular as described in the Examples. As described in the Examples, it was surprisingly found that cerebral LRP1 and MDR1 protein levels increased (the first one in transgenics by trend) following anti-KLK8 antibody delivery (Fig. 6c, d), suggesting facilitated elimination of cerebral Aβ via BBB-mediated clearance. Further, anti-KLK8 antibody treatment increased plasma Aβ₄₀ at *t₁₀* (and at *t₄₀* by trend) as well as Aβ₄₂ at *t₄₀,* indicating improved Aβ clearance across the BBB (Fig. 6e). Preferably, LRP1 and/or MDR1 protein levels in a brain tissue of a patient increase by 5%, 10%, 15%, 20%, 30% or more by as compared to a brain sample prior to administration. Further, preferably, the clearance of Aβ₄₀ and/or Aβ₄₂ increases by 5%, 10%, 15%, 20%, 30% or more in a patient, as compared to clearance prior to administration of the agent.

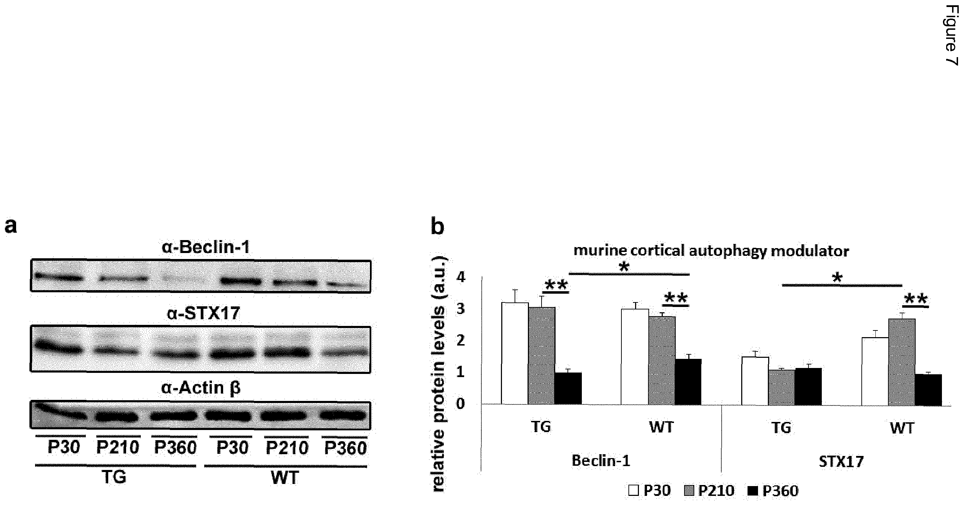

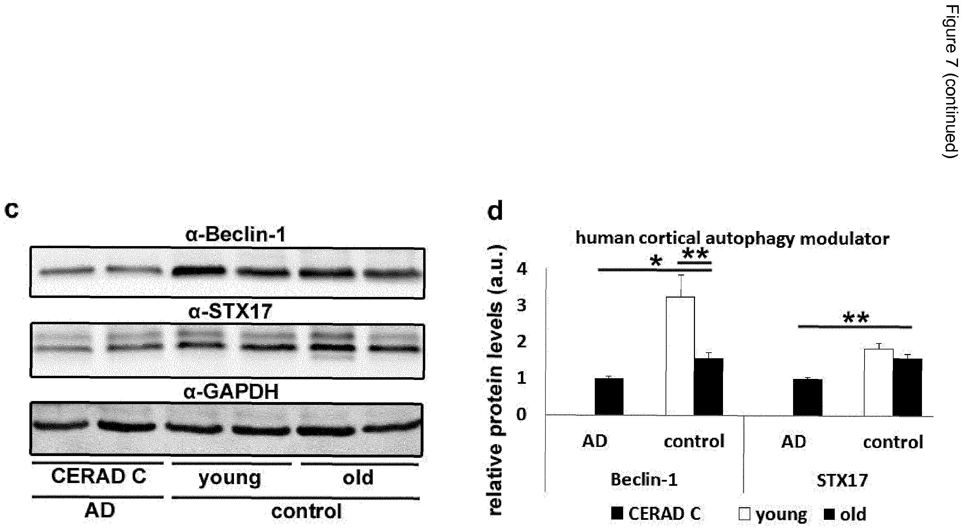

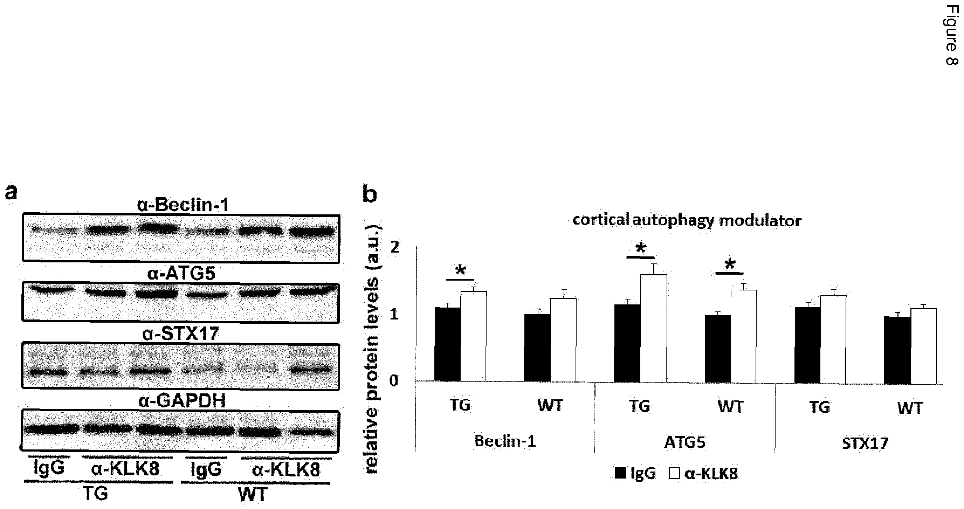

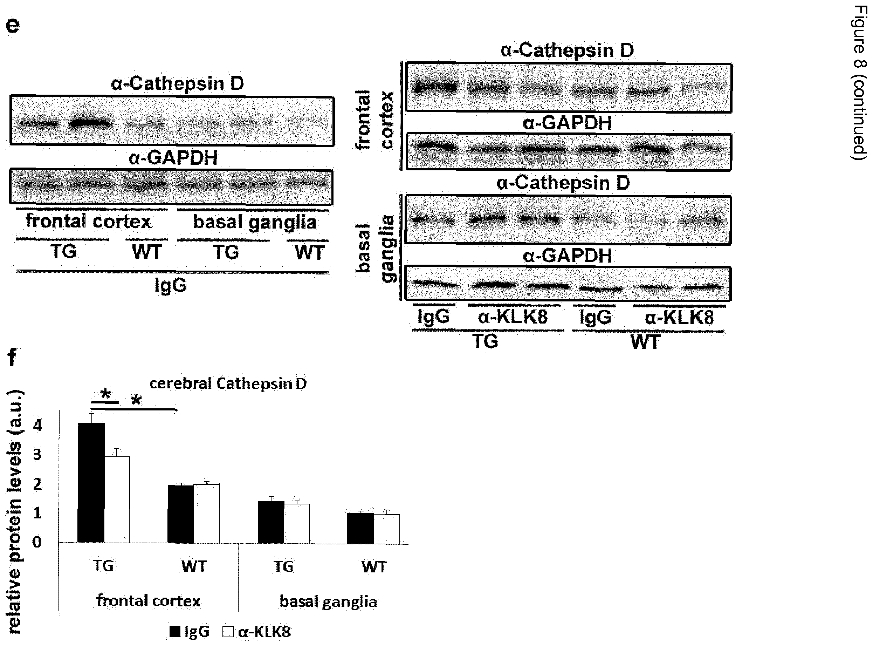

Further, it could be shown that KLK8 blockade induces autophagy and Aβ phagocytosis. As described in the Examples, we corroborated an AD-related cerebral perturbation of autophagy modulators, i.e. beclin-1, involved in the initiation of autophagosome assemblies, and STX17, a protein that triggers fusion of autophagosomes with lysosomes in both mouse (Fig. 7a, b) and man (Fig. 7c, d). The next step in the Examples was to assess the protein levels of beclin-1, ATG5, essential for the autophagosome assembly, and STX17 in anti-KLK8 antibody versus IgG-treated mice. In transgenics, and to a lesser extent in wildtypes, KLK8 inhibition elevated the levels of beclin-1 and ATG5 in the frontal cortex and basal ganglia (Fig. 8a-d). By rescuing the autophagy machinery, anti-KLK8 antibody treatment also reversed the cortical accumulation of intraneuronal cathepsin D (Fig. 8e-g) - a lysosomal enzyme, which is present in excess in murine and human AD affected brain. The levels of cathepsin D were lower in the basal ganglia per se, and remained unaffected by treatment (Fig. 8e, f).

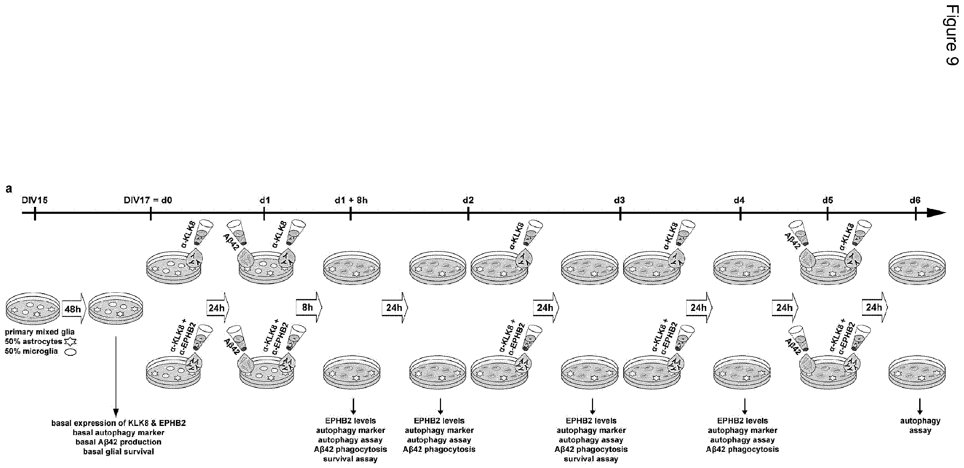

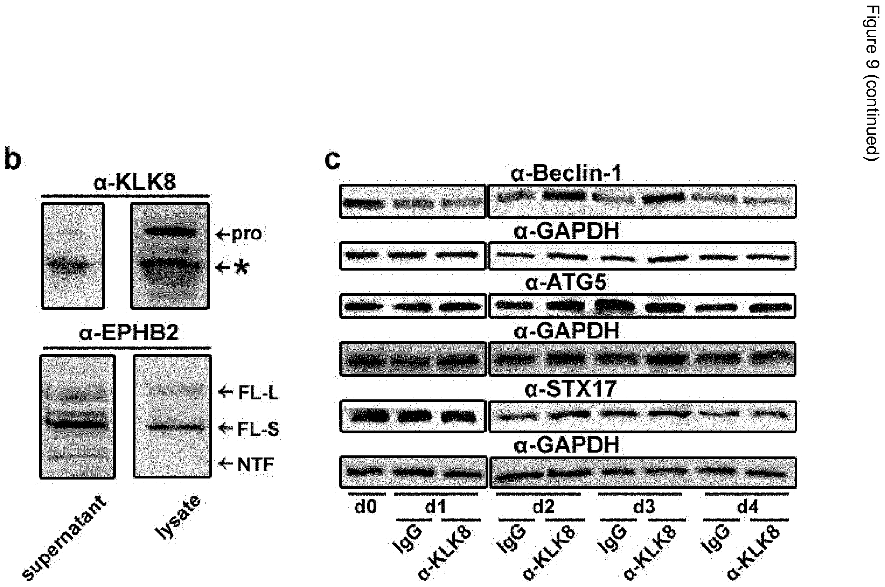

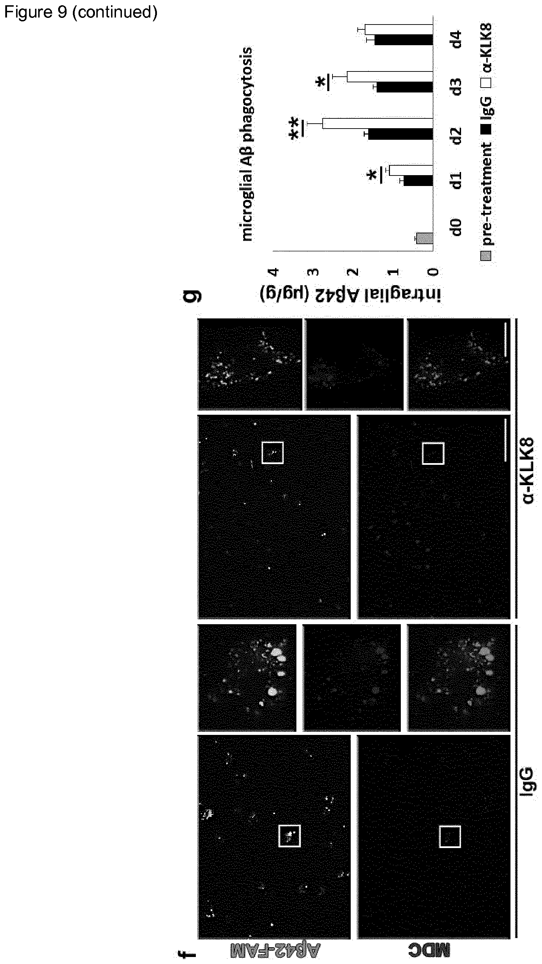

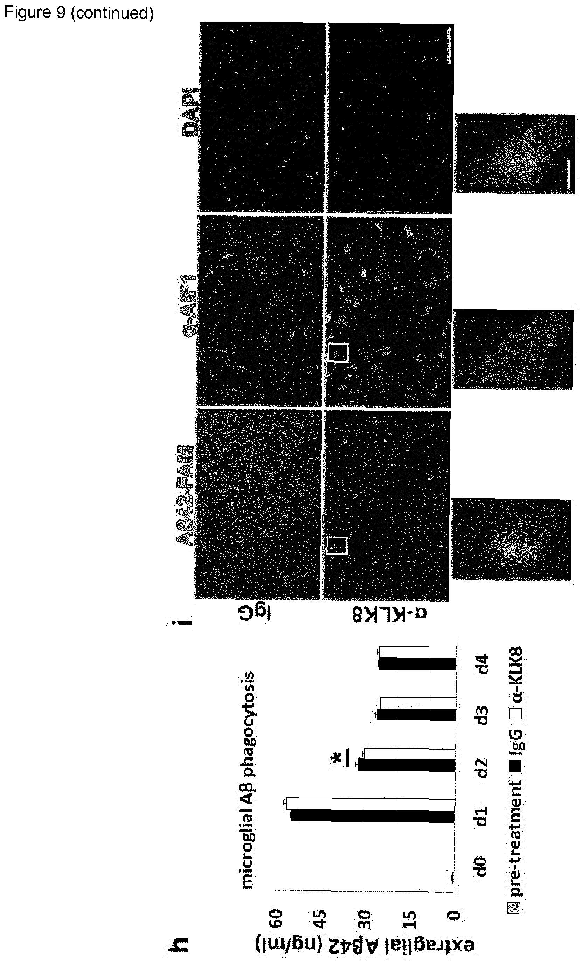

Impaired Aβ phagocytosis and dysfunctional beclin-1-associated autophagy in the AD affected brain is tightly linked to reduced microglial activity. Accordingly, we examined the effect of anti-KLK8 antibody treatment on primary transgenic and wildtype microglial/astroglial co-cultures (for experimental design see Fig. 9a) after demonstrating KLK8 secretion and EPHB2 expression (but virtually no Aβ generation) in these naïve cells (Fig. 9b). While incubation with Aβ₄₂ (at d1) knocked down the expression levels of glial autophagy molecules beclin-1, ATG5, and STX17 and weakened fluorescence emission in the autophagy assay, simultaneous co-treatment with anti-KLK8 antibody protected the autophagy machinery in both transgenic and wildtype glial cells (Fig. 9c-f). Of note, anti-KLK8 antibody treatment doubled intramicroglial Aβ₄₂ levels (co-localizing with microglial marker AIF1, Fig. 9g, i), while Aβ₄₂ levels in the supernatant were reduced when compared to IgG control (Fig. 9h, i), pinpointing an enhanced clearance of extracellular Aβ₄₂ via microglial phagocytosis.

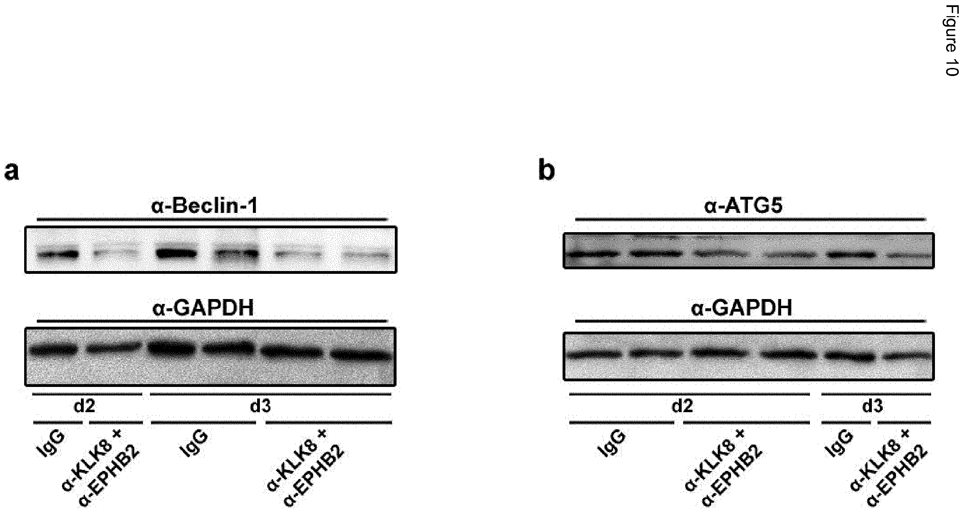

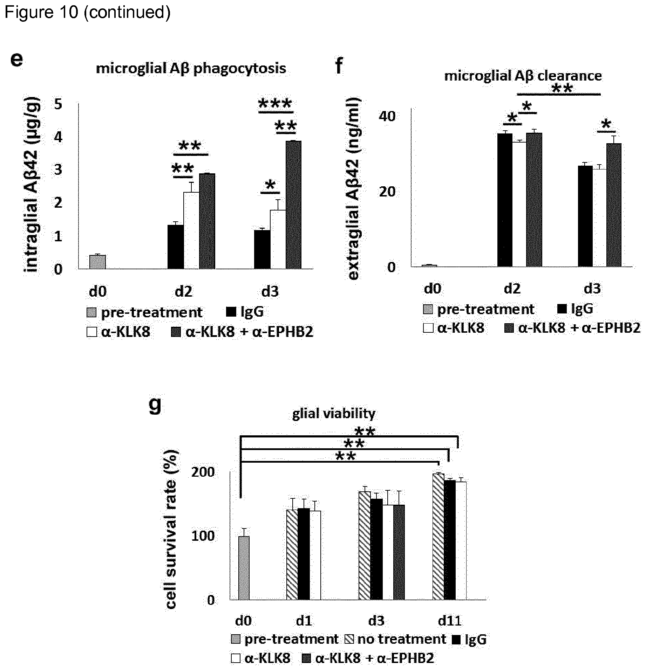

To test whether the positive effects of KLK8 inhibition on autophagy protection and Aβ clearance were transduced by EPHB2 receptor, we co-incubated anti-KLK8 antibody-treated glial cells with an EPHB2 inhibitory antibody (Attwood et al., supra). In spite of anti-KLK8-antibody presence, inhibition of EPHB2 abolished autophagy and reduced microglial Aβ clearance to levels indistinguishable from or even, below IgG control treated cells, underlining the decisive role of EPHB2 in this context (Fig. 10a-f). Prolonged cell viability monitoring for up to 11 days of treatment revealed that neither anti-KLK8 antibody nor anti-EPHB2 antibody affected glial survival or proliferation (Fig. 10g). A genotype-specific difference in basal autophagy and Aβ phagocytosis efficacy could not be detected in primary glia (data not shown), supporting the data of previous publications that microglial functional impairment coincides with amyloid deposition and does not precede it.

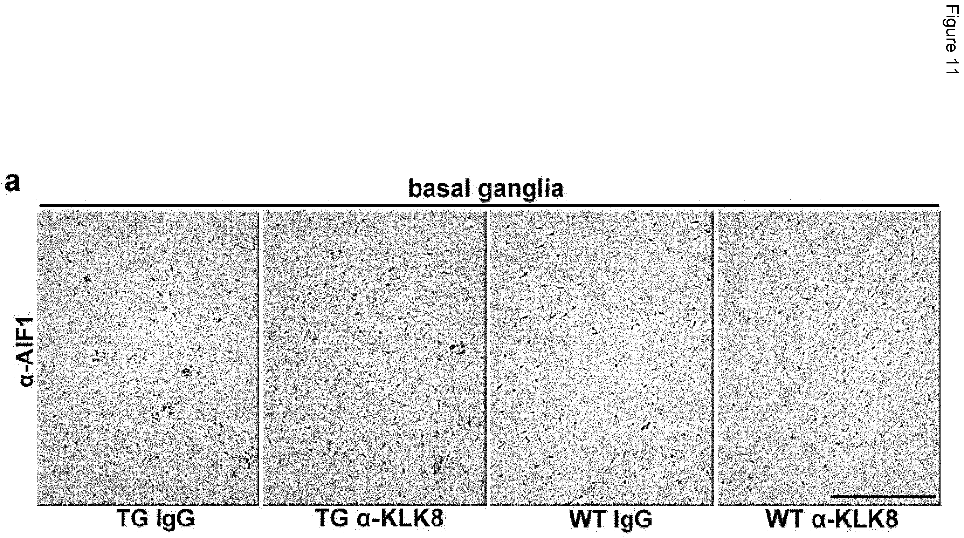

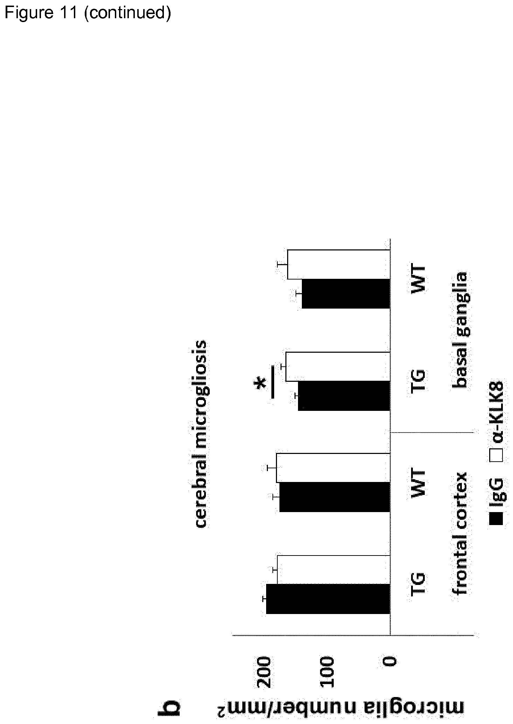

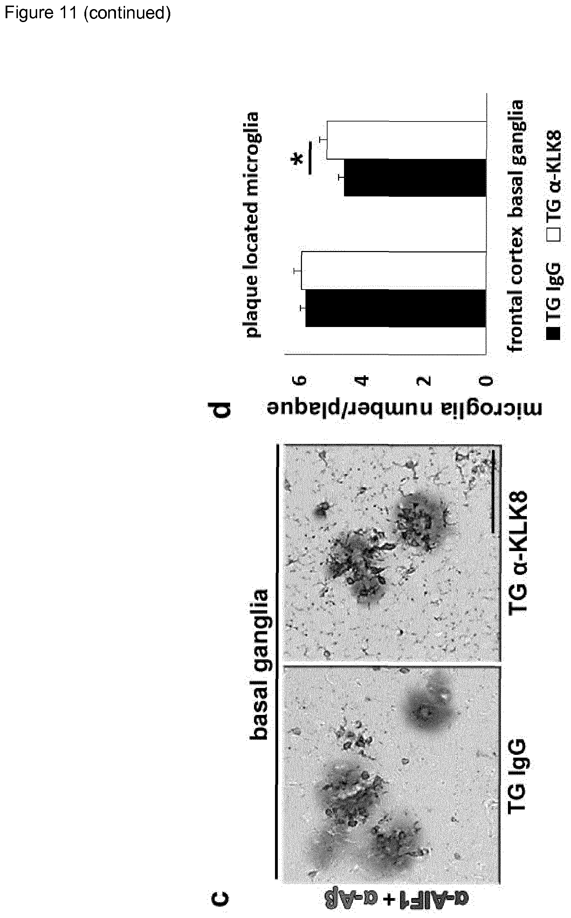

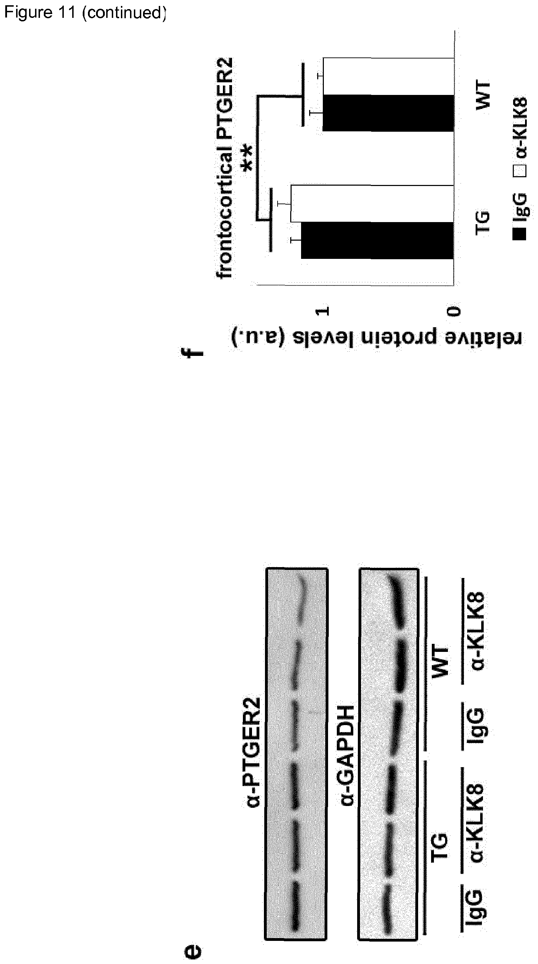

Next, we searched for evidence of an enhanced microglial Aβ phagocytosis triggered by KLK8 inhibition *in vivo.* Stereological quantification revealed an increase in the total number of activated AIF1-positive microglia in the basal ganglia (but not frontal cortex) of transgenic (but not wildtype) mice (Fig. 11a, b). Additionally, the average number of plaques surrounding microglia was elevated in the basal ganglia (but not in frontal cortex) in verum-treated transgenics (Fig. 11c, d). As the expression of the pro-inflammatory prostaglandin E receptor 2 (PTGER2) was not affected by anti-KLK8 antibody treatment (Fig. 11e-h), the utilisation of anti-KLK8 antibody seems to promote the proliferation of phagocytic rather than cytotoxic microglia, plaque approximation and subsequent Aβ uptake also *in vivo.* Together, our *in vitro* and *in vivo* data strongly support that KLK8 inhibition induces autophagy and Aβ clearance via microglial phagocytosis.

Accordingly, in a more preferred embodiment, levels of beclin-1 and/or ATG5 and/or and STX17 increase by 5%, 10%, 15%, 20%, 30% or more in the frontal cortex and basal ganglia in a patient as compared to prior to administration of the agent. In another preferred embodiment, microglial Aβ phagocytosis, as characterized by intramicroglial Aβ₄₂ levels increases by 5%, 10%, 15%, 20%, 30% or more in a sample of a patient as compared to prior to administration of the agent. Intramicroglial Aβ₄₂ levels may be determined as known by a skilled person and preferably as described in the Examples.

Therefore, in a further preferred embodiment of the present invention, administration to a patient results in reduced amyloidogenic APP processing and/or reduced Aβ load and/or reduced Tau hyperphosphorylation and/or a decreased proportion of neuritic plaques and/or improved neurovascular function and/or improved Aβ clearance across the blood-brain-barrier, and/or enhanced autophagy and/or enhanced microglial Aβ phagocytosis.

Therefore, in a further preferred embodiment of the present invention, administration to a patient results in diminished tau pathology, in particular in reduced Tau hyperphosphorylation in the brain and/or a decrease in the proportion of neuritic plaques in the brain, in particular a decrease in the proportion of neuritic plaques in the frontal cortex.

"Prevention" of Alzheimer's disease and/or of a precursor stage of Alzheimer's disease includes that Alzheimer's disease or a precursor stage of Alzheimer's disease or at least one symptom of Alzheimer's disease or at least one symptom of a precursor stage of Alzheimer's disease does not occur, that the onset of Alzheimer's disease or of a precursor stage of Alzheimer's disease is delayed or that its severity is attenuated, or that the onset of at least one clinical symptom of the Alzheimer's disease and/or of a precursor stage of Alzheimer's disease is delayed and/or that its severity is attenuated. Preferably, at least one symptom of Alzheimer's disease which is prevented, in particular by attenuating its severity or delaying its onset, is increased anxiety and/or cognitive impairment. In case of a precursor stage of AD, cognitive impairment is mild cognitive impairment (MCI).

Accordingly, in a further preferred embodiment, prevention of Alzheimer's disease and/or of a precursor stage of Alzheimer's disease is attenuating the severity or delaying the onset of at least one clinical symptom of the Alzheimer's disease and/or of a precursor stage of Alzheimer's disease, in particular increased anxiety and/or cognitive impairment.

"Treatment" of Alzheimer's disease and/or of a precursor stage of Alzheimer's disease includes that Alzheimer's disease or a precursor stage of Alzheimer's disease or at least one symptom of Alzheimer's disease or at least one symptom of a precursor stage of Alzheimer's disease is not observed anymore, that there is stop of progression or a mitigation of Alzheimer's disease or of a precursor stage of Alzheimer's disease, or a stop of progression or a mitigation of at least one clinical symptom of the Alzheimer's disease and/or a precursor stage of Alzheimer's disease. Preferably, the at least one symptom of Alzheimer's disease which is treated is increased anxiety and/or cognitive impairment. In case of a precursor stage of AD, cognitive impairment is mild cognitive impairment (MCI).

Accordingly, in a further preferred embodiment, treatment of Alzheimer's disease and/or of a precursor stage of Alzheimer's disease is mitigation of at least one symptom of Alzheimer's disease, and/or of a precursor stage of Alzheimer's disease, in particular mitigation of increased anxiety and/or cognitive impairment.

According to Attwood et al. (2011; supra), target substrates of KLK8 for proteolytic cleavage contain the amino acid sequence YGRY (SEQ ID No: 1).

A BLAST search performed by us identified eight putative and one assured KLK8 substrates. Two of these proteins, i.e. fibronectin (Monning, U., Sandbrink, R., Weidemann, A., Banati, R.B., Masters, C.L., and Beyreuther, K. 1995. Extracellular matrix influences the biogenesis of amyloid precursor protein in microglial cells. J Biol Chem 270:7104-7110; Muenchhoff, J., Poljak, A., Song, F., Raftery, M., Brodaty, H., Duncan, M., McEvoy, M., Attia, J., Schofield, P.W., and Sachdev, P.S. 2015. Plasma protein profiling of mild cognitive impairment and Alzheimer's disease across two independent cohorts. J Alzheimers Dis 43:1355-1373); and steroid 5 alpha-reductase 1 (Guidotti, A., and Costa, E. 1998. Can the antidysphoric and anxiolytic profiles of selective serotonin reuptake inhibitors be related to their ability to increase brain 3 alpha, 5 alpha-tetrahydroprogesterone availability? Biol Psychiatry 44:865-873; Naylor, J.C., Kilts, J.D., Hulette, C.M., Steffens, D.C., Blazer, D.G., Ervin, J.F., Strauss, J.L., Allen, T.B., Massing, M.W., Payne, V.M., et al. 2010. Allopregnanolone levels are reduced in temporal cortex in patients with Alzheimer's disease compared to cognitively intact control subjects. Biochim Biophys Acta 1801:951-959) play a role in AD and are therefore candidates for further testing in the context of anti-KLK8 therapy. Cerebral fibronectin levels are reduced already before AD onset in patients with mild cognitive impairment. Allopregnanolone levels are reduced in temporal cortex in patients with Alzheimer's disease compared to cognitively intact control subjects (Wang, J.M., Singh, C., Liu, L., Irwin, R.W., Chen, S., Chung, E.J., Thompson, R.F., and Brinton, R.D. 2010. Allopregnanolone reverses neurogenic and cognitive deficits in mouse model of Alzheimer's disease. Proc Natl Acad Sci U S A 107:6498-6503) are already known to play a role in AD and it is therefore expected that an anti-KLK8 therapy will positively affect these proteins

In a particularly preferred embodiment, the agent for use of the invention which inhibits Kallikrein-8 inhibits proteolytic fragmentation by Kallikrein-8 of steroid 5 alpha-reductase 1. The sequence of human steroid 5 alpha-reductase 1 or 3-oxo-5-alpha-steroid 4-dehydrogenase 1 is shown in UniProtKB Accession number entry of P18405. The sequence of murine steroid 5 alpha-reductase 1 or 3-oxo-5-alpha-steroid 4-dehydrogenase 1 is UniProtKB accession number Q68FF9.

The further targets of Kallikrein8 identified are casein, fibronectin, collagen type IV, fibrinogen, kininogen, neuregulin-1, CAM-L1, single-chain tPA, PAR2, pro-KLK1 and pro-KLK11. The sequences of these proteins are known to a skilled person and are shown in following UniProtKB accession number entries:

| | |
|---|---|
| human Alpha-S1-casein: | P47710 |
| human Beta-casein: | P05814 |
| murine Casein alpha s2-like A: | Q547D1 |
| murine Beta-casein: | P10598 |
| human Fibronectin: | P02751 |
| murin Fibronectin: | P11276 |
| human Collagen type IV a1: | P02462 |
| human Collagen type IV a2: | P08572 |
| human Collagen type IV a3: | Q01955 |
| human Collagen type IV a4: | P53420 |
| human Collagen type IV a5: | P29400 |
| human Collagen type IV a6: | Q14031 |
| murine Collagen type IV a1: | P02463 |
| murine Collagen type IV a2: | P08122 |
| murine Collagen type IV a3: | Q9QZS0 |
| murine Collagen type IV a4: | Q9QZR9 |
| murine Collagen type IV a5: | Q61436 |
| human Fibrinogen alpha chain: | P02671 |
| human Fibrinogen beta chain: | P02675 |
| murine Fibrinogen alpha chain: | E9PV24 |
| murine Fibrinogen beta chain: | Q8K0E8 |
| human Kininogen 1, isoform CRA_b: | B4E1C2 |
| human Kininogen 1, isoform CRA_a: | D3DNU8 |
| murine Kininogen-1: | 008677 |
| human Neuregulin-1: | B9EK51 |
| murine Neuregulin-1: | Q6DR99 |
| human Neural cell adhesion molecule L1 (CAM-L1): | P32004 |
| murine Neural cell adhesion molecule L1 (CAM-L1): | P11627 |
| human Tissue-type plasminogen activator: | P00750 |
| murine Tissue-type plasminogen activator: | P11214 |
| human Proteinase-activated receptor 2: | P55085 |
| murine Proteinase-activated receptor 2: | P55086 |
| human KLK1: | P06870 |
| murine KLK1: | P15947 |
| humane KLK11: | Q9UBX7 |
| murine KLK11: | Q9QYN3 |

Therefore, in a particularly preferred embodiment, the agent for use of the invention which inhibits Kallikrein-8 inhibits proteolytic fragmentation by Kallikrein-8 of EPHB2, as shown in the Examples. Murine EPHB2 sequences are shown in SEQ ID No: 4 and 5. Human EPHB2 sequences, which are preferred, are depicted in in SEQ ID No: 6 and 7.

In a yet further preferred embodiment, the agent for use of the invention which inhibits Kallikrein-8 inhibits proteolytic fragmentation by Kallikrein-8 of at least one protein comprising the sequence YGRY (SEQ ID No: 1), preferably wherein the at least one protein is selected from EPHB2, steroid 5 alpha-reductase 1, casein, fibronectin, collagen type IV, fibrinogen, kininogen, neuregulin-1, CAM-L1, single-chain tPA, PAR2, pro-KLK1 and pro-KLK11.

For administration to a patient, the agent for use is typically comprised in a pharmaceutical composition, which preferably further comprises at least one pharmaceutically acceptable excipient. Suitable pharmaceutically acceptable excipients depend on the administration mode and are known to a skilled person. Pharmaceutical compositions are sterile and pyrogen-free. For intraventricular administration or intravenous administration, the pharmaceutical composition is preferably pharmaceutical solution, in particular a pharmaceutical saline solution. A pharmaceutical saline solution, which is preferably buffered, is preferably suitable for intraventricular administration. Intraventricular administration may be achieved e.g. using a pump, which may be implanted, a cannula or a catheter, in particular using a pump. Cannulae and catheters can be implanted stereotaxically. It is contemplated that multiple administrations over time are preferably performed. Catheters and pumps can be used separately or in combination. Intraventricular administration can be achieved e.g. by injection or infusion (which form is also possibly suitable for intravenous or intrathecal administration). Suitable pharmaceutically acceptable excipients include, for example, physiological saline, bacteriostatic water, Cremophor EL™ (BASF, Parsippany, N.J.) or phosphate buffered saline (PBS), other saline solutions, dextrose solutions, glycerol solutions, water and oils emulsions such as those made with oils of petroleum, animal, vegetable, or synthetic origin such as peanut oil, soybean oil, mineral oil, or sesame oil. The concentration of the agent in the pharmaceutical composition can vary widely, i.e., from at least about 0.01 % by weight, to 0.1 % by weight, to about 1% weight, to as much as 20% by weight or more of the total composition. Intraventricular delivery can be achieved by intraventricular injection or intraventricular infusion.

Therefore, in a yet further preferred embodiment, the agent for use of the invention is comprised in a pharmaceutical composition which further comprises at least one pharmaceutically acceptable excipient, preferably the agent is comprised in a pharmaceutical solution, in particular a pharmaceutical saline solution, in a pharmaceutical solution suitable for intraventricular administration or in a pharmaceutical composition suitable for intravenous, nasal or oral administration or by implantation into the brain.

A pharmaceutically acceptable excipient encompasses excipients such as a liquid or solid filler, diluent, solvent, or an encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, Remington: The Science and Practice of Pharmacy, 21st ed.; Lippincott Williams & Wilkins: Philadelphia, Pa., 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; and Pharmaceutical Preformulation and Formulation, 2nd ed.; Gibson Ed.; CRC Press LLC: Boca Raton, Fla., 2009.

The pharmaceutical compositions provided herein for oral administration can be provided in solid, semisolid, or liquid dosage forms. As used herein, oral administration also includes buccal, lingual, and sublingual administration. Suitable oral dosage forms include, but are not limited to, tablets, fastmelts, chewable tablets, capsules, pills, strips, troches, lozenges, pastilles, cachets, pellets, medicated chewing gum, bulk powders, effervescent or non-effervescent powders or granules, oral mists, solutions, emulsions, suspensions, wafers, sprinkles, elixirs, and syrups. In addition to the active agent(s), the pharmaceutical compositions can contain one or more pharmaceutically acceptable excipients, including, but not limited to, binders, fillers, diluents, disintegrants, wetting agents, lubricants, glidants, coloring agents, dye-migration inhibitors, sweetening agents, flavoring agents, emulsifying agents, suspending and dispersing agents, preservatives, solvents, non-aqueous liquids, organic acids, and sources of carbon dioxide.

Binders or granulators impart cohesiveness to a tablet to ensure the tablet remaining intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, Pa.); and mixtures thereof. Suitable fillers include, but are not limited to, talc, calcium carbonate, microcrystalline cellulose, powdered cellulose, dextrates, kaolin, mannitol, silicic acid, sorbitol, starch, pre-gelatinized starch, and mixtures thereof. The amount of a binder or filler in the pharmaceutical compositions provided herein varies upon the type of formulation, and is readily discernible to those of ordinary skill in the art. The binder or filler may be present from about 50 to about 99% by weight in the pharmaceutical compositions provided herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets. The amount of a diluent in the pharmaceutical compositions provided herein varies upon the type of formulation, and is readily discernible to those of ordinary skill in the art.

Suitable disintegrants include, but are not limited to, agar; bentonite; celluloses, such as methylcellulose and carboxymethylcellulose; wood products; natural sponge; cation-exchange resins; alginic acid; gums, such as guar gum and Veegum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as corn starch, potato starch, tapioca starch, and pre-gelatinized starch; clays; aligns; and mixtures thereof. The amount of a disintegrant in the pharmaceutical compositions provided herein varies upon the type of formulation, and is readily discernible to those of ordinary skill in the art. The amount of a disintegrant in the pharmaceutical compositions provided herein varies upon the type of formulation, and is readily discernible to those of ordinary skill in the art.

The pharmaceutical compositions provided herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, corn oil, and soybean oil; zinc stearate; ethyl oleate; ethyl laureate; agar; starch; lycopodium; silica or silica gels, such as AEROSIL® 200 (W.R. Grace Co., Baltimore, Md.) and CAB-O-SIL® (Cabot Co. of Boston, Mass.); and mixtures thereof. The pharmaceutical compositions provided herein may contain about 0.1 to about 5% by weight of a lubricant.

Suitable glidants include, but are not limited to, colloidal silicon dioxide, CAB-O-SIL® (Cabot Co. of Boston, Mass.), and asbestos-free talc. Suitable coloring agents include, but are not limited to, any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Suitable flavoring agents include, but are not limited to, natural flavors extracted from plants, such as fruits, and synthetic blends of compounds which produce a pleasant taste sensation, such as peppermint and methyl salicylate. Suitable sweetening agents include, but are not limited to, sucrose, lactose, mannitol, syrups, glycerin, and artificial sweeteners, such as saccharin and aspartame. Suitable emulsifying agents include, but are not limited to, gelatin, acacia, tragacanth, bentonite, and surfactants, such as polyoxyethylene sorbitan monooleate (TWEEN® 20), polyoxyethylene sorbitan monooleate 80 (TWEEN® 80), and triethanolamine oleate. Suitable suspending and dispersing agents include, but are not limited to, sodium carboxymethylcellulose, pectin, tragacanth, Veegum, acacia, sodium carbomethylcellulose, hydroxypropyl methylcellulose, and polyvinylpyrrolidone. Suitable preservatives include, but are not limited to, glycerin, methyl and propylparaben, benzoic add, sodium benzoate and alcohol. Suitable wetting agents include, but are not limited to, propylene glycol monostearate, sorbitan monooleate, diethylene glycol monolaurate, and polyoxyethylene lauryl ether. Suitable solvents include, but are not limited to, glycerin, sorbitol, ethyl alcohol, and syrup. Suitable non-aqueous liquids utilized in emulsions include, but are not limited to, mineral oil and cottonseed oil. Suitable organic acids include, but are not limited to, citric and tartaric acid. Suitable sources of carbon dioxide include, but are not limited to, sodium bicarbonate and sodium carbonate.

The pharmaceutical compositions provided herein for oral administration can be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenyl salicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms can be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein for oral administration can be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient, i.e. the agent for use of the invention.

The pharmaceutical compositions provided herein for oral administration can be provided in liquid and semisolid dosage forms, including emulsions, solutions, suspensions, elixirs, and syrups.

The pharmaceutical compositions provided herein for oral administration can be also provided in the forms of liposomes, micelles, microspheres, or nanosystems. Micellar dosage forms can be prepared as described in U.S. Pat. No. 6,350,458.

The pharmaceutical compositions provided herein for oral administration can be provided as non-effervescent or effervescent, granules and powders, to be reconstituted into a liquid dosage form.

The pharmaceutical compositions provided herein for oral administration can be formulated as immediate or modified release dosage forms, including delayed-, sustained, pulsed-, controlled, targeted-, and programmed-release forms.

The pharmaceutical compositions provided herein can be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, intravesical, and subcutaneous administration.

The pharmaceutical compositions provided herein for parenteral administration can be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (see, Remington: The Science and Practice of Pharmacy, supra).

The pharmaceutical compositions intended for parenteral administration can include one or more pharmaceutically acceptable excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Suitable non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Suitable water-miscible vehicles include, but are not limited to, ethanol, 1,3-butanediol, liquid polyethylene glycol (e.g., polyethylene glycol 300 and polyethylene glycol 400), propylene glycol, glycerin, N-methyl-2-pyrrolidone, N,N-dimethylacetamide, and dimethyl sulfoxide.

Suitable antimicrobial agents or preservatives include, but are not limited to, phenols, cresols, mercurials, benzyl alcohol, chlorobutanol, methyl and propyl p-hydroxybenzoates, thimerosal, benzalkonium chloride (e.g., benzethonium chloride), methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents are those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including α-cyclodextrin, β-cyclodextrin, hydroxypropyl-β-cyclodextrin, sulfobutylether-β-cyclodextrin, and sulfobutylether 7-β-cyclodextrin.

In one embodiment, the pharmaceutical compositions for parenteral administration are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions provided herein for parenteral administration can be formulated as immediate or modified release dosage forms, including delayed-, sustained, pulsed-, controlled, targeted-, and programmed-release forms.

The pharmaceutical compositions provided herein for parenteral administration can be formulated as a suspension, solid, semi-solid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include, but are not limited to, polymethylmethacrylate, polybutyl-methacrylate, plasticized or unplasticized polyvinylchloride, plasticized nylon, plasticized polyethylene terephthalate, natural rubber, polyisoprene, polyisobutylene, polybutadiene, polyethylene, ethylene-vinyl acetate copolymers, silicone rubbers, polydimethylsiloxanes, silicone carbonate copolymers, hydrophilic polymers, such as hydrogels of esters of acrylic and methacrylic acid, collagen, cross-linked polyvinyl alcohol, and cross-linked partially hydrolyzed polyvinyl acetate.

Suitable outer polymeric membranes include but are not limited to, polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinyl chloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

The pharmaceutical compositions provided herein can be administered topically to the skin, orifices, or mucosa. The topical administration, as used herein, includes (intra)dermal, conjunctival, intracorneal, intraocular, ophthalmic, auricular, transdermal, nasal, vaginal, urethral, respiratory, pulmonary, and rectal administration, in particular by nasal delivery.

The pharmaceutical compositions provided herein can be formulated in any dosage forms including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, and dermal patches. The topical formulation of the pharmaceutical compositions provided herein can also comprise liposomes, micelles, microspheres, nanosystems, and mixtures thereof.

Pharmaceutically acceptable carriers and excipients suitable for use in the topical formulations provided herein include, but are not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The pharmaceutical compositions provided herein can be administered in one preferred embodiment intranasally. The pharmaceutical compositions can be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions can also be provided as a dry powder for insufflation, alone or in combination with an inert carrier such as lactose or phospholipids; and nasal drops. For intranasal use, the powder can comprise a bioadhesive agent, including chitosan or cyclodextrin.

Solutions or suspensions for use in a pressurized container, pump, spray, atomizer, or nebulizer can be formulated to contain ethanol, aqueous ethanol, or a suitable alternative agent for dispersing, solubilizing, or extending release of the active ingredient provided herein; a propellant as solvent; and/or a surfactant, such as sorbitan trioleate, oleic acid, or an oligolactic acid.

The pharmaceutical compositions provided herein can be micronized to a size suitable for delivery by inhalation, such as about 50 micrometers or less, or about 10 micrometers or less. Particles of such sizes can be prepared using a comminuting method known to those skilled in the art, such as spiral jet milling, fluid bed jet milling, supercritical fluid processing to form nanoparticles, high pressure homogenization, or spray drying.

Pharmaceutical compositions suitable for implantation into the brain are known to a skilled person and include gels, such as hydrogels, sponges and/or polymers which allow release of an agent for use of the invention to brain tissue. Such gels, such as hydrogels, sponges and/or polymers are preferably biocompatible. Further, they may be biodegradable or non-biodegradable. Preferably, the gels, such as hydrogels, sponges and/or polymers allow for sustained or extended release of an agent. Such gels, such as hydrogels, sponges and/or polymers are known to a skilled person. For example, composites of bio-compatible polymers, such as poly(lactic acid), poly(lactic-co-glycolic acid), methylcellulose, hyaluronic acid, collagen, and the like may be used. The structure, selection and use of degradable polymers in drug delivery vehicles have been reviewed in several publications, including, A. Domb et al., Polymers for Advanced Technologies 3:279-292 (1992). Additional guidance in selecting and using polymers in pharmaceutical formulations can be found in the text by M. Chasin and R. Langer (eds.), "Biodegradable Polymers as Drug Delivery Systems", Vol. 45 of "Drugs and the Pharmaceutical Sciences", M. Dekker, New York, 1990, and U.S. Pat. No. 5,573,528 to Aebischer et al. (issued Nov. 12, 1996). For example, ethylene vinyl acetate is dissolved in methylene chloride (10% w/v), the agent for use is added in the desired concentration and the resulting emulsion is then shock-frozen, lyophilized and extruded into tubes at 50° C.

Accordingly, the present disclosure relates a pharmaceutical composition comprising an agent which inhibits Kallikrein-8 and at least one pharmaceutically acceptable excipient, preferably wherein the pharmaceutical composition is a pharmaceutical solution, in particular a pharmaceutical saline solution, a pharmaceutical solution suitable for intraventricular administration, or wherein the pharmaceutical composition is suitable for intravenous, nasal or oral administration or administration by implantation into the brain. Preferred disclosure for an agent which inhibits Kallikrein-8 as well as preferred pharmaceutical compositions are described above and apply to this disclosure.

Further, we could surprisingly show that elevated levels of Kallikrein 8 protein and kallikrein 8 mRNA are present in diseased human and mice, and that such elevated levels are already measured at a time point in transgenic mice at which no clinical symptoms of AD-like pathology are detectable yet. Accordingly, the determination of Kallikrein 8 protein and/or kallikrein 8 mRNA in an appropriate bodily sample of an individual who exhibits cognitive impairment, such as mild cognitive impairment, allows for prediction and/or diagnosis. In particular, about 70% of individuals with MCI develop AD. For example, determining Kallikrein 8 protein and/or kallikrein 8 mRNA levels, either in vitro, in an appropriate bodily sample of an MCI individual, or in vivo using an imaging methods and the finding that Kallikrein 8 protein and/or kallikrein 8 mRNA levels are elevated as compared to a healthy reference population, allows the prediction that the individual will very likely develop Alzheimer's disease. For example, determining Kallikrein 8 protein and/or kallikrein 8 mRNA levels in an appropriate bodily sample of an individual suffering from cognitive impairment and the finding that Kallikrein 8 protein and/or kallikrein 8 mRNA levels are elevated as compared to a healthy reference population, allows the diagnosis that the patient has Alzheimer's disease and/or the prediction that the individual will very likely progress in Alzheimer's disease. Such levels may be determined in vitro or in vivo.

Therefore, both Kallikrein 8 protein and kallikrein 8 mRNA were surprisingly identified as biomarkers for identifying individuals with a risk for developing Alzheimer's disease.

Therefore, the present disclosure relates to a compound specifically binding to Kallikrein-8 protein or a probe specifically recognizing kallikrein-8 mRNA for use in the prediction and/or diagnosis of Alzheimer's disease and/or of a precursor stage of Alzheimer's disease of an individual exhibiting cognitive impairment, preferably in the prediction and/or diagnosis of a precursor stage of Alzheimer's disease of an individual exhibiting cognitive impairment.

Therefore, the present disclosure relates to a compound specifically binding to Kallikrein-8 protein or a probe specifically recognizing kallikrein-8 mRNA for use for use in the identification and/or stratification of at least one individual to be responsive to treatment or prevention of Alzheimer's disease, and/or precursor stages of Alzheimer's disease with an agent which inhibits Kallikrein-8.

Preferably, the compound or probe is bound to a detectable label. For in vivo diagnostic applications, such label may be any label which can be detected by imaging methods, such a PET tracer, a moiety useable in MRI or a moiety detectable in X-ray-based methods. For example, an agent such as a small molecule or antibody may be bound to an iodine atom or an iodine radioisotope, and the iodine atom or an iodine radioisotope, respectively, may be detected by an X-ray based in vivo imaging method.

In a further disclosure, a compound specifically binding to Kallikrein-8 protein or of a probe specifically recognizing kallikrein-8 mRNA may be used in the in vitro diagnosis or prediction. Such compounds and probes may be used for detecting and measuring the level of Kallikrein 8 protein or kallikrein 8 mRNA in a bodily sample of an individual. Typically, such individual exhibits cognitive impairment, such as MCI, and is therefore suspected to have Alzheimer's disease or a precursor stage thereof. Various bodily samples may be used, like a cerebrospinal fluid sample, blood sample, such as plasma sample, whole blood sample or serum sample, urine sample or saliva sample, or a biopsy, such as a brain tissue biopsy. In a more preferred disclosure, a brain tissue biopsy. In particular, a blood sample, a cerebrospinal fluid sample or a brain tissue biopsy may be used, even more preferably the bodily fluid sample is a cerebrospinal fluid sample.

Further, a compound specifically binding to Kallikrein-8 protein may be used for diagnostic purposes. For diagnostic purposes, it is not required that the compound inhibits Kallikrein 8, in particular inhibits the proteolytic activity of Kallikrein 8. Rather, it is merely required that the compound specifically binds to Kallikrein-8 protein, as described above. For example, an antibody or functionally active part thereof, such as MabB5 used in the Examples may be used. However, it is possible to use a compound which inhibits Kallikrein 8, in particular which inhibits the proteolytic activity of Kallikrein 8 also for diagnostic purposes. Accordingly, an agent for use according to the present invention may be used both for treatment or prevention and for diagnostic purposes. For the diagnostic purposes, such compound or probe may further be bound to a detectable label which may not be present for treatment or prevention purposes.

The present disclosure relates to the use of a compound specifically binding to Kallikrein-8 protein or of a probe specifically recognizing kallikrein-8 mRNA for the *in vitro* prediction and/or *in vitro* diagnosis of Alzheimer's disease and/or of a precursor stage of Alzheimer's disease. Typically, in vitro diagnosis is performed in a bodily sample of an individual.

Therefore, the present disclosure relates to the use of a compound specifically binding to Kallikrein-8 protein or of a probe specifically recognizing kallikrein-8 mRNA for the *in vitro* prediction and/or *in vitro* diagnosis of Alzheimer's disease and/or of a precursor stage of Alzheimer's disease in a bodily sample of an individual exhibiting cognitive impairment. In a more preferred disclosure, the present disclosure relates to above use for the prediction and/or diagnosis of a precursor stage of Alzheimer's disease.

For the *in vitro* diagnostic, predictive and stratification purposes, the bodily sample is preferably selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample.

Moreover, the detection and determination of Kallikrein 8 protein levels and/or kallikrein 8 mRNA levels allow for the identification and thereby stratification of individuals for such individuals which are responsive to treatment with an agent for use of the invention. In particular, individuals, which exhibit elevated levels of Kallikrein 8 protein and/or kallikrein 8 mRNA will likely respond to administration of an agent for use of the invention, in particular of a therapeutically effective amount of an agent for use of the invention.

Disclosed is the use of a compound specifically binding to Kallikrein-8 protein or of a probe specifically recognizing kallikrein-8 mRNA for the identification and/or stratification of at least one individual to be responsive to treatment or prevention of Alzheimer's disease, and/or precursor stages of Alzheimer's disease with an agent which inhibits Kallikrein-8.

Disclosed is an *in vitro* method of predicting and/or diagnosing Alzheimer's disease and/or a precursor stage of Alzheimer's disease,
comprising the steps of:
(1) detecting the level of Kallikrein-8 protein and/or kallikrein-8 mRNA in a bodily sample of an individual exhibiting cognitive impairment, and
(2) comparing the level determined in step (1) with the level(s) determined in one or more reference samples from healthy individuals and/or from patients known to have Alzheimer's disease and/or a precursor stage of Alzheimer's disease,
wherein an increased level determined in step (1) compared to the level(s) determined in one or more reference samples from healthy individuals, and/or a level determined in step (1) which is identical or similar to the level(s) determined in one or more reference samples from patients known to have Alzheimer's disease and/or a precursor stage of Alzheimer's disease indicates that:
(i) the individual has Alzheimer's disease and/or a precursor stage of Alzheimer's disease, and/or
(ii) the individual has high risk for developing Alzheimer's disease.

The one or more reference samples from healthy individuals may be 1, 2, 3, 4, 5, 6, 7, 8, 9, 10, 50, 100, 1000 or more reference samples. The reference sample levels may be determined in parallel to the bodily sample under investigation, or consecutively. Also, the one or more reference samples may be analyzed at some time point in the past and the results may be used for later analysis. The level(s) determined in one or more reference samples may be used for determining a cut-off value, based on a mean or median value.

The one or more reference samples from patients known to exhibit Alzheimer's disease and/or a precursor stage of Alzheimer's disease may be 1, 2, 3, 4, 5, 6, 7, 8, 9, 10, 50, 100, 1000 or more reference samples. The reference sample levels may be determined in parallel to the bodily sample under investigation, or consecutively. Also, the one or more reference samples may be analyzed at some time point in the past and the results may be used for later analysis. The level(s) determined in one or more reference samples may be used for determining a cut-off value, based on a mean or median value.

The reference samples are typically obtained from age- and/or sex-matched individuals.

Typically, statistical methods known to a skilled person may be applied to determine suitable cut-off values to achieve suitable sensitivity at a given specificity and specificity at a given sensitivity.

Therefore, in one preferred embodiment, the level(s) determined in one or more reference samples is understood to represent a cut-off value.

In particular, such method is applied to patients for whom it is not yet known whether they have AD or will develop AD in the future. Accordingly, the method is preferably applied to an individual who exhibits cognitive impairment, such as MCI, but was not yet diagnosed to have AD.

Accordingly, in a preferred embodiment, the individual was not yet diagnosed to exhibit Alzheimer's disease and/or a precursor stage of Alzheimer's disease and/or a preclinical stage of Alzheimer's disease.

Preferably, the bodily sample is selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample.

Accordingly, the present disclosure relates to an *in vitro* method of identifying and/or stratifying of at least one individual to be responsive to treatment or prevention of Alzheimer's disease, and/or precursor stages of Alzheimer's disease with an agent which inhibits Kallikrein-8, comprising the steps of:
(1) detecting the level of Kallikrein-8 protein and/or kallikrein-8 mRNA in a bodily sample of an individual, and
(2) comparing the level determined in step (1) with the level(s) determined in one or more reference samples from healthy individuals and/or from patients known to exhibit Alzheimer's disease and/or a precursor stage of Alzheimer's disease, or known to be responsive to treatment or prevention of Alzheimer's disease and/or precursor stages of Alzheimer's disease with an agent which inhibits Kallikrein-8,
wherein
an increased level determined in step (1) compared to the level(s) determined in one or more reference samples from healthy individuals, and/or
a level determined in step (1) which is identical or similar to the level(s) determined in one or more reference samples from patients
- known to exhibit Alzheimer's disease and/or a precursor stage of Alzheimer's disease or a preclinical stage of Alzheimer's disease, and/or
- known to be responsive to treatment or prevention of Alzheimer's disease, and/or precursor stages of Alzheimer's disease, with an agent which inhibits Kallikrein-8
indicates that the individual is responsive to treatment or prevention of Alzheimer's disease, and/or precursor stages of Alzheimer's disease with an agent which inhibits Kallikrein-8.

The one or more reference samples from healthy individuals may be 1, 2, 3, 4, 5, 6, 7, 8, 9, 10, 50, 100, 1000 or more reference samples. The reference sample levels may be determined in parallel to the bodily sample under investigation, or consecutively. Also, the one or more reference samples may be analyzed at some time point in the past and the results may be used for later analysis. The level(s) determined in one or more reference samples may be used for determining a cut-off value, based on a mean or median value.

The one or more reference samples from patients known to exhibit Alzheimer's disease and/or a preclinical stage of Alzheimer's disease or a precursor stage of Alzheimer's disease, or known to be responsive to treatment or prevention of Alzheimer's disease, and/or precursor stages of Alzheimer's disease with an agent which inhibits Kallikrein-8 may be 1, 2, 3, 4, 5, 6, 7, 8, 9, 10, 50, 100, 1000 or more reference samples. The reference sample levels may be determined in parallel to the bodily sample under investigation, or consecutively. Also, the one or more reference samples may be analyzed at some time point in the past and the results may be used for later analysis. The level(s) determined in one or more reference samples may be used for determining a cut-off value, based on a mean or median value.

Preferably wherein the bodily sample is selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample.
wherein
an increased level determined in step (1) compared to the level(s) determined in one or more reference samples from healthy individuals, and/or
a level determined in step (1) which is identical or similar to the level(s) determined in one or more reference samples from patients
- known to have Alzheimer's disease, and/or
- known to be responsive to treatment or prevention of Alzheimer's disease with an antibody or functionally active part thereof which specifically binds to Kallikrein-8 and inhibits the proteolytic activity of Kallikrein-8
indicates that the individual is responsive to treatment or prevention of Alzheimer's disease, with the antibody or functionally active part thereof.

The one or more reference samples from healthy individuals may be 1, 2, 3, 4, 5, 6, 7, 8, 9, 10, 50, 100, 1000 or more reference samples. The reference sample levels may be determined in parallel to the bodily sample under investigation, or consecutively. Also, the one or more reference samples may be analyzed at some time point in the past and the results may be used for later analysis. The level(s) determined in one or more reference samples may be used for determining a cut-off value, based on a mean or median value.

The one or more reference samples from patients known to exhibit Alzheimer's disease and/or a preclinical stage of Alzheimer's disease or a precursor stage of Alzheimer's disease, or known to be responsive to treatment or prevention of Alzheimer's disease, and/or precursor stages of Alzheimer's disease with an agent which inhibits Kallikrein-8 may be 1, 2, 3, 4, 5, 6, 7, 8, 9, 10, 50, 100, 1000 or more reference samples. The reference sample levels may be determined in parallel to the bodily sample under investigation, or consecutively. Also, the one or more reference samples may be analyzed at some time point in the past and the results may be used for later analysis. The level(s) determined in one or more reference samples may be used for determining a cut-off value, based on a mean or median value.

Preferably wherein the bodily sample is selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample.

The term "detectable label" or "label" as used herein refers to any substance that is capable of producing a signal for direct or indirect detection. The detectable label thus may be detected directly or indirectly. For direct detection label suitable for use in the present invention can be selected from any known detectable marker groups, like chromogens, fluorescent groups, chemiluminescent groups (e.g. acridinium esters or dioxetanes), electrochemiluminescent compounds, catalysts, enzymes, enzymatic substrates, dyes, fluorescent dyes (e.g. fluorescein, coumarin, rhodamine, oxazine, resorufin, cyanine and derivatives thereof), colloidal metallic and nonmetallic particles, and organic polymer latex particles. Other examples of detectable labels are luminescent metal complexes, such as ruthenium or europium complexes, e.g. as used for ECLIA, enzymes, e.g. as used for ELISA, and radioisotopes; e.g. as used for RIA. For *in vivo* applications, the label or detectable label is suitable for in vivo imaging, for example may be a PET tracer or an iodine radioisotope.

Indirect detection systems comprise, for example, that the detection molecule, e.g. an antibody or functionally active fragment thereof specifically binding to Kallikrein 8, is labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g. steroid hormone receptor/steroid hormone. Preferred first binding pair members comprise hapten, antigen and hormone. Also preferred are haptens like digoxin and biotin and analogues thereof. The second partner of such binding pair, e.g. an antibody, streptavidin, etc., usually is labeled to allow for direct detection, e.g. by the detectable labels as mentioned above.

The measured level of Kallikrein 8 may be a primary measurement of the level of the quantity of Kallikrein 8 itself, such as by detecting the number or concentration of Kallikrein 8 molecules in the sample, or it may be a secondary measurement of the biomarker Kallikrein 8 protein, such as a measure of proteolytic activity.

Although some assay formats will allow testing of bodily samples, such as bodily fluid samples without prior processing of the sample, it is expected that most peripheral biological fluid samples will be processed prior to testing. Processing generally takes the form of elimination of cells (nucleated and non-nucleated), such as erythrocytes, leukocytes, and platelets in blood samples, and may also include the elimination of certain proteins, such as certain clotting cascade proteins from blood. For example, a blood sample is collected in a container comprising EDTA. In a further preferred embodiment, a CSF sample is a native CSF sample. In a further preferred embodiment, a biopsy, in particular a brain biopsy, is a native biopsy.

Commonly, Kallikrein 8 protein levels will be measured using an affinity-based measurement technology. "Affinity" as relates to an antibody is a term well understood in the art and means the extent, or strength, of binding of antibody to the binding partner, in the present case Kallikrein 8 protein (or epitope thereof). Affinity may be measured and/or expressed in a number of ways known in the art, including, but not limited to, equilibrium dissociation constant (KD or Kd), apparent equilibrium dissociation constant (KD' or Kd'), and IC50 (amount needed to effect 50% inhibition in a competition assay; used interchangeably herein with "150"). It is understood that, for purposes of this invention, an affinity is an average affinity for a given population of antibodies which bind to an epitope.

Affinity-based measurement technology utilizes a molecule that specifically binds to the molecule being measured (an "affinity reagent" such as an antibody or aptamer), although other technologies, such as spectroscopy-based technologies (e.g., matrix-assisted laser desorption ionization-time of flight, or MALDI-TOF, spectroscopy) or assays measuring bioactivity (e.g., assays measuring enzymatic activity) may be used.

Affinity-based technologies include antibody-based assays (immunoassays) and assays utilizing aptamers (nucleic acid molecules which specifically bind to other molecules), such as ELONA. Additionally, assays utilizing both antibodies and aptamers are also contemplated (e.g., a sandwich format assay utilizing an antibody for capture and an aptamer for detection).

If immunoassay technology is employed, any immunoassay technology which can quantitatively or qualitatively measure the level of a Kallikrein 8 in a biological sample may be used. Suitable immunoassay technology includes radioimmunoassay, immunofluorescent assay, enzyme immunoassay, chemiluminescent assay, ELISA, immuno-PCR, and western blot assay.

Likewise, aptamer-based assays which can quantitatively or qualitatively measure the level of Kallikrein 8 in a bodily sample may be used in the methods and uses of the invention. Generally, aptamers may be substituted for antibodies in nearly all formats of immunoassay, although aptamers allow additional assay formats (such as amplification of bound aptamers using nucleic acid amplification technology such as PCR (U.S. Pat. No. 4,683,202) or isothermal amplification with composite primers (U.S. Pat. Nos. 6,251,639 and 6,692,918).

A wide variety of affinity-based assays are known in the art. Affinity-based assays will utilize at least one epitope derived from the biomarker Kallikrein 8 of interest, and many affinity-based assay formats utilize more than one epitope (e.g., two or more epitopes are involved in "sandwich" format assays; at least one epitope is used to capture the marker, and at least one different epitope is used to detect the marker).

Affinity-based assays may be in competition or direct reaction formats, utilize sandwich-type formats, and may further be heterogeneous (e.g., utilize solid supports) or homogenous (e.g., take place in a tingle phase) and/or utilize or immunoprecipitation. Most assays involve the use of labeled affinity reagent (e.g., antibody, polypeptide, or aptamer); the labels may be, for example, enzymatic, fluorescent, chemiluminescent, radioactive, or dye molecules. Assays which amplify the signals from the probe are also known; examples of which are assays which utilize biotin and avidin, and enzyme-labeled and mediated immunoassays, such as ELISA and ELONA assays.

In a heterogeneous format, the assay utilizes two phases (typically aqueous liquid and solid). Typically a Kallikrein 8-specific affinity reagent, such as an anti-Kallikrein 8 antibody, is bound to a solid support to facilitate separation of Kallikrein 8 from the bulk of the bodily sample. After reaction for a time sufficient to allow for formation of affinity reagent/Kallikrein 8 complexes, the solid support or surface containing the antibody is typically washed prior to detection of bound polypeptides. The affinity reagent in the assay for measurement of Kallikrein 8 may be provided on a support (e.g., solid or semi-solid); alternatively, the polypeptides in the sample can be immobilized on a support or surface. Examples of supports that can be used are nitrocellulose (e.g., in membrane or microtiter well form), polyvinyl chloride (e.g., in sheets or microtiter wells), polystyrene latex (e.g., in beads or microtiter plates), polyvinylidine fluoride, diazotized paper, nylon membranes, activated beads, glass and Protein A beads. Both standard and competitive formats for these assays are known in the art.

Array-type heterogeneous assays are suitable for measuring levels of AD biomarkers in addition to Kallikrein 8. Array-type assays will commonly utilize a solid substrate with two or more capture reagents specific for Kallikrein 8 and the different further AD biomarkers bound to the substrate in a predetermined pattern (e.g., a grid). The bodily sample is applied to the substrate and Kallikrein 8 and the further AD biomarkers in the sample are bound by the capture reagents. After removal of the sample (and appropriate washing), the bound Kallikrein 8 and the further AD biomarkers are detected using a mixture of appropriate detection reagents that specifically bind Kallikrein 8 and the various AD biomarkers. Binding of the detection reagent is commonly accomplished using a visual system, such as a fluorescent dye-based system. Because the capture reagents are arranged on the substrate in a predetermined pattern, array-type assays provide the advantage of detection of Kallikrein 8 and the further multiple AD biomarkers without the need for a multiplexed detection system.

In a homogeneous format the assay takes place in single phase (e.g., aqueous liquid phase). Typically, the biological sample is incubated with an affinity reagent, such as an antibody, specific for the Kallikrein 8 in solution. For example, it may be under conditions that will precipitate any affinity reagent/antibody complexes which are formed. Both standard and competitive formats for these assays are known in the art.

In a standard (direct reaction) format, the level of Kallikrein 8/affinity reagent complex is directly monitored. This may be accomplished by, for example, determining the amount of a labeled detection reagent that forms is bound to Kallikrein 8/affinity reagent complexes. In a competitive format, the amount of Kallikrein 8 in the sample is deduced by monitoring the competitive effect on the binding of a known amount of labeled Kallikrein 8 (or other competing ligand) in the complex. Amounts of binding or complex formation can be determined either qualitatively or quantitatively.

For a non-competitive assay or sandwich assay, two different antibodies or functionally active fragments thereof are needed, which bind to the same antigen and which do not hinder each other when binding to the antigen. Non-competitive assays or sandwich assays are advantageous over competitive assays due to their higher sensitivity. In case of a sandwich assay, one of the antibodies can be immobilized to a support. Upon addition of a probe solution, the antigen therein (i.e. Kallikrein 8) binds to the capture antibody, and the detection antibody can bind to a different binding site of the analyte Kallikrein-8. For detection of the capture antibody - Kallikrein-8 complex, the detection antibody is used.

In a preferred embodiment, the sandwich assay is a sandwich immunoassay, in particular, an enzyme-linked immunoassay (ELISA).

The use of a calibrator is often employed in immunoassays. Calibrators are solutions that are known to contain the analyte in question, and the concentration of that analyte is generally known. Comparison of an assay's response to a real sample against the assay's response produced by the calibrators makes it possible to interpret the signal strength in terms of the presence or concentration of analyte in the sample.

Suitable sandwich assays other than ELISA are (electro-) chemo luminescence immunoassay (ECLIA), radioimmunoassay (RIA), fluorescence immunoassay (FIA), Microparticle capture enzyme immunoassay (MEIA), Solid-phase fluorescence immunoassays (SPFIA), Particle concentration fluorescence immunoassay (PCFIA), Nephelometric and Turbidimetric assay with and without latex particle enhancement (LPIA). Also, the assay may be in the form of test strips.

The compound which specifically binds to Kallikrein-8 protein may bind to Kallikrein-8 in its proteolytically active form, and/or to its inactive pro-form or pre-pro-form.

In a preferred embodiment, the compound which specifically binds to Kallikrein 8 is selected from a small molecule, a ribozyme, a peptide and a protein. In a further more preferred embodiment, the compound which specifically binds to Kallikrein 8 is selected from a protein, more preferably an antibody or functionally active part thereof or an antibody mimetic, even more preferably the compound is selected from a monoclonal antibody, chimeric antibody, human antibody, humanized antibody, Fab, a Fab', a F(ab')2, a Fv, a disulfide-linked Fv, a scFv, a (scFv)2, a bivalent antibody, a bispecific antibody, a multispecific antibody, a diabody, a triabody, a tetrabody and a minibody, and/or the compound which specifically binds to Kallikrein-8 is monoclonal antibody MabB5 or functionally active part thereof, or an agent, in particular selected from an antibody or functionally active part thereof and an antibody mimetic, binding to the same epitope as MabB5.

The compound which specifically binds to Kallikrein-8 is preferably bound to a detectable label.

In a more preferred embodiment, the compound which specifically binds to Kallikrein-8 further has one or more features of an agent for use of the invention above.

A suitable probe for above uses and methods of the invention may comprise or consist of an oligonucleotide or oligonucleotide analogue as described above. For diagnostic, predictive and stratification purposes, the probe does not need to be capable of reducing the Kallikrein-8 levels. Rather, it is merely required that the probe specifically binds to Kallikrein-8 mRNA. Typically, such oligonucleotide or oligonucleotide analogue is capable of specifically hybridizing to Kallikrein-8 mRNA under stringent conditions. In a further preferred embodiment, a suitable probe comprises or consists of an oligonucleotide or oligonucleotide as described above and is bound to a detectable label.

The level of kallikrein 8 mRNA may be determined by methods known in the art, in particular using a probe. Suitable probes are in particular oligonucleotides or oligonucleotide derived molecules which are capable to specifically hybridize to Kallikrein-8 mRNA under stringent conditions.

In a preferred embodiment, Kallikrein-8 mRNA has a sequence of any of SEQ ID Nos 8 to 13, preferably 9 to 13, more preferably the coding regions of any of SEQ ID Nos 9 to 13.

In a preferred embodiment, the probe comprises or consists of, preferably consists of an oligonucleotide or oligonucleotide analogue, preferably oligonucleotide having a sequence identity of at least 80 %, 90%, 91%, 92%, 93%, 94%, 95%, 96%, 97%, 98%, 99%, or a sequence identity of 100% to any of SEQ ID Nos 8 to 13, preferably 9 to 13, more preferably the coding regions of any of SEQ ID Nos 9 to 13, preferably wherein the probe is bound to a detectable label. Such detectable label may be attached covalently or non-covalently to non-specifically binding portions of a probe, which may be optionally present at one or both terminal ends, or may be attached covalently or non-covalently to the specifically binding portions of a probe. For example, a probe may be biotinylated.

In a preferred embodiment, probe consists of an oligonucleotide having a sequence identity of at least 80 %, 90%, 91%, 92%, 93%, 94%, 95%, 96%, 97%, 98%, 99%, or a sequence identity of 100% to any of SEQ ID Nos 8 to 13, preferably 9 to 13, more preferably the coding regions of any of SEQ ID Nos 9 to 13, preferably wherein the probe is bound to a detectable label.

The probe length may vary. Typical probe lengths are 10, 15, 16, 17, 18, 19, 20, 21, 22, 23, 24, 25, 26, 27, 28, 29, 30 or more nucleotides, such as up to 50, 100, 150, or 200 nucleotides.

Kallikrein-8 mRNA may be detected in situ, by methods known in the art, such as *in situ* hybridization methods, such as FISH and chromogenic *in situ* hybridization (CISH). In these applications, the probe is typically bound to a detectable label itself or a binding partner of a bioaffine binding pair. In the latter case, the second partner of a bioaffine binding pair comprises a detectable label, such as a fluorescent or chromogenic label, depending on the application. Alternatively, the level of mRNA may be determined using amplification methods, such as PCR, optionally using a reverse transcription step.

Kallikrein-8 and kallikrein-8 mRNA may be used as biomarkers for the diagnosis of AD either alone, or as biomarker combination. As a biomarker combination, Kallikrein-8 and/or kallikrein-8 mRNA may be used together with one or more further biomarkers for the diagnosis of AD. A number of suitable biomarkers in the context of AD are known in the art.

Further, it is preferred to include further biomarkers in a kit. Preferably, a biomarker combination is thereby obtained, which provides for improved diagnostic and predictive suitability. Although no biomarker is currently available to clearly diagnose or predict Alzheimer's disease, a number of biomarkers are available, which show a certain diagnostic or predictive potential. These include Aβ and isoforms thereof, and combinations of Aβ and isoforms thereof, Tau and isoforms thereof, and combinations of Tau and isoforms thereof, as well as miRNA107. In particular, it has been shown that miRNA107 expression in plasma has a high capability to discriminate between patients with amnestic mild cognitive impairment and healthy controls (Wang T. et al., J Clin Psychiatry. 2015 Feb;76(2):135-41). Further, it has been recently shown that a CSF biomarker panel, and combined assessment of Aβ1-42, T-tau, and P-tau181P renders, to present date, the highest diagnostic power to discriminate between AD and non-AD dementias (Struyfs H. et al.; Front Neurol. 2015, 17;6:138).

Further, Neurogranin was recently shown to be a biomarker in CSF in the field of AD (Zetterberg, H and Blennow K., JAMA Neurol. 2015, 14:1-7).

In particular, one or more compound(s) capable of detecting Tau phosphorylated at threonine 181 (P-tau181P), one or more compound(s) capable of detecting total Tau protein (T-tau) and/or one or more compound(s) capable of detecting Aβ1-42 may be used in combination together with a compound specifically binding to Kallikrein-8 protein or a probe specifically recognizing kallikrein-8 mRNA. Such combination and kit is in particular useful for in vitro applications for CSF samples.

Compound(s) capable of detecting at least one biomarker, such as P-tau181P, T-tau, miRNA107, Neurogranin and Aβ1-42 are known in the art and are for example described in the Examples, in Wang T. et al., supra and in Struyfs et al., supra).

The present disclosure relates to a kit comprising:
(a) a compound specifically binding to Kallikrein-8 protein or a probe specifically recognizing kallikrein-8 mRNA,
   and
(b) one or more compound(s) capable of detecting at least one biomarker for the prediction and/or diagnosis of Alzheimer's disease and/or a precursor stage of Alzheimer's disease,

In a disclosure, the at least one biomarker is for *in vitro* diagnosis, in particular for *in vitro* diagnosis in a bodily sample selected from cerebrospinal fluid sample, blood sample, such as plasma sample, whole blood sample and serum sample, urine sample and saliva sample, and a biopsy, in particular a brain tissue biopsy.

In a disclosure, the at least one biomarker is selected from Aβ and isoforms thereof, such as Aβ1-42 and combinations of Aβ and isoforms thereof, in particular including Aβ1-42, Tau, in particular total Tau protein, and isoforms thereof, in particular P-tau181P, and combinations of Tau and isoforms thereof, in particular a combination comprising total Tau protein and P-tau181P, and miRNA107 and Neurogranin.

For example, miRNA107 and kallikrein-8 mRNA may be detected in a blood sample, such as a plasma sample.

The present disclosure relates to a method of treating or preventing Alzheimer's disease, and/or treating or preventing precursor stages of Alzheimer's disease, comprising administering to an individual an agent which inhibits Kallikrein-8.

The present disclosure relates to a method of diagnosing or predicting Alzheimer's disease, and/or diagnosing or predicting precursor stages of Alzheimer's disease, comprising administering to an individual a diagnostically effective amount of a compound specifically binding to Kallikrein-8 protein or a probe specifically recognizing kallikrein-8 mRNA, and detecting the level of Kallikrein-8 protein and/or kallikrein-8 mRNA in at least one brain region of the individual, and comparing the level with the level(s) determined in one or more reference healthy individuals and/or patients known to exhibit Alzheimer's disease and/or a precursor stage of Alzheimer's disease,
wherein an increased level compared to the level(s) determined in one or more reference healthy individuals, and/or a level which is identical or similar to the level(s) determined in one or more reference patients known to exhibit Alzheimer's disease and/or a precursor stage of Alzheimer's disease indicates that:
(i) the individual has Alzheimer's disease and/or a precursor stage of Alzheimer's disease,
   and/or
(ii) the individual has a high risk of developing Alzheimer's disease, or for progression of Alzheimer's disease, and/or a precursor stage of Alzheimer's disease,
preferably wherein the individual was not yet diagnosed to have Alzheimer's disease and/or a precursor stage of Alzheimer's disease.

The individual may be an individual who exhibits cognitive impairment or who does not exhibit cognitive impairment.

In one preferred disclosure, the individual exhibits cognitive impairment.

In another preferred disclosure, does not exhibit cognitive impairment.

The methods are suitable for example for screening populations, such as elder populations, such as individuals older than about 70 years or 80 years, which have a high risk to develop AD.

Surprisingly, it was further found in the Examples that antibody-mediated cerebral KLK8 inhibition has anxiolytic effects in transgenic mice, as anti-KLK8 antibody-treated animals spent more time in the open arms and less time in the closed arms of the EPM (Fig. 3b, c), spent more time in the center and border areas and less time in the corners of the OF arena (Fig. 3e, f), took less time to enter the center area for the first time (Fig. 3h), and showed reduced freezing and increased exploratory behaviour (Fig. 3i), when compared to controls (IgG & saline). Even more surprisingly, anti-KLK8 treatment elicited anxiolytic effects also in wildtypes, as they spent less time in the closed arms of the EPM (Fig. 3b, c) and exhibited increased exploratory behaviour in the OF (Fig. 3e, i).

Therefore, the agents for use are surprisingly also suitable for treating and preventing disorders characterized by increased anxiety.

Therefore, the present disclosure relates to an agent which inhibits Kallikrein-8, for use in the treatment or prevention of a disorder accompanied and/or characterized by increased anxiety.

For the agent which inhibits Kallikrein-8, all aspects described above in the context of AD also apply to the aspects relating to increased anxiety. Further, to the extent applicable, all further aspects described above in the context of AD also apply to anxiety disorders.

For example, Rapamycin is an mTOR inhibitor and is used as immunosuppressive agent in preparation of organ transplantation and for cancer treatment. Rapamycin administration results in adverse events such as affective disorders like increased anxiety and/or depression. Further, rapamycin was proposed to be useful in the course of treatment of Alzheimer's disease. Acute administration of rapamycin to rats results in increased anxiety in rats, and further, expression of KLK8 and FKBP5 is increased (Hadamitzky M, Herring A, Keyvani K, Doenlen R, Krügel U, Bösche K, Orlowski K, Engler H, Schedlowski M. Acute systemic rapamycin induces neurobehavioral alterations in rats. Behav Brain Res. 2014, 273:16-22).

Accordingly, the administration of an agent which inhibits Kallikrein-8, for use in the treatment or prevention of a disorder accompanied and/or characterized by increased anxiety is in particular useful for preventing and/or treating an anxiety disorder caused by medical treatment, preferably by an mTOR inhibitor, in particular by rapamycin.

Suitable mTOR inhibitors are well known in the art and include rapamycin, ridaforolimus, everolimus, temsirolimus, a rapamycin-analog and a pharmaceutically acceptable salt thereof.

The mTOR inhibitor may be administered in a dose between 1 µg or 1 mg to 200 mg or 500 mg, in particular 10 mg and 40 mg. Further it may be administered repeatedly or once, preferably repeatedly.

The agent which inhibits Kallikrein-8 and the mTOR inhibitor can be prepared for simultaneous, separate or successive administration.

The present disclosure relates to a composition comprising an agent which inhibits Kallikrein-8 and an mTOR inhibitor, in particular by rapamycin, or a kit of parts comprising an agent which inhibits Kallikrein-8 and an mTOR inhibitor, in particular by rapamycin.

The present disclosure relates to an agent which inhibits Kallikrein-8 and an mTOR inhibitor, for use as a medicament.

The present disclosure relates to an agent which inhibits Kallikrein-8 and an mTOR inhibitor, for use in treating Alzheimer's disease and/or a precursor stage of Alzheimer's disease.

The administration of an agent which inhibits Kallikrein-8 is particularly useful for a disorder accompanied and/or characterized by increased anxiety which is selected from a generalized anxiety disorder, a phobic disorder, such as agoraphobia, specific phobia, and social anxiety disorder, an obsessive-compulsive disorder, post-traumatic stress disorder, separation anxiety disorder, a panic disorder, and/or an anxiety disorder caused by stress, genetics, drugs or medical treatment, in particular by a mTOR inhibitor, such as rapamycin, or combinatory effects thereof, schizophrenia accompanied by increased anxiety and major depression.

Accordingly, the present disclosure relates to a compound specifically binding to Kallikrein-8 protein or a probe specifically recognizing kallikrein-8 mRNA
(a) for use in the prediction and/or diagnosis of a disorder accompanied and/or characterized by increased anxiety,
   preferably in the prediction and/or diagnosis of a disorder accompanied and/or characterized by increased anxiety of an individual exhibiting anxiety, in particular increased anxiety,
   and/or
(b) for use in the identification and/or stratification of at least one individual to be responsive to treatment or prevention of a disorder accompanied and/or characterized by increased anxiety with an agent which inhibits Kallikrein-8.

In a disclosure, the compound or probe is bound to a detectable label.

Accordingly, the present disclosure relates to the use of a compound specifically binding to Kallikrein-8 protein or of a probe specifically recognizing kallikrein-8 mRNA
(a) for the *in vitro* prediction and/or *in vitro* diagnosis of disorder accompanied and/or characterized by increased anxiety in a bodily sample of an individual exhibiting anxiety, in particular increased anxiety,
   more preferably wherein the bodily sample is selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample, and/or
(b) for the identification and/or stratification of at least one individual to be responsive to treatment or prevention of a disorder accompanied and/or characterized by increased anxiety with an agent which inhibits Kallikrein-8.

Accordingly, the present disclosure relates to an *in vitro* method of predicting and/or diagnosing disorder accompanied and/or characterized by increased anxiety,
comprising the steps of:
(1) detecting the level of Kallikrein-8 protein and/or kallikrein-8 mRNA in a bodily sample of an individual exhibiting anxiety, in particular increased anxiety, and
(2) comparing the level determined in step (1) with the level(s) determined in one or more reference samples from healthy individuals and/or from patients known to have a disorder accompanied and/or characterized by increased anxiety,
wherein an increased level determined in step (1) compared to the level(s) determined in one or more reference samples from healthy individuals, and/or a level determined in step (1) which is identical or similar to the level(s) determined in one or more reference samples from patients known to have a disorder accompanied and/or characterized by increased anxiety indicates that:
(i) the individual has a disorder accompanied and/or characterized by increased anxiety,
   and/or
(ii) the individual has a high risk for developing a disorder accompanied and/or characterized by increased anxiety,
preferably wherein the individual was not yet diagnosed to have a disorder accompanied and/or characterized by increased anxiety,
more preferably wherein the bodily sample is selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample.

Accordingly, the present disclosure relates to an *in vitro* method of identifying and/or stratifying of at least one individual to be responsive to treatment or prevention of a disorder accompanied and/or characterized by increased anxiety with an agent which inhibits Kallikrein-8, comprising the steps of:
(1) detecting the level of Kallikrein-8 protein and/or kallikrein-8 mRNA in a bodily sample of an individual, and
(2) comparing the level determined in step (1) with the level(s) determined in one or more reference samples from healthy individuals and/or from patients known to have a disorder accompanied and/or characterized by increased anxiety, or known to be responsive to treatment or prevention of a disorder accompanied and/or characterized by increased anxiety with an agent which inhibits Kallikrein-8,
wherein
an increased level determined in step (1) compared to the level(s) determined in one or more reference samples from healthy individuals, and/or
a level determined in step (1) which is identical or similar to the level(s) determined in one or more reference samples from patients
- known to have a disorder accompanied and/or characterized by increased anxiety, and/or
- known to be responsive to treatment or prevention of a disorder accompanied and/or characterized by increased anxiety with an agent which inhibits Kallikrein-8
indicates that the individual is responsive to treatment or prevention of a disorder accompanied and/or characterized by increased anxiety with an agent which inhibits Kallikrein-8,
more preferably wherein the bodily sample is selected from a bodily fluid sample, in particular selected from cerebrospinal fluid sample, blood sample, such as plasma sample, whole blood sample and serum sample, urine sample and saliva sample, and a biopsy, in particular a brain tissue biopsy, even more preferably the bodily fluid sample is a cerebrospinal fluid sample.

For the compound specifically binding to Kallikrein-8 protein or of a probe specifically recognizing kallikrein-8 mRNA, all embodiments and preferred embodiments described above in the context of AD also apply to the aspects relating to increased anxiety. Further, to the extent applicable, all further embodiments described above in the context of AD also apply to anxiety disorders.

The present disclosure relates to a method of treating or preventing a disorder accompanied and/or characterized by increased anxiety, comprising administering to an individual an agent which inhibits Kallikrein-8.

The present disclosure relates to a method of diagnosing or predicting a disorder accompanied and/or characterized by increased anxiety, comprising administering to an individual who preferably exhibits anxiety or increased anxiety a diagnostically effective amount of a compound specifically binding to Kallikrein-8 protein or a probe specifically recognizing kallikrein-8 mRNA, and detecting the level of Kallikrein-8 protein and/or kallikrein-8 mRNA in at least one brain region of the individual, and comparing the level with the level(s) determined in one or more reference healthy individuals and/or patients known to have a disorder accompanied and/or characterized by increased anxiety,
wherein an increased level compared to the level(s) determined in one or more reference healthy individuals, and/or a level which is identical or similar to the level(s) determined in one or more reference patients known to have a disorder accompanied and/or characterized by increased anxiety indicates that:
(i) the individual has a disorder accompanied and/or characterized by increased anxiety,
   and/or
(ii) the individual has a high risk of developing a disorder accompanied and/or characterized by increased anxiety,
preferably wherein the individual was not yet diagnosed to have a disorder accompanied and/or characterized by increased anxiety.

### Sequences

KLK8 substrate motif: YGRY (SEQ ID No: 1)
murine KLK8 pro-form sequence:
human KLK8:
murine EPHB2 isoform 1 sequence:
murine EPHB2 isoform 2 sequence:
human EPHB2 isoform 1 sequence:
human EPHB2 isoform 2 sequence: highlighted in above sequences in fat and bold = target sequence for KLK8 to cleave EPHB2.
murine KLK8 mRNA:
human KLK8 mRNA transcript variant 1:
human KLK8 mRNA transcript variant 2:
human KLK8 mRNA transcript variant 3:
human KLK8 mRNA transcript variant 4:
human KLK8 mRNA transcript variant 5:

### Figures

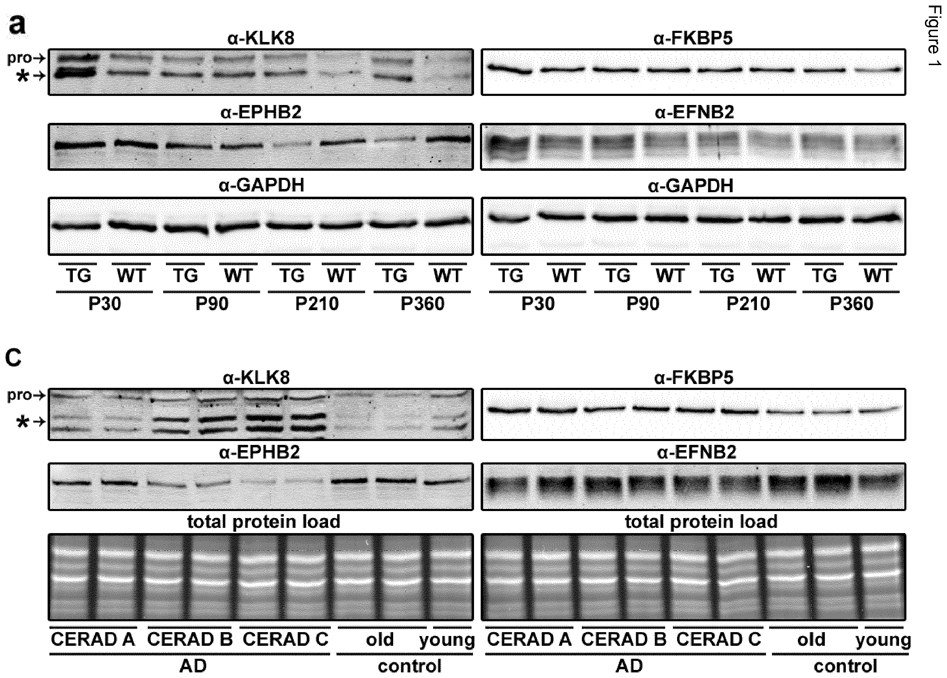

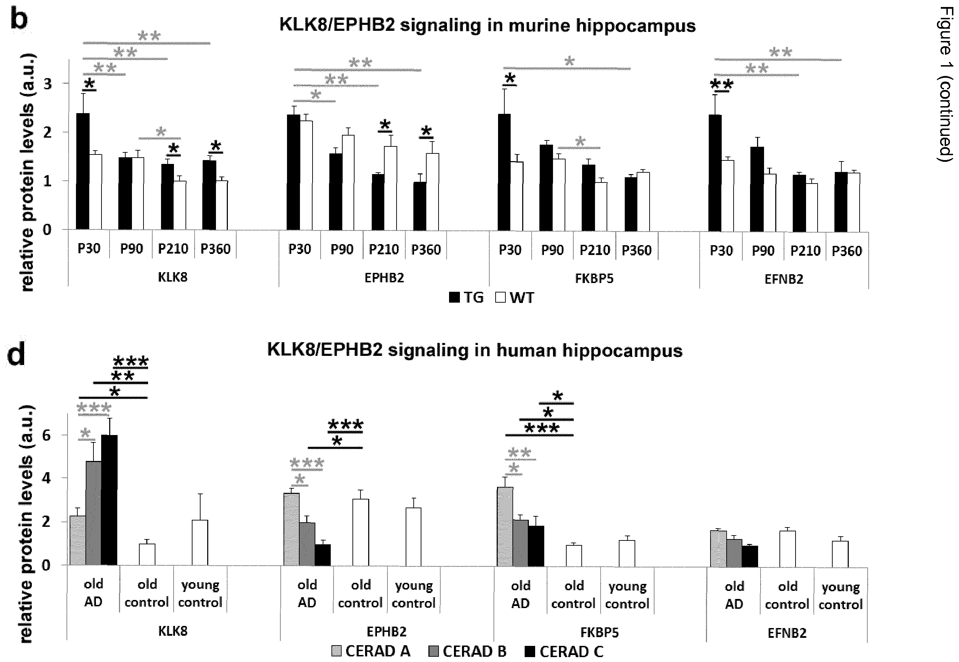

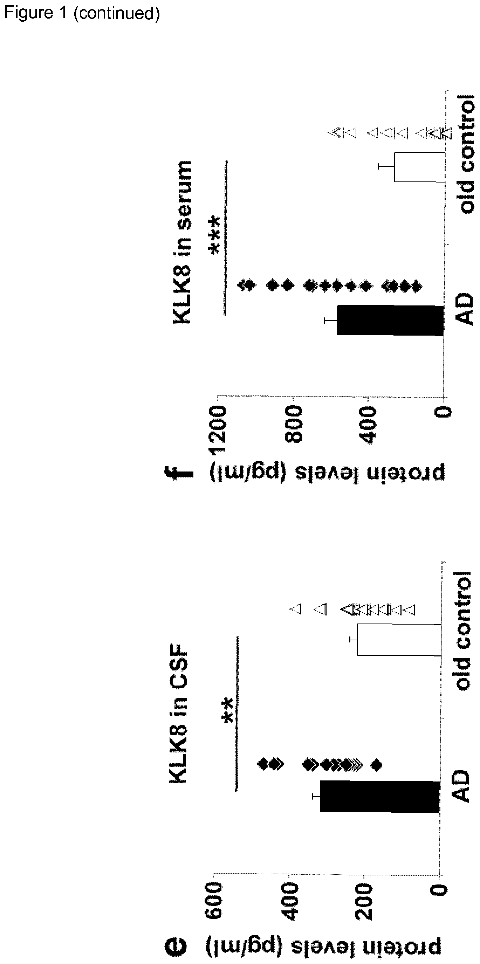

**Figure 1****: KLK8 overexpression at incipient stages of AD precedes the depletion of its proteolytic target EPHB2**
   **a**, **b**, Hippocampal expression of KLK8, EPHB2, FKBP5 and EFNB2 in young (P30), adolescent (P90), adult (P210) and old (P360) transgenic (TG) and wildtype (WT) mice. **c**, **d**, Hippocampal expression of KLK8, EPHB2, FKBP5 and EFNB2 in AD patients at CERAD A/Braak I-II, CERAD B/Braak III-IV, CERAD C/Braak V-VI stages, and young and old controls. KLK8 protein levels in cerebrospinal fluid (CSF) (**e**) and in blood serum (**f**) of AD patients and old controls. A combined quantification of the pro-form of KLK8 and activated KLK8* is depicted for murine and human samples. **P*<0.05, ***P*<0.01, ****P*<0.001 (**a-d:** 2-way ANOVA and Bonferroni *post hoc* test **e**, **f:** Student's t test and Bonferroni correction for multiple testing). Black asterisks indicate comparison between diseased and control groups, grey asterisks indicate comparison between different disease and age stages within diseased or control groups. *n*=8 per murine group and *n*=5-12 per human group (for **c, d**) or n=17 per human group (for **e, f**). Results are shown as mean ± s.e.m.

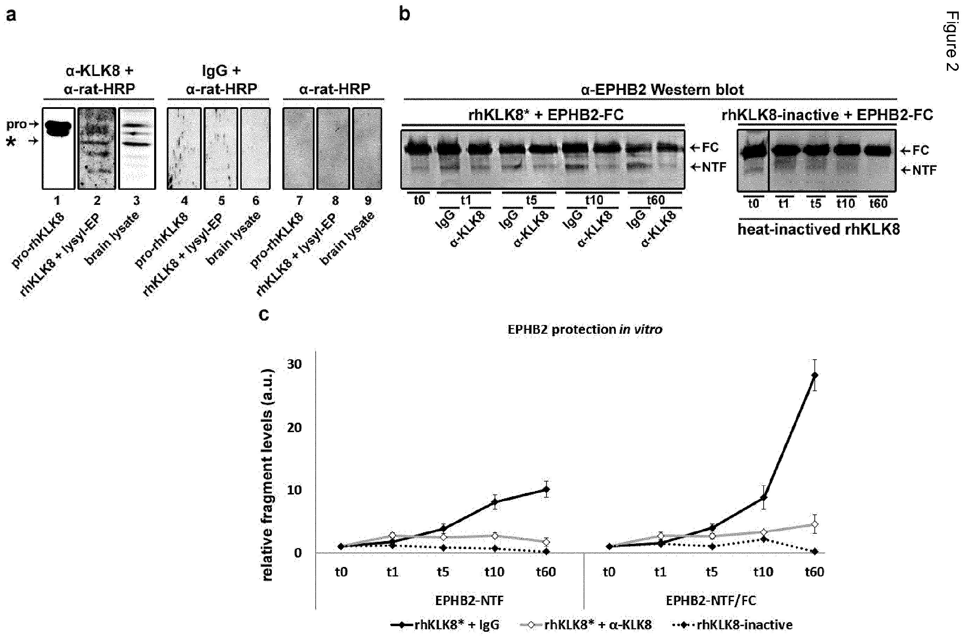

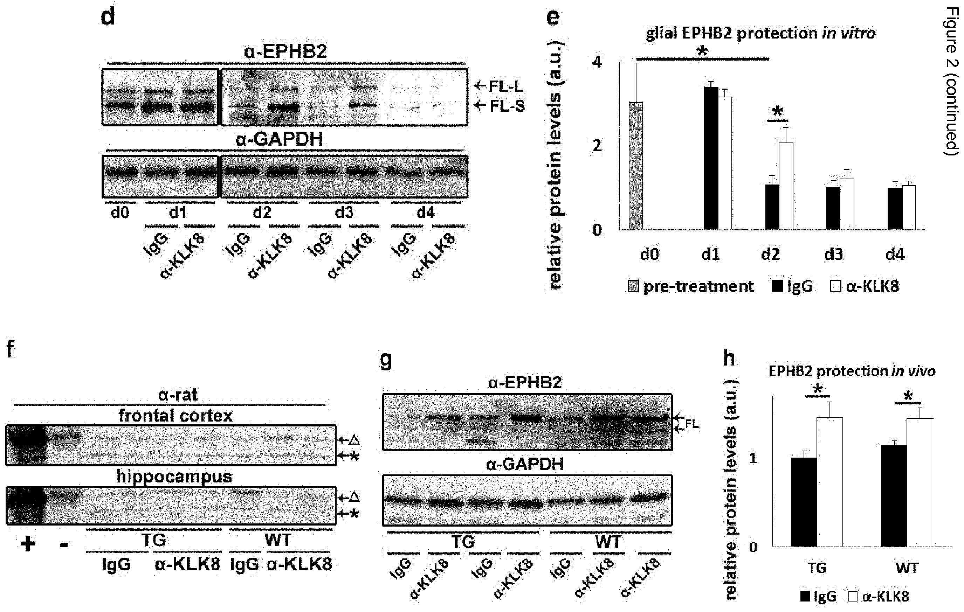

**Figure 2****: Anti-KLK8 antibody protects EPHB2 from fragmentation in a cell-free assay, in primary glial cell culture and in murine brain**
   **a,** The anti-KLK8 antibody (α-KLK8) binds to recombinant human KLK8 in its pro-form (pro-rhKLK8, lane 1), to lysyl endopeptidase (lysly-EP)-activated rhKLK8 (=rhKLK8*, lane 2) and to KLK8 from mouse cortical homogenate in different glycosylated pro-forms and in activated form (=KLK8*) (lane 3), whereas a rat control IgG does not bind pro-rhKLK8 (lane 4), rhKLK8* (lane 5) or murine pro-KLK8 or KLK8* (lane 6). Secondary anti-rat-HRP antibody (a-rat) does not bind human (lanes 7 & 8) or murine (lane 9) KLK8 when primary antibodies are omitted. **b, c,** In the presence of IgG, rhKLK8* processed EPHB2-FC into N-terminal fragments (EPHB2-NTF), resulting in increasing EPHB2-NTF/FC ratios. EPHB2 fragmentation was almost completely abolished when rhKLK8* was pre-blocked for different durations (t1=1 min, t5, t10, t60) with α-KLK8 or following heat inactivation (rhKLK8-inactive). **d, e**, In primary mixed glia, addition of Aβ₄₂ at test day 1 (d1) resulted in EPHB2 full length (EPHB2-FL) depletion at d2, whereas α-KLK8 treatment delayed this decline. **f**, Penetration of α-KLK8 or IgG (both produced in rat) into frontal cortices (upper image) and hippocampi (lower image) was validated in transgenic (TG) and wildtype (WT) mice via immunoblotting with α-rat, revealing a positive signal at 40 kDa (indicated by *) which is also detectable in the positive control (+, naïve brain homogenate mixed with both α-KLK8 and IgG). Δ indicates an unspecific band at 50 kDa due to the cross-reactivity of the α-rat with murine immunoglobulins, as this band is also detectable in the naïve brain homogenate of untreated mice (-, negative control). **g**, **h,** α-KLK8 treatment protected cerebral full-length EPHB2 (EPHB2-FL) in TG and WT mice. **P*<0.05 (2-way ANOVA and Bonferroni *post hoc* test). *n*=6-8 cell culture plates from 3 independent batches per treatment condition and test day, n=8 per murine group. Results are shown as mean ± s.e.m.
**Figure 3****: KLK8 inhibition reduces fear and improves cognition**
   **a,** Anti-KLK8 treatment scheme. At P150 (two months after disease onset), 16 female transgenic (TG) and 16 female wildtype (WT) mice were subjected to subcutaneous implantation of osmotic pumps, enabling constant intraventricular delivery of either the anti-KLK8 antibody (α-KLK8) (n=8 per genotype), IgG (n=7 per genotype), or saline (n=1 per genotype) over a 4 week period. At P178, mice were tested in the Elevated Plus Maze (EPM) for anxiety, at P179 in the Open Field (OF) for exploratory behavior and between P180 and P184 for spatial memory and learning performance in the Barnes Maze (BM). At P185, TG mice received an intravenous injection of an anti-Aβ antibody (α-Aβ) or saline, followed by blood and brain tissue collection. **b, e, j**, Representative paths from the EPM **(b),** the OF **(e)** and the BM (test day 1 [d1], trial 1 [t1], **j). c**, Total time mice spent in the center area, open arms and closed arms of the EPM platform. **d**, Total distance mice travelled in all three compartments of the EPM. **f,** Total time mice spent in the center area, borders and corners of the OF platform. **g**, Total distance mice travelled in all three compartments of the OF. **h** Time it took mice to enter the center area of the OF for the first time. **i**, Total time mice showed freezing or exploring behavior in the OF. **k, m, o,** Learning curves during spatial training in the BM. Total time (**k, l**), number of wrong holes approached (**m, n**), and total distance (**o, p**) mice needed to escape from the BM platform on d1-t1 and d4 (**l, n, p**). ^{T}*P*<0.1, **P*<0.05, ***P*<0.01 (2-way ANOVA and Bonferroni *post hoc* test). *n*=8 per genotype and treatment. Results are shown as mean ± s.e.m.

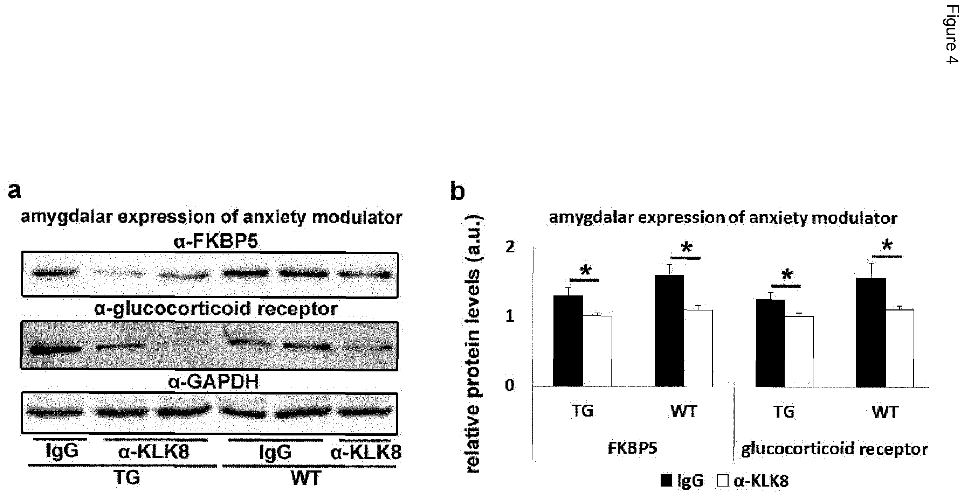

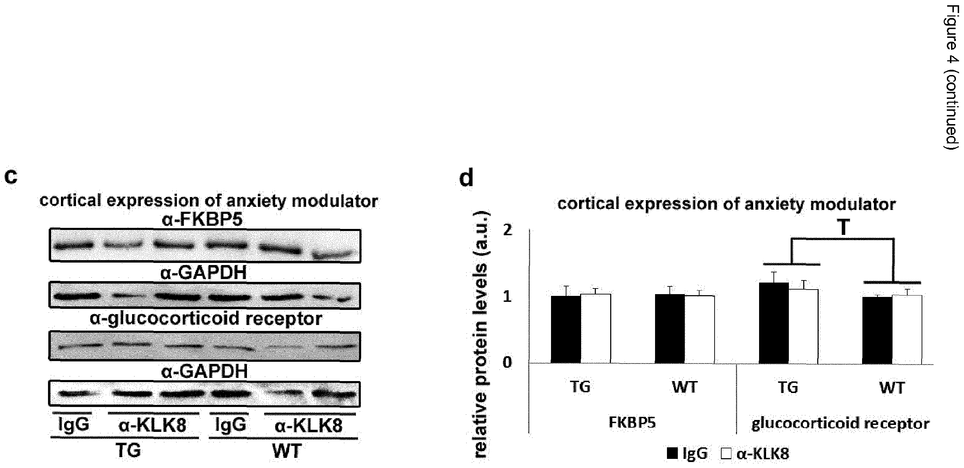

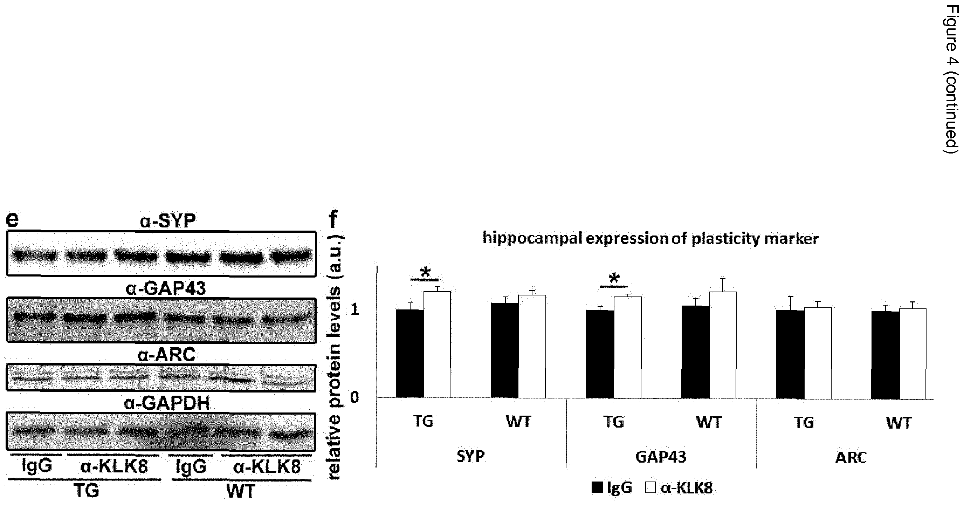

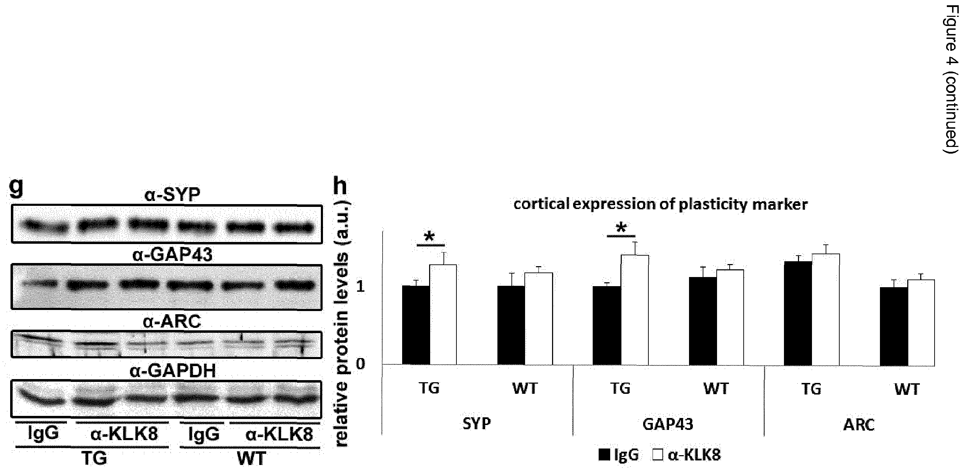

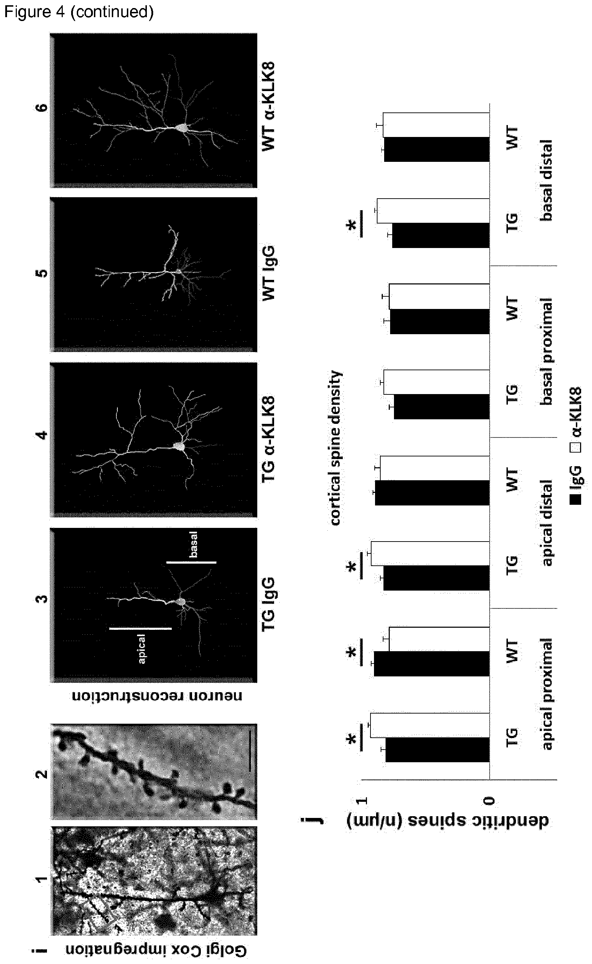

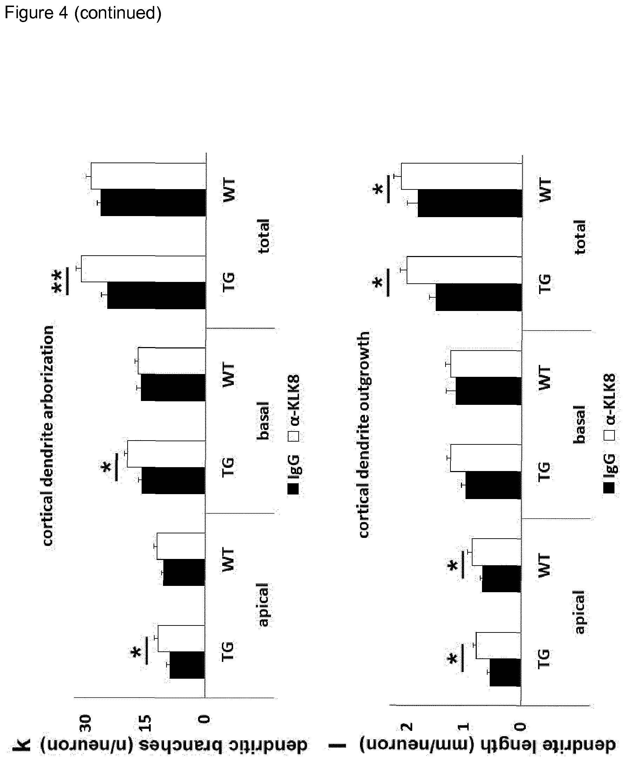

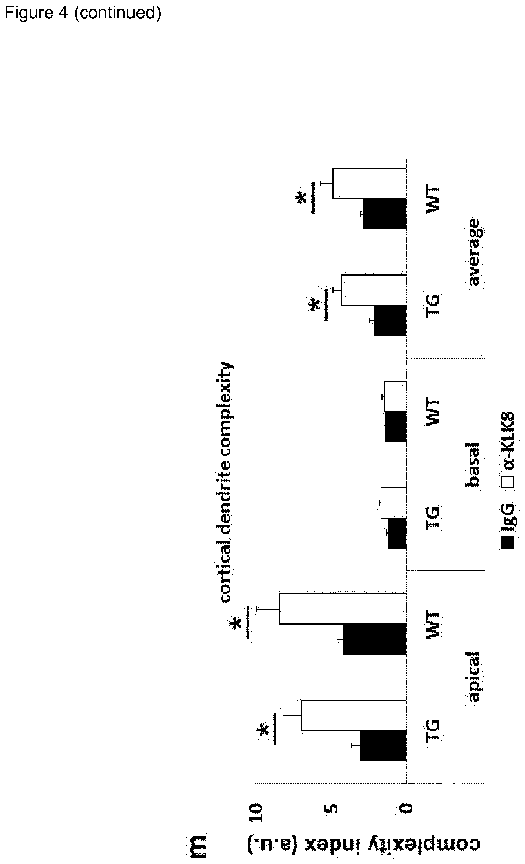

**Figure 4****: KLK8 inhibition reverses the molecular signatures of anxiety and enhances structural neuroplasticity**
   Levels of anxiety-modulating FKBP5 and glucocorticoid receptor in the amygdala (**a, b**) and frontal cortex (**c, d**) of transgenic (TG) and wildtype (WT) mice treated with IgG or anti-KLK8 antibody (α-KLK8). Levels of structural plasticity markers SYP, GAP43 and ARC in the hippocampus (**e, f**) and frontal cortex (**g, h**) of TG and WT mice treated with IgG or α-KLK8. **i,** Representative image of a Golgi Cox impregnated layer V frontal cortex neuron (image 1) and a magnified dendrite with spines (image 2) from the same neuron (indicate by the red rectangle). Scale bar: 100 µm. Representative images (images 3-6) of digital reconstructions from Golgi Cox impregnated neurons from all four murine groups. **j**, Quantification of the average apical proximal and distal as well as basal proximal and distal spine density. The average number of branches **(k),** the average length **(I)** and the complexity index **(m)** of apical, basal and total dendrites per neuron was determined in the same neurons. ^{T}*P*<0.1, **P*<0.05, ***P*<0.01 (2-way ANOVA and Bonferroni *post hoc* test). *n*=8 per genotype and treatment. Results are shown as mean ± s.e.m.

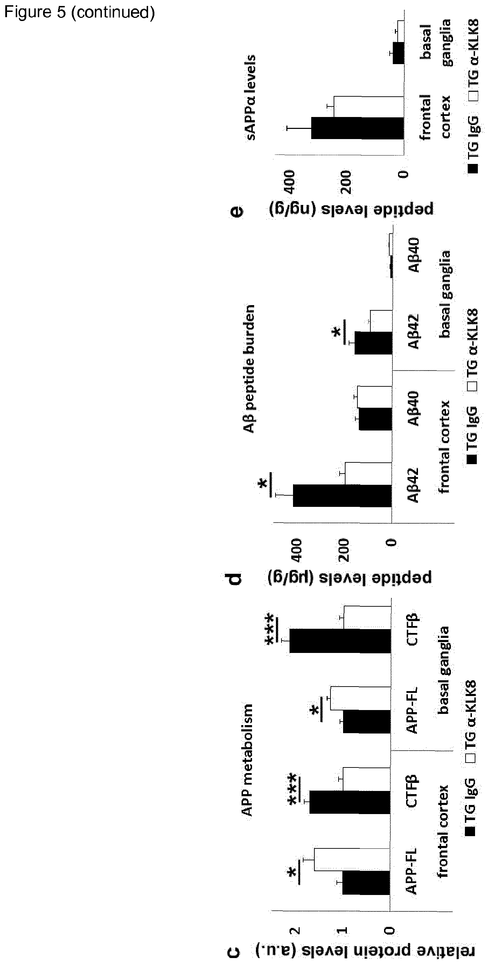

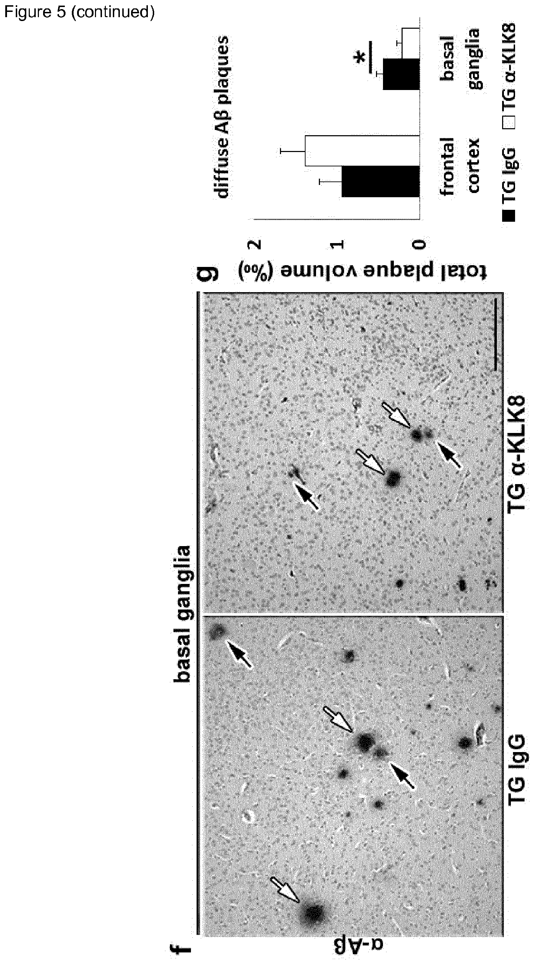

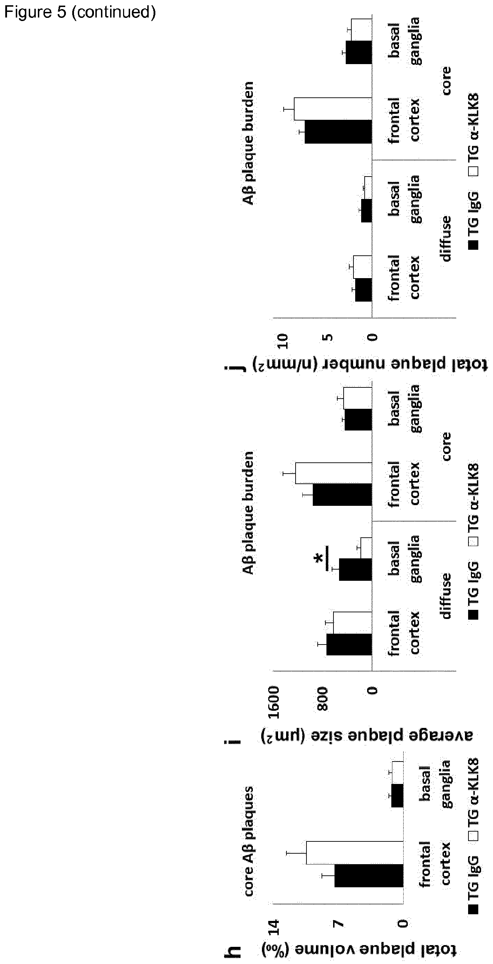

**Figure 5****: Anti-KLK8 antibody treatment counteracts Aβ pathology**
   **a**, *hAPP* mRNA levels in the frontal cortex and basal ganglia of transgenic (TG) mice treated with IgG or anti-KLK8 antibody (α-KLK8). Full-length APP (APP-FL) and C-terminal fragment β (APP-CTFβ) levels **(b, c),** Aβ₄₂ and Aβ₄₀ **(d),** and sAPPa peptide levels **(e)** in the frontal cortex and basal ganglia of IgG or α-KLK8-treated TG mice. **f**, Anti-Aβ immunostaining of basal ganglia showing diffuse (black arrows) and core (white arrows) Aβ plaque burden in TG IgG (left) and TG α-KLK8 (right) mice. Scale bar: 200 µm. Stereological quantification of total diffuse (**g**) and core (**h**) plaque volume, as well as of the average plaque size (**i**) and the total plaque number (**j**) in the frontal cortex and basal ganglia. **P*<0.05, ***P*<0.01, ****P*<0.001 (Student's t test and Bonferroni correction for multiple testing). *n*=8 per treatment. Results are shown as mean ± s.e.m.

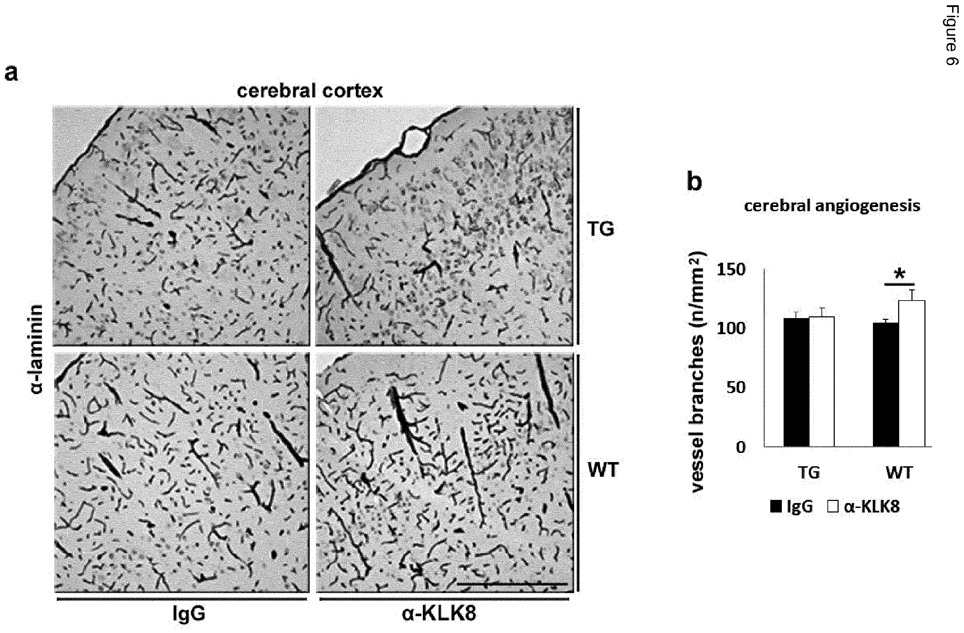

**Figure 6****: KLK8 inhibition improves neurovascular function**
   **a,** Representative cerebral anti-laminin immunostaining of transgenic (TG) and wildtype (WT) mice treated with IgG or anti-KLK8 antibody (α-KLK8). Scale bar: 100 µm. **b**, Stereological quantification of cerebral (frontal cortex and basal ganglia) blood vessel branches. **c, d,** Cerebral levels of Aβ transporters LRP1, MDR1 and RAGE from IgG or α-KLK8-treated TG and WT mice. **e**, Aβ efflux dynamics across the blood-brain-barrier (BBB) were determined over various time spans (*t₀*=0 min, before anti-Aβ antibody (α-Aβ) injection, baseline plasma Aβ levels, *t₁₀*=10 min following α-Aβ injection and *t₄₀*). ^{T}*P*<0.1, **P*<0.05, ***P*<0.01 (2-way ANOVA and Bonferroni *post hoc* test). *n*=8 per treatment. Results are shown as mean ± s.e.m.
**Figure 7****: Depletion of autophagy markers in AD-affected murine and human brain**
   **a, b,** Frontocortical expression of autophagy markers beclin-1 and STX17 in young (P30), adult (P210) and old (P360) transgenic (TG) and wildtype (WT) mice. **c, d**, Cortical beclin-1 and STX17 expression in AD patients at stage CERAD C/Braak V-VI, and young and old controls. **P*<0.05, ***P*<0.01 (2-way ANOVA and Bonferroni *post hoc* test). *n*=8 per murine group, *n*=6-10 per human group. Results are shown as mean ± s.e.m.

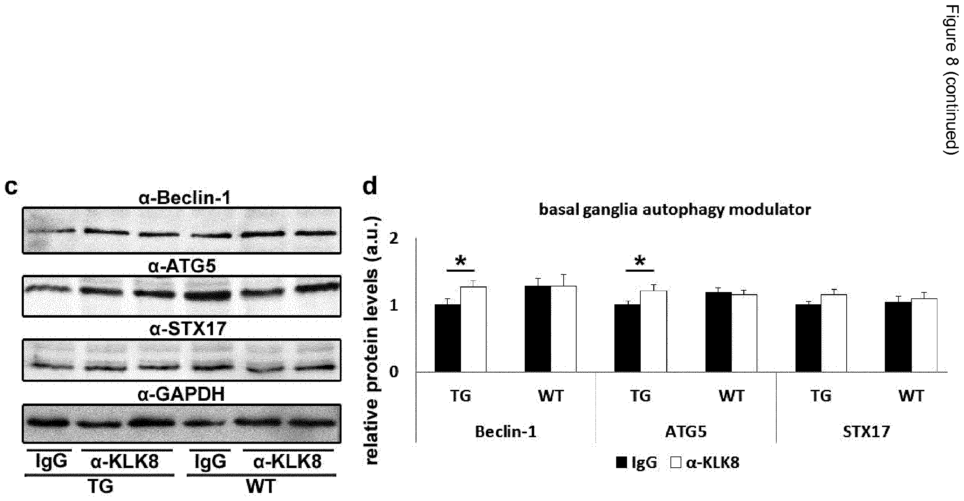

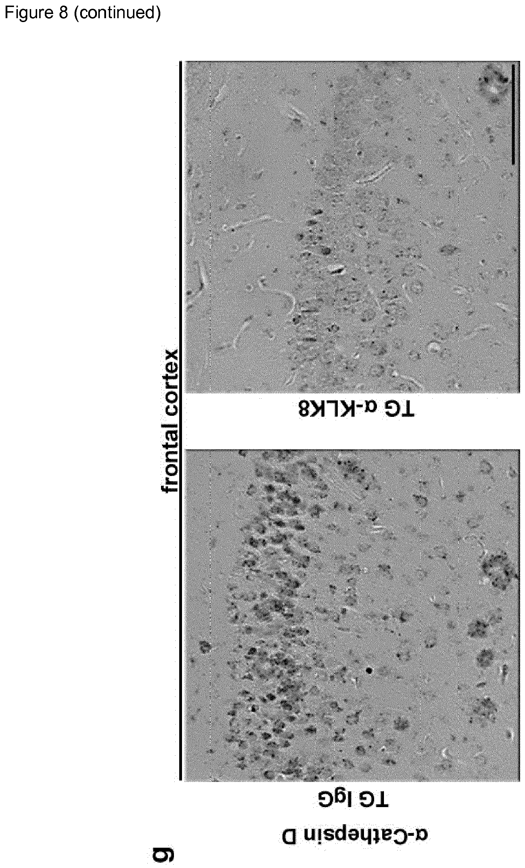

**Figure 8****: KLK8 inhibition boosts the autophagy machinery in murine brain**
   Levels of beclin-1, ATG5 and STX17 in IgG and anti-KLK8 antibody-treated (α-KLK8) transgenic (TG) and wildtype (WT) mice in the frontal cortex (**a, b**) and basal ganglia (**c, d**). **e, f**, Cathepsin D levels in the frontal cortex and basal ganglia of TG and WT mice after IgG or α-KLK8 treatment. **g**, Representative anti-cathepsin D immunostaining of the frontal cortex in TG mice treated with IgG (left image) or α-KLK8 (right image) demonstrate reduced cathepsin D accumulation following KLK8 blockade. Scale bar: 100 µm. **P*<0.05 (2-way ANOVA and Bonferroni *post hoc* test). *n*=8 per murine group. Results are shown as mean ± s.e.m.

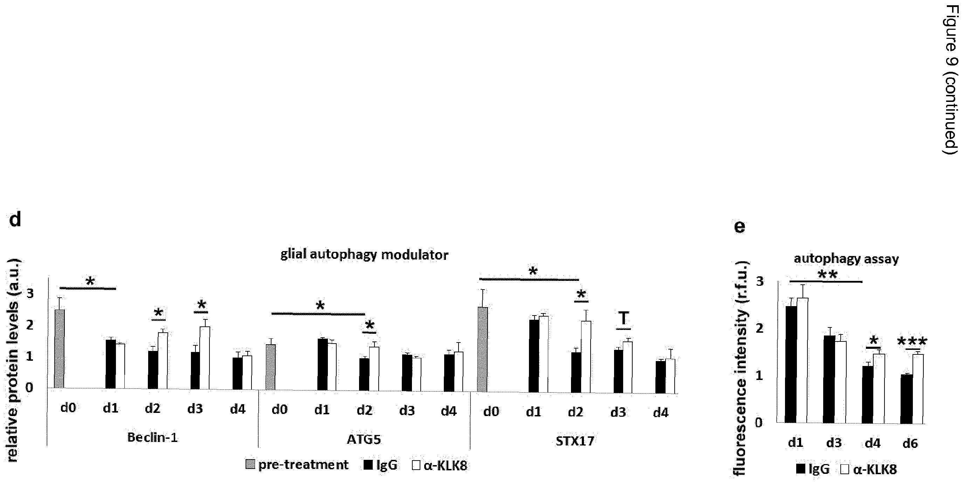

**Figure 9****: KLK8 inhibition improves microglial Aβ phagocytosis and autophagy *in vitro***
   **a,** Primary glia were grown until DIV15 and then plated at a 1/1 astrocytes/microglia ratio. **b,** At DIV17 (before treatment, d0), expression and secretion of KLK8 (upper images) and EPHB2-FL (in its long and short splice variants FL-L and FL-S and in the secreted EPHB2-NTF version) (lower images) were determined in lysates and supernatant. **a**, At d0, glia were either incubated with α-KLK8 (upper row), with α-KLK8 plus α-EPHB2 (lower row) or with IgG. 24h later, Aβ₄₂ was added to the medium. 8h later (d1+8h), EPHB2 levels, autophagy, Aβ₄₂ phagocytosis and survival were monitored. Antibody treatments were repeated at d2, d3 and d5, Aβ₄₂ addition at d5 and outcome measures between d2 and d6. **c, d**, Intraglial beclin-1, ATG5 and STX17 levels between d0 and d4. **e**, Fluorescence emission following glial treatment with monodansylcadaverine (MDC, a probe detecting autophagic vacuoles) between d1 and d6. **f**, Following incubation with Aβ₄₂-FAM and MDC, α-KLK8-treated glia (five images on the right) demonstrate increased MDC incorporation, indicating increased autophagic vacuole density when compared to IgG-treated glia (five images on the left) at d6. Scale bar: 100 µm. Microglia depicted in the smaller images demonstrate swollen autophagic vacuoles heavily loaded with Aβ₄₂-FAM in the IgG group and smaller autophagic vacuoles in α-KLK8-treated glia. Scale bar: 10 µm. Intraglial (g) and extraglial (h) Aβ₄₂ levels between d0 and d4. **i,** Fluorescence images of IgG (upper row) and α-KLK8-treated (lower row) glia incubated with Aβ₄₂-FAM and α-AlF1 at d2, demonstrating increased AIF1 signal and hence microglial activation, increased intra-microglial Aβ₄₂-FAM and reduced extra-glial Aβ₄₂-FAM signal in α-KLK8-treated glia, indicating enhanced Aβ phagocytosis. Scale bars: 100 µm (upper image) and 10 µm (lower image). ^{T}*P*<0.1, **P*<0.05, ***P*<0.01, ****P*<0.001 (2-way ANOVA and Bonferroni *post hoc* test). *n*=6-8 cell culture plates from 3 independent batches per treatment condition and test day. Data represent the mean ± s.e.m.

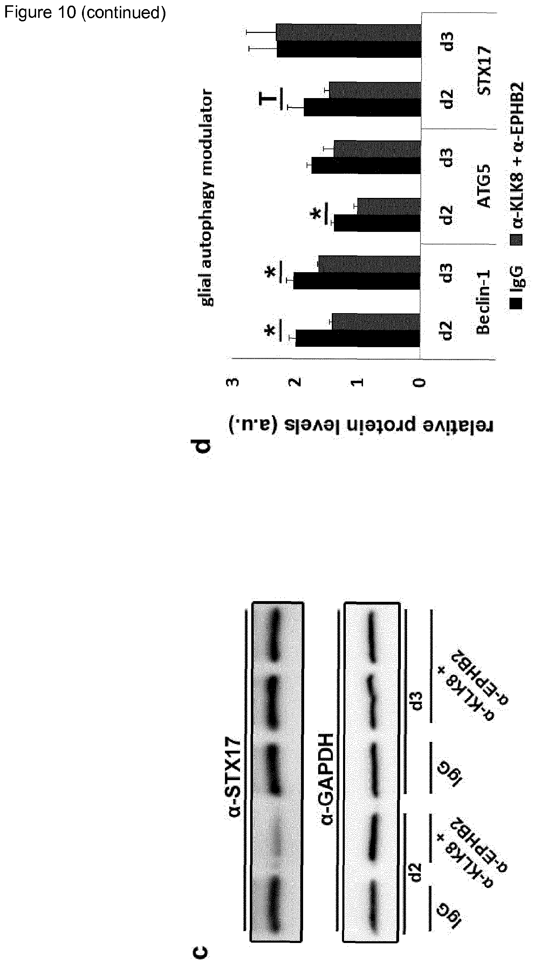

**Figure 10****: Co-incubation of anti-KLK8 antibody with anti-EPHB2 antibody reverses the protective effects of anti-KLK8 treatment**
   Simultaneous administration of α-KLK8 and anti-EPHB2 antibody (α-EPHB2) reduced glial protein levels of autophagy marker beclin-1 at d2 and d3 (a, d), of ATG5 at d2 (**b, d**) and of STX17 (by trend) at d2 (**c**, **d**) when compared to control IgG treatment. Following α-KLK8 treatment intraglial Aβ₄₂ levels (measured by ELISA) increased (**e**), while extraglial (**f**) Aβ₄₂ levels decreased when compared to IgG treated cells, indicating a rise in Aβ phagocytosis and degradation. Co-incubation with α-KLK8 and α-EPHB2 resulted in even higher intraglial Aβ levels compared to α-KLK8 alone (**e**), but the amount of extraglial Aβ (**f**) remained unchanged, indicating that not the Aβ phagocytosis but its breakdown is EPHB2-mediated, leading to intracellular Aβ accumulation. **g**, Cell viability monitoring with a XTT assay revealed that none of the compounds tested disturbed cell survival at d1 or d3, nor cell proliferation at d11. ^{T}*P*<0.1, **P*<0.05, ***P*<0.01, ****P*<0.001 (2-way ANOVA and Bonferroni *post hoc* test). *n*=6-8 cell culture plates from 3 independent batches per treatment condition and test day. Results are shown as mean ± s.e.m.

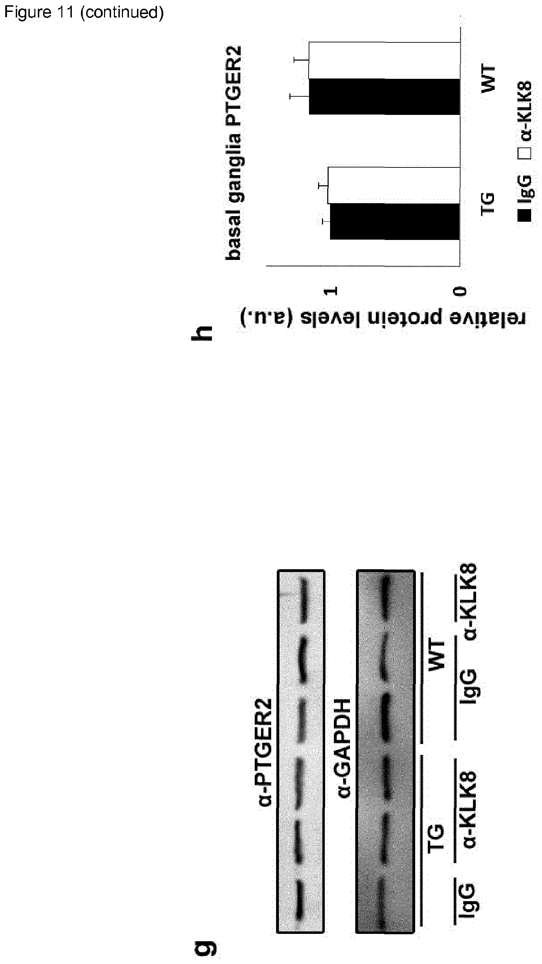

**Figure 11****: KLK8 inhibition promotes microglial activity *in vivo***
   **a**, Anti-AIF1 immunostaining of basal ganglia of IgG or anti-KLK8 antibody-treated (α-KLK8) transgenic (TG) and wildtype (WT) mice. Scale bar: 500 µm. **b**, Stereological quantification of microglial density in the frontal cortex and basal ganglia. **c**, Anti-AIF1 and anti-Aβ double immunostaining of basal ganglia of IgG or α-KLK8-treated TG mice. Scale bar: 100 µm. **d**, Number of microglia surrounding Aβ plaques in the frontal cortex and basal ganglia. Cerebral expression of the pro-inflammatory PTGER2 remained unchanged following α-KLK8 treatment (**e**, **f**: in the frontal cortices; **g, h**: in the basal ganglia), with PTGER2 levels being increased in frontal cortices of diseased mice. **P*<0.05, ***P*<0.01 (2-way ANOVA and Bonferroni *post hoc* test). *n*=8 per murine group. Results are shown as mean ± s.e.m.

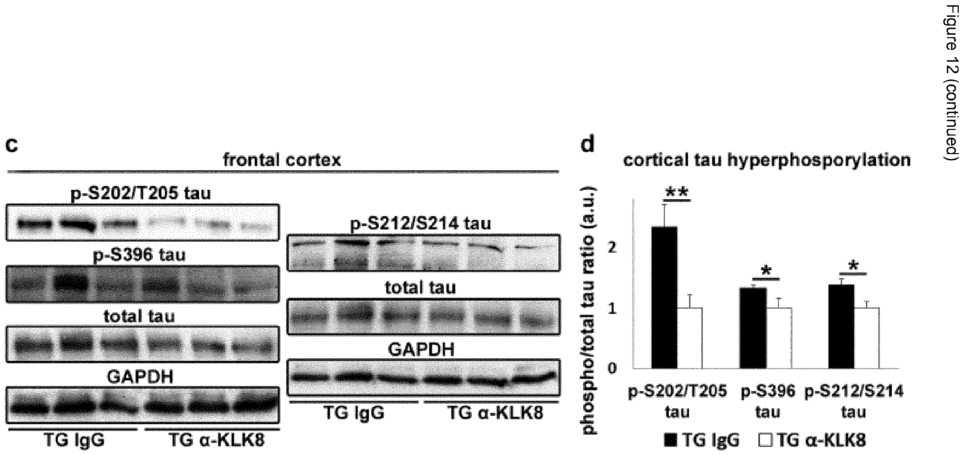

**Figure 12****: Anti-KLK8 antibody treatment counteracts tau pathology**
   **a**, Anti-phospho-tau (AT8) and anti-Aβ double immunostaining showing neuritic, AT8-positive (black arrows) and non-neuritic, AT8-negative (white arrows) Aβ plaques in frontal cortices of transgenic (TG) mice treated with IgG or anti-KLK8 antibody (α-KLK8). Scale bar: 100 µm. **b**, Stereological quantification of the neuritic to total plaque ratio in the frontal cortex and basal ganglia. **c, d**, Levels of tau phosphorylation at S202/T205, S396 and S212/214 normalized against total tau amounts and GAPDH. **e**, **f**, Levels of PI3K phosphorylation at T199/T458, Akt phosphorylation at S473 and GSK3β phosphorylation S9, normalized against levels of total PI3K, Akt or GSK3β and GAPDH. **g**, Model illustrating that α-KLK8 protects EPHB2 and its receptor tyrosine kinase (RTK) from fragmentation, thereby increasing the phosphorylation and activation of PI3K and Akt and the down-stream phosphorylation and inactivation of GSK3β, finally resulting in decreased tau phosphorylation and less dystrophic neurites. **P*<0.05, ***P*<0.01, ****P*<0.001 (Student's t test and Bonferroni correction for multiple testing). n=8 per treatment. Results are shown as mean ± s.e.m.

### Examples

### Kallikrein-8 inhibition attenuates Alzheimer's pathology in mice

### Methods:

### General

All data presented in this paper were generated in blind-coded experiments, in which the investigators who collected the data were unaware of the specific genotype and treatment of mice, brain slices and cell cultures. Protein, peptide and mRNA levels were quantified individually in duplicates or triplicates in separate brain areas from mice and humans.

### Descriptive approach

### A. Murine samples

Female TgCRND8 mice (hemizygously carrying and over-expressing a double-mutant human APP 695 transgene [*hAPP*+/-] harbouring the "Swedish" and "Indiana" mutations [KM670/671NL & V717F] under the control of the hamster Prion protein promoter) (Chishti, M.A., Yang, D.S., Janus, C., Phinney, A.L., Horne, P., Pearson, J., Strome, R., Zuker, N., Loukides, J., French, J., et al. 2001. Early-onset amyloid deposition and cognitive deficits in transgenic mice expressing a double mutant form of amyloid precursor protein 695. J Biol Chem 276:21562-21570) as well as wildtype littermates from the hybrid C57BL/6-C3H/HeJ background strain were kept in standard housing in groups of 4 animals from postnatal day 30 (P30) until P360 in 2 independent batches. To minimize a biased effect of the parental genotype on the phenotype of the investigated mice, equal numbers of transgenic and wildtype mice were used per litter. Brains from both genotypes were harvested at P30 (early juvenile period, before the onset of Aβ pathology, n=8 per genotype), P90 (around the onset of Aβ pathology, n=8 per genotype), P210 (advanced stage of Aβ pathology, n=8 per genotype) and P360 (full-blown stage of AD-like pathology, n=8 per genotype). The brain regions vulnerable for AD pathology and linked with AD-related cognitive decline, i.e. frontal cortex, entorhinal cortex and hippocampus as well as the lesser and later affected cerebellum were isolated and used for DNA, RNA and protein extraction (15596-018, TRIzol-reagent, life technologies).

### B. Human samples

Frozen frontal cortices (gyrus frontalis medius), entorhinal cortices, hippocampi and cerebella from AD patients classified (by two neuropathologists) as CERAD (Mirra, S.S., Hart, M.N., and Terry, R.D. 1993. Making the diagnosis of Alzheimer's disease. A primer for practicing pathologists. Arch Pathol Lab Med 117:132-144) A/Braak & Braak (Braak, H., and Braak, E. 1991. Neuropathological stageing of Alzheimer-related changes. Acta Neuropathol 82:239-259) I-II (n=12, 5 females, 7 males, 73.92±1.94 years, post-mortem interval (PMI) 35±3.97 h), CERAD B/Braak & Braak III-IV (n=5, 5 females, 84±2.42 years, PMI 27.38±6.64 h), CERAD C/Braak & Braak V-VI (n=7, 4 females, 3 males, 79.71±2.2 years, PMI 25.29±5.2 h) as well as from neurologically healthy young controls (n=6, 3 per sex, 30.67±2.97 years, PMI 31.33±10.18 h) and age-matched controls (n=10, 5 per sex, 65.6±2.62 years, PMI 28.5±3.37 h) were separately isolated and subjected to DNA, RNA and protein extraction.
Cerebrospinal fluid (CSF) as well as blood serum from the same individual were obtained from an independent cohort of patients diagnosed for AD (n=17, 9 females, 8 males, 71.44±8.5 years) as well as from neurologically healthy age-matched controls (n=17, 9 females, 8 males, 63.2±9.18 years). The diagnosis was based on the clinical status, including neuropsychological tests, and was further confirmed by quantification of Aβ and Tau levels in CSF as well as imaging data.

### Characterization of the anti-KLK8 antibody

Binding of the inhibitory anti-KLK8 antibody (1:200, M021-3, MBL International) to recombinant human KLK8 (rhKLK8, 20 pmol/µl, 2025-SE-010, R&D Systems) and to KLK8 from mouse brain homogenate (20 µg/sample) was validated by immunoblotting. Specificity of the anti-KLK8 antibody has been reported before, demonstrating that it does not cross-react with other cerebral serine proteases such as α-thrombin, trypsin, kallikrein, tissue plasminogen activator, or urokinase plasminogen activator (Y. Momota, S. Yoshida, J. Ito, M. Shibata, K. Kato, K. Sakurai, et al., Blockade of neuropsin, a serine protease, ameliorates kindling epilepsy. Eur J Neurosci 1998;10:760-764). EPHB2 fragmentation by KLK8 was tested by incubation of recombinant chimeric EPHB2-FC (400 ng at 100 ng/µl, E9402, Sigma-Aldrich) with lysyl endopeptidase (0.01 A.U./µg rhKLK8, 125-02543, Wako) activated rhKLK8* (4 ng at 1 ng/µl) and subsequent EPHB2 immunoblotting (1:100, AF467, R&D Systems). The capacity of the anti-KLK8 antibody to protect EPHB2 from fragmentation was analyzed by incubation of 400 ng EPHB2-FC with 4 ng rhKLK8* which was blocked by pre-treatment with 80 ng anti-KLK8 antibody (at 20 ng/µl, rhKLK8*-block) in comparison to incubation of EPHB2-FC with rhKLK8* which was pre-incubated with 80 ng control rat IgG (MCA1124R, AbD Serotec) and subsequent EPHB2 immunoblotting.

### Experimental manipulations

### In vivo approach

### 1. Intraventricular anti-KLK8 antibody delivery

16 female transgenic mice and 16 female wildtype littermates were standard housed in groups of 4 mice from P30 until P150 in 3 independent batches. At P150, all mice were subjected to subcutaneous implantation of osmotic pumps (7147160-6, Alzet 2004, Durect) and brain infusion kits (0008859-2, Alzet Brain Infusion Kit 3, Durect). A hole was drilled in the stereotaxically correct location (-0.2 Bregma, +0.9 parasagittal), and a cannula, attached to the pump was inserted through the skull into the lateral ventricle and cemented in place, enabling constant intraventricular delivery of the anti-KLK8 antibody (M021-3, 62 µg/kg/d at an average concentration of 0.21 µg/µl diluted in saline and a flow rate of 0.25 µl/h, n=8 per genotype), control rat IgG (MCA1124R, n=7 per genotype), or saline (n=1 per genotype) over a 4 week period. Antibodies were dialysed against sterilized PBS, pH 7.4 to remove NaN₃ (88401, Slide-A-Lyzer Mini Dialysis Devices, Thermo Scientific) before injection into pumps. To minimize a biased effect of the parental genotype on the phenotype of the experimental mice, littermates were equally distributed to verum and control groups.

### 2. Behavioral phenotyping

From P178 until P184, mice were behaviorally phenotyped. Prior to testing they had been adapted to the inverted day/night cycle for one week. First, anxiety behavior was evaluated in the Elevated Plus Maze (EPM) according to Young et al. (Young, E.J., Lipina, T., Tam, E., Mandel, A., Clapcote, S.J., Bechard, A.R., Chambers, J., Mount, H.T., Fletcher, P.J., Roder, J.C., et al., 2008; Reduced fear and aggression and altered serotonin metabolism in Gtf2ird1-targeted mice. Genes Brain Behav 7:224-234). The EPM consisted of two opposite open arms (each 27.5 x 6 x 0.5 cm, length x width x height), two opposite closed arms (each 27.5 x 6 x 16 cm) and a center platform (5 x 5 x 0.5 cm). The maze was 75 cm elevated above the ground. Each mouse was placed in the center and observed for 5 min. Number of entries, latencies until first time entries, duration and distance covered in closed and open arms, and in the center as well as velocity were automatically tracked and analyzed by Video Mot 3D software (TSE, version 7.0.1). The next day, mice were tested in the Open Field (OF) for exploratory behavior and general activity according to Kilic et al. (Kilic, E., ElAli, A., Kilic, U., Guo, Z., Ugur, M., Uslu, U., Bassetti, C.L., Schwab, M.E., and Hermann, D.M. 2010. Role of Nogo-A in neuronal survival in the reperfused ischemic brain. J Cereb Blood Flow Metab 30:969-984). The OF arena (52 x 52 x 30 cm) was located 72 cm above the floor on a circular platform. Each mouse was placed near the wall and observed for 10 min. The test arena was divided into one center (31.2 x 31.2 cm), four border (each 10.4 x 31.2 cm) and four corner (each 10.4 x 10.4 cm) areas. Number of entries, latencies until first time entries, duration and distance covered in each area as well as velocity were automatically tracked. From P180 until P184, hippocampus-associated spatial memory and learning performance was assessed by the Barnes Maze (BM) according to Sunyer et al. (Sunyer, B., Patil, S., Höger, H., and Lubec, G. 2007. Barnes maze, a useful task to assess spatial reference memory in the mice). The BM arena consisted of a circular platform (92 cm diameter, 120 cm above the ground) with 20 equally distributed holes (5 cm diameter, 7.5 cm distance between holes) located at the border. One hole (escape hole) was connected to a box (15.5 × 9.5 x 6 cm), allowing to escape from the BM platform, whereas the other 19 holes were closed (error holes). 24h before tests started, mice were habituated to the setup for 2 trials, each lasting for 3 min. Tests were performed between 10:00 am and 6:00 pm. Each mouse was placed in a black cylinder located in the middle of the platform for 10 sec. During that time, red light was switched to bright light (180 Ix) and the cylinder was lifted, defining the start of a 3 min trial. During each trial, primary errors, total errors, primary latency, total latency, path length covered and velocity were automatically recorded. Primary errors and latencies were defined as the number and duration of approximations to error holes until approaching the escape hole for the first time. Total errors and latencies were defined as the number and duration of approximations to error holes and to the escape hole until final escape. Once a mouse escaped, it was allowed to stay in the escape box for 1 min before being transferred to the home cage. If a mouse did not escape during the 3 min interval, it was gently guided to the escape hole until the mouse escaped; otherwise it was placed directly into the escape box for 1 min. On test day 1, each mouse was tested in four trials. On test days 2, 3, 4 and 5, each mouse was tested in two trials. Inter-trial intervals lasted for 15 min. Following the four test days, the escape hole was blocked at day 5 (probe trial). Mice were allowed to explore the platform for 90 sec per trial. In all behavioral tests, freezing and exploration behavior were manually recorded. Before and after each test, the EPM, OF and BM arenas were cleaned with 70% ethanol.

### 3. Blood and tissue sampling

Following behavioral testing, blood was collected from retrobulbar venous plexus of each transgenic animal to determine baseline circulating plasma Aβ levels. In order to quantify Aβ efflux efficacy across the brain-blood barrier (BBB), 7 anti-KLK8 antibody-treated, 6 IgG treated and 1 saline-treated transgenic mouse received an intravenous (tail vein) injection of an Aβ stabilizing anti-Aβ antibody (HJ5.1, 150 µg/animal) (purchased from Dr. David M. Holtzman's lab, St.Louis, Missouri (Castellano, J.M., Deane, R., Gottesdiener, A.J., Verghese, P.B., Stewart, F.R., West, T., Paoletti, A.C., Kasper, T.R., DeMattos, R.B., Zlokovic, B.V., et al. 2012. Low-density lipoprotein receptor overexpression enhances the rate of brain-to-blood Abeta clearance in a mouse model of beta-amyloidosis. Proc Natl Acad Sci U S A 109:15502-15507)) that only marginally enters brain parenchyma and does not affect the Aβ-brain-to-blood-equilibrium to prevent the naturally occurring rapid Aβ degradation in blood (*t*_{*1*/*2*}=2-3 min) which precludes a direct and sensitive quantification of brain-derived Aβ over baseline circulating plasma Aβ. 1 anti-KLK8 antibody-treated versus 1 IgG treated transgenic mouse received saline as control. 10 and 40 minutes after HJ5.1-antibody or saline injection blood was collected again. Pump implantation, intravenous injection and retrobulbar venous blood collection was conducted in anesthetized mice with 2% isoflurane in oxygen/nitrous oxide (20%:40%) using a vaporizer. Afterwards, mice were sacrificed (in deep anesthesia). Frontal cortex, hippocampus, amygdala (basolateral & central nuclei), and basal ganglia were isolated and separately homogenized from one hemisphere. Homogenates were subjected to DNA, RNA and protein extraction (TRIzol-reagent). From the remaining intact hemisphere the frontal pole (+4 to +1.5 Bregma) was impregnated with Golgi-Cox solution (PK401-A, FD Rapid GolgiStain Kit, FD NeuroTechnologies) and subsequently cut into 200 µm coronal sections. The rest of the brain hemisphere was formalin-fixed, paraffin embedded and cut into 10 µm coronal sections for immunohistochemistry (for experimental setting see also Fig. 3a).

### In vitro approach

Between 14 days *in vitro* (DIV14) and DIV21, primary mixed glial cell cultures (50%:50% microglia:astroglia) from whole cerebrum of neonatal (P0) transgenic or wildtype mice were characterized for the presence of extracellular and intracellular KLK8 (1:200, ABIN759116, antibodies-online.com) and for intracellular EPHB2 (1:100, AF467) via immunoblot as well as for intraglial and extraglial Aβ₄₀ and Aβ₄₂ by ELISA (Aβ₄₀ and Aβ₄₂, KHB3482 and KHB3442, Invitrogen).

At DIV17, primary mixed glia (50%:50% microglia:astroglia, 250,000 cells per well for lysates, 60,000 cells per well for immunofluorescence cytochemistry, 100,000 cells per well for autophagy monitoring, 150,000 cells per well for cell viability monitoring) were incubated either with anti-KLK8 antibody (M021-3) or control IgG antibody (MCA1124R) at a concentration of 5 µg/ml (dialysed against PBS as described above). At DIV18, Aβ₄₂ (100 ng/ml, 72071, SensoLyte Fluorescent Aβ₄₂ Sampler Kit, Anaspec) as well as fresh antibody (2.5 µg/ml) were added to the medium (41966-029, DMEM, 16050-122, 10% horse serum, 15140-122, 1% Penicillin/Streptomycin, all from Thermo Fisher Scientific). At DIV19 and DIV20, antibody but not Aβ₄₂ treatment was repeated. Supernatant and cell lysates were collected at DIV17 to DIV 21. To test whether effects of anti-KLK8 antibody-treatment were transduced by the EPHB2 receptor, we simultaneously co-incubated anti-KLK8-treated glial cells with an inhibitory anti-EPHB2 antibody (2 µg/ml, AF467, R&D Systems) (for experimental setting see also Fig. 9a).

### Outcome measures

### Descriptive approach

Cerebral protein levels of EPHB2 (1:100, AF467), EFNB2 (murine: 1:500, AF496, R&D Systems; human: 1:100, ab96264, abcam), KLK8 (1:100, ABIN759116), FKBP5 (murine: 1:500, human: 1:200, ab2901, abcam), beclin-1 (1:2,000, ab62557, abcam), and STX17 (1:1,000, 17815-1-AP, Acris) were quantified by immunoblot. Protein levels of GAPDH (1:15,000, G9545, Sigma-Aldrich), actin β (1:15,000, A5441, Sigma-Aldrich) or determination of total protein load via fluorescent gel electrophoresis (TGX stain free gels, 161-0183, Bio-Rad) served for normalization. Human KLK8 protein levels in CSF and serum were determined by ELISA (#EK0919, Boster Biological Technology). Transcription levels of murine and human *KLK8, FKBP5, EPHB2* and *EFNB2* were measured via quantitative real-time PCR (TaqMan® assays, ABI 7500 Fast real-time PCR cycler, Applied Biosystems) following reverse transcription of DNase-treated RNA extracts to cDNA (ABI High-Capacity RT Kit, #4368814, Applied Biosystems) and normalized against *GAPDH* or cytochrome c-1 (*CYC1*). Intron-spanning forward and reverse primer sequences and the TaqMan probe forward sequences are available upon request.

### In vivo approach

Sufficient cerebral penetration of anti-KLK8 antibody or control IgG antibody (both antibodies produced in rat) following intraventricular delivery was validated in lysates from frontal cortex, basal ganglia, hippocampus, and amygdala by visualization of rat immunoglobulins with secondary anti-rat-HRP antibody (1:5,000, A9037, Sigma-Aldrich) via immunoblotting.

Protein levels of EPHB2 (1:100, AF467), synaptophysin (1:10,000, M7315, DAKO), GAP43 (1:4,000, GTX11136, GeneTex), ARC (1:500, sc-15325, Santa Cruz Biotechnology), FKBP5 (1:500, ab2901), glucocorticoid receptor (1:1,000, ab109022, abcam), APP (and the processing fragment CTFβ, 1:2,000, A8717, Sigma-Aldrich), MDR1 (1:1,000, APO94346PU-N, Acris), LRP1 (1:500, 438192, Calbiochem), RAGE (1:1,000, SP5151P, Acris), beclin-1 (1:2,000, ab62557, abcam), ATG5 (1:500, A0856, Sigma-Aldrich), STX17 (1:1,000, 17815-1-AP, Acris), cathepsin D (1:1,000, ab6313, abcam) and prostaglandin E receptor 2 (1:500, APO01201PU-N, Acris) were determined by immunoblotting. GAPDH served for normalization. Tau phosphorylation levels at amino acids S202/T205 (1:500, AT8, MN1020, Thermo Scientific), S396 (1:1,000, PA5-35455, Thermo Scientific) and S212/214 (1:500, AT100, MN1060, Thermo Scientific), PI3K phosphorylation at T199/T458 (1:2,000, #4228, Cell Signaling Technology), Akt phosphorylation at S473 (1:1,000, #9271, Cell Signaling Technology), GSK3β phosphorylation at S9 (1:1,000, #9336, Cell Signaling Technology) and total protein levels of tau (1:500, tau-5, MAB361, Millipore), Akt (1:1,000, #9272, Cell Signaling Technology), and GSK3β (1:2,000, #9315, Cell Signaling Technology) were determined by immunoblotting. Total protein levels of tau, Akt and GSK3β as well as GAPDH served for normalization.

sAPPa (JP27734, IBL International), Aβ₄₀ and Aβ₄₂ peptide levels were quantified by ELISA in protein homogenates containing 1% SDS.

Transcription levels of *hAPP* were determined via quantitative real-time PCR and normalized against *Gapdh* mRNA levels. Primer and TaqMan probe sequences are available upon request.

For morphometric analyses, a Nikon 80i microscope with an integrated CFI ocular lens (10x magnification), equipped with CFI PLAN ACHROMAT objectives (2x magnification, numerical aperture 0.06; 10x magnification, numerical aperture 0.25), CFI PLAN FLUOR objectives (20x magnification, numerical aperture 0.5; 40x magnification, numerical aperture 0.75) and a PLAN APOCHROMAT VC objective (100x magnification, numerical aperture 1.4) (all objectives from NIKON), a colour digital camera (3/4" chip, 36-bit colour, DV-20, MicroBrightField), and MicroBrightField software were used. Aβ plaques (1:100, 6F/3D, DAKO), microglia (AIF1, 1:100, APO8912PU-N, Acris) and cerebral vessels (laminin, 1:300, L9393, Sigma-Aldrich) were visualized and stereologically quantified in 10 sections (with 100 µm interspace between sections) per staining and animal as previously described (Herring, A., Donath, A., Yarmolenko, M., Uslar, E., Conzen, C., Kanakis, D., Bosma, C., Worm, K., Paulus, W., and Keyvani, K. 2012. Exercise during pregnancy mitigates Alzheimer-like pathology in mouse offspring. FASEB J 26:117-128) with minor modifications. Briefly, Aβ plaque load was quantified at 200x magnification. Aβ plaque volume (i.e., percentage of cerebral volume covered by deposits) and Aβ plaque number (n/mm²) were calculated in an unbiased manner by area fraction fractionator and fractionator (counting frame and grid size, each 500x500 µm), respectively. The number of microglia with a visible soma and blood vessel bifurcations (n/mm²) [(vessel ends + vessel branch point)/2] were determined at 200x magnification via fractionator (counting frame 250x250 µm, grid size 375x375 µm). Additionally, 6 sections per animal were co-stained for Aβ plaques and microglia. The average number of plaques surrounding microglia was calculated using the fractionator probe (counting frame 250x250 µm, grid size 375x375 µm) at 200x magnification. Absolute values were related to the investigated area (Stereo Investigator 11, MicroBrightField). Additionally, 6 sections per animal were co-stained for Aβ plaques and phosphorylated tau (1:80, AT8, MN1020, Thermo Scientific). To determine the proportion of neuritic plaques, the number of AT8-positive Aβ plaques was set in relation to the total number of Aβ plaques (with or without surrounding AT8-positive neurites) using the fractionator probe (counting frame 250x250 µm, grid size 375x375 µm) at 200x magnification (Stereo Investigator 11, MicroBrightField).

The effect of KLK8 inhibition on structural neuronal plasticity was tested in Golgi-Cox impregnated coronal sections. We analyzed 10 pyramidal neurons from layer V frontal cortex (+4 to +1.5 Bregma) per animal. All apical and basal dendrites extending from the neuron soma were traced (Neurolucida 11, MicroBrightField) at 400x magnification for 3D reconstruction. To determine dendritic spine density, spines were counted on four 20 µm long dendritic segments per neuron, i.e. on a proximal apical segment, on a distal apical segment, on a proximal basal segment and on a distal basal segment at 1000x magnification. A proximal segment was defined to start after the 1^{st} dendritic branch point, a distal one after the 3^{rd} branch point. In order to quantify apical and basal dendritic morphology, a morphometric analysis of dendritic number, length and branching complexity was performed (NeuroExplorer, MicroBrightField).

### In vitro approach

The influence of anti-KLK8 antibody on glial EPHB2 protection was validated by quantification of EPHB2-FL in lysates using immunoblotting (1:100, AF467).

Anti-KLK8 antibody triggered autophagy modulation was determined by beclin-1 (1:500, ab62557), ATG5 (1:500, A0856, Sigma-Aldrich), and STX17 (1:1,000, 17815-1-AP) immunoblot and via an autophagy assay (600140, autophagy/cytotoxicity dual staining kit, Cayman Chemical) following manufacturer's instructions analyzed on a fluorescence microplate reader (Flx800, BioTek).

Aβ phagocytosis was determined by quantification of Aβ₄₂ in glial lysates and supernatant by ELISA and microglial Aβ₄₂ uptake visualized by fluorescent Aβ₄₂ and AIF1 immunofluorescence cytochemistry (1:100, APO8912PU-N, Acris Antibodies).

Cell viability was monitored following manufacturer's instructions (9095S, XTT cell viability kit, Cell Signaling Technology). Wildtype and transgenic cells were examined separately. Due to lack of significant differences data of both genotypes were pooled.

### Statistics

Data are presented as means ± SE. Distribution of all data sets was evaluated by 1-sample Kolmogorov-Smirnov test and Q-Q plots. Homogeneity of variance was calculated with the Levene test. Student's t test with Bonferroni correction for multiple testing was applied for the analyses of two groups. For the descriptive approach, multiple groups comparison was analyzed by 2-way ANOVA with aging (in mice: P30-P360, in humans: young versus old) and murine genotype (transgene versus wildtype) or human disease status (AD versus control) as between-subject factors and Bonferroni *post hoc* test. For the experimental *in vivo* approach, multiple groups' comparison was analyzed by 2-way ANOVA with treatment (verum versus control) and genotype (transgene versus wildtype) as between-subject factors and Bonferroni *post hoc* test. Cell culture experiments were analyzed by 2-way ANOVA with treatment (verum versus control) and time as between-subject factors and Bonferroni *post hoc* test. Pearson-correlation analysis was performed to test the strength of association between protein levels of KLK8/EPHB2 signaling members. A significance level (α) of P<0.05 was selected. All tests were performed utilizing the software package SPSS 22 (IBM).

### Study approval

Permission for mice breeding and decapitation (AZ 84-02.04.2014.A488) as well as for animal experiments (G1338/12; AZ 84-02.04.2012.A412) was granted by the local committee LANUV NRW, Germany.
Using *post mortem* human material was approved by the ethics committees of the Medical Faculties, University of Duisburg-Essen, (14-5861-BO) and Ludwig-Maximilians-University Munich (#345-13), Germany. Written informed consent was received from the family members of the dead patients prior to inclusion in the study.

### Results:

### KLK8 overexpression precedes EPHB2 depletion in AD

First, we assessed the temporal and spatial mRNA and protein expression patterns of KLK8, and its downstream cascade members EPHB2, FKBP5 and EFNB2 during the course of AD progression in the hippocampus, frontal cortex, entorhinal region and cerebellum of both transgenic CRND8 and wildtype mice as well as AD patients and neurologically healthy (young and old) controls. In particular, in the hippocampus (Fig. 1) but also in other brain regions (data not shown) KLK8 mRNA (data not shown) and protein were overexpressed long before disease onset at P30 in transgenic mice (Fig. 1a, b) and at CERAD A/Braak I-II stage in patients when compared to age-matched controls (Fig.1c, d). Human KLK8 protein levels increased further with AD progression (CERAD A/Braak I-II versus CERAD C/Braak V-VI, Fig. 1c, d). KLK8 overexpression was followed by a reduction of full-length EPHB2 (EPHB2-FL) receptor levels in the hippocampus of transgenic mice at P210 (Fig. 1a, b) and in patients with moderate AD at CERAD B/Braak III-IV stage (Fig. 1c, d). EPHB2 levels declined also in the murine cerebellum and human frontal cortex, but not in the entorhinal cortex (data not shown). FKBP5 protein levels were up-regulated in the frontal, entorhinal, and cerebellar cortex (data not shown) as well as in the hippocampus very early at P30 in transgenic mice and at CERAD A/Braak I-II stage in humans but declined during the course of disease (Fig. 1a-d). EPHB2 ligand EFNB2 was down-regulated during disease progression, in the murine hippocampus (Fig. 1a-b) and displayed reduced entorhinal and cerebellar levels, only in the later stages of disease in mouse (P210) and man (CERAD C) (data not shown). To determine the potential of KLK8 as a diagnostic biomarker for AD, we further assessed KLK8 protein levels in cerebrospinal fluid (CSF) and in blood serum of the same patients from an independent cohort of AD patients and neurologically healthy, age-matched control subjects. Confirmatory to KLK8 up-regulation in the brain, KLK8 protein levels were elevated also in CSF (Fig. 1e) and blood serum (Fig. 1f) of AD patients.

The exceedingly early and multifocal mRNA and protein up-regulation of the protease KLK8, even in the scarcely AD-affected cerebellum not only explains the subsequent decline of its proteolytic target EPHB2 (supported also by a strong negative correlation between hippocampal KLK8 and EPHB2 protein levels, *r*=-0.663, P<0.001), but even suggests a causal role in the cascade of events leading to AD. We therefore sought to verify whether inhibiting the proteolytic enzyme KLK8 would mitigate Alzheimer's pathology.

### Anti-KLK8 antibody protects EPHB2 from fragmentation

As EPHB2-FL contains the target recognition sequence YGRY, membranous EPHB2-FL is cleaved by extracellular KLK8, resulting in a 70 kDa extracellular, N-terminal fragment (EPHB2-NTF)(Attwood, B.K. et al., supra). After confirmation that a KLK8-inhibiting anti-KLK8 antibody (which had been successfully used previously in a murine epilepsy model (Momota, Y. et al., supra)) but not a control IgG binds to human and murine KLK8 (Fig. 2a), we next validated EPHB2 cleavage by KLK8 in a cell free *in vitro* assay (Fig. 2b, c). Then, we demonstrated that the anti-KLK8 antibody is able to protect EPHB2-FL from fragmentation in the cell free assay (Fig. 2b, c) as well as in primary glial cell culture (Fig. 2d, e). To test the inhibitory efficacy of the anti-KLK8 antibody *in vivo,* anti-KLK8 antibody, control IgG or saline was intraventricularly delivered over a four-week period to 150 day old transgenic and wildtype mice utilizing osmotic pumps. Following confirmation of sufficient cerebral antibody penetration (Fig. 2f), quantification of EPHB2-FL revealed that anti-KLK8 antibody treatment successfully blocked EPHB2 cleavage (Fig. 2g, h). During the treatment period, none of the mice exhibited any signs of adverse reaction. Body mass were not affected by treatment (data not shown).

### KLK8 inhibition reduces fear and improves cognition

Progressive cognitive decline and increased anxiety are the clinical hallmarks of AD. Previous work demonstrated that KLK8-triggered amygdalar EPHB2 fragmentation controls anxiety (Attwood, B.K. et al., supra) and that lentivirus-mediated EPHB2 up-regulation reduces cognitive impairment in transgenic AD mice (Cisse, M. et al., supra). We therefore postulated that cerebral KLK8 inhibition and subsequent EPHB2 protection might exert anxiolytic effects and improve memory performance. Accordingly, following four weeks of antibody delivery, mice were phenotyped for anxiety-related behavior in the Elevated-Plus Maze (EPM), for avoidance and exploratory behavior in the Open Field Test (OF) and for spatial memory in the Barnes Maze Test (BM) (for experimental design see Fig. 3a). Automated video tracking analysis substantiated that antibody-mediated cerebral KLK8 inhibition has anxiolytic effects and increases exploratory behavior in transgenic mice, as anti-KLK8 antibody-treated animals spent more time in the open arms and less time in the closed arms of the EPM (Fig. 3b, c), spent more time in the center and border areas and less time in the corners of the OF arena (Fig. 3e, f), took less time to enter the center area for the first time (Fig. 3h), and showed reduced freezing and increased exploratory behavior (Fig. 3i), when compared to controls (IgG & saline). Evaluation of the BM further revealed that blockade of KLK8 improved spatial memory performance in AD-affected mice, as verum-treated transgenics had reduced latencies (Fig 3j-l), explored fewer wrong holes (Fig. 3m, n), and covered shorter distances (Fig. 3o, p) before escaping through the escape hole at trial 1 on test day 1 (24 h following 2 trials of habituation) as well as on test day 4. Cognitive improvement through KLK8 blockade was not accompanied by changes in motor skills, as the average speed did not differ between the two transgenic groups (data not shown). Wildtype control mice (treated with IgG or saline) showed less anxiety, more exploration activity (Fig. 3b-i) and better cognitive performance (Fig. 3j-p) when compared to transgenic control mice. In contrast to the positive effects of anti-KLK8 antibody administration to transgenics, wildtype mice barely benefited from this treatment. Although anti-KLK8 treatment elicited anxiolytic effects also in wildtypes, as they spent less time in the closed arms of the EPM (Fig. 3b, c) and exhibited increased exploratory behavior in the OF (Fig. 3e, i), their memory performance deteriorated significantly, accompanied by a tendency to hyperactivity. They showed increased latencies (Fig 3j-l), explored more wrong holes (Fig. 3m, n), and covered longer distances (Fig. 3o, p) before escaping through the escape hole at trial 1 on test day 1 and on test day 4, presumably due to hyperactivity as indicated by increased total distance travelled in the EPM, OF, and BM arenas (Fig. 3d, g, p).

### KLK8 inhibition reverses the molecular signatures of anxiety and enhances structural neuroplasticity

Next, we verified the effect of anti-KLK8 antibody treatment on molecular and structural signatures of anxiety and cognition. The first step was to quantify the protein levels of anxiety-modulating FKBP5 and glucocorticoid receptor in the amygdala (as published in (Attwood, B.K. et al., supra)) and in the frontal cortex (an area which is not ostensibly involved in anxiety control). In line with the anxiolytic effect of KLK8 inhibition, anti-KLK8 antibody treatment strongly suppressed FKBP5 and glucocorticoid receptor expression in the amygdala of both transgenic and wildtype mice (Fig. 4a, b). In the frontal cortex, antibody administration influenced neither FKBP5 nor glucocorticoid receptor expression (Fig. 4c, d). In the next step, we measured the protein levels of structural neuroplasticity markers synaptophysin (SYP), GAP43 and ARC in the hippocampus and frontal cortex. In both transgenic (but not wildtype) brain areas, KLK8 inhibition increased the expression of the synaptogenesis and growth cone markers SYP and GAP43 (but not ARC, Fig. 4e-h). Accordingly, we determined the spine density and complexity of dendritic branching in Golgi-Cox-impregnated pyramidal neurons from layer V frontal cortex (utilizing Neurolucida and Neuro Explorer software) and could corroborate increased spine density in anti-KLK8 antibody-treated transgenics (but not wildtypes) (Fig. 4i₁₋₂, j), as well as elevated dendritic complexity following cerebral KLK8 blockade in both genotypes (Fig. 4i₃₋₆, k-m).

### KLK8 blockade counteracts Aβ pathology

To determine whether blockade of KLK8 might reverse AD-related Aβ pathology, we investigated APP metabolism and Aβ plaque pathology in the frontal cortex and in the later and less affected basal ganglia. In both areas four weeks of anti-KLK8 antibody administration increased APP-FL levels (without affecting the transcription of the *hAPP* transgene), decreased APP C-terminal fragments β (CTFβ) and Aβ₄₂ peptide concentration, while Aβ₄₀ and sAPPa peptide levels remained unaffected (Fig. 5a-e). These results indicate that blockade of KLK8 impedes amyloidogenic APP processing. We then tested whether changes in APP metabolism led to changes in Aβ plaque burden. Stereological quantification revealed that in the basal ganglia, KLK8 blockade diminished the total volume and average size (but not the total number) of diffuse Aβ plaques (which gradually evolve into core plaques), without affecting the load of the longer established core plaques, whereas in the frontal cortex plaque load remained unchanged (Fig. 5f-j). The fact that anti-KLK8 antibody treatment affected only those plaques with a younger history in the plaque ontogeny implies that KLK8 inhibition interferes with early steps in the amyloid cascade. Taken together, cerebral KLK8 inhibition seems to suppress amyloidogenic APP processing and the subsequent Aβ production, thereby counteracting Aβ aggregation into diffuse plaques.

### KLK8 inhibition improves neurovascular function

As EPHB2 induces angiogenesis, we hypothesized that cerebral KLK8 inhibition and the subsequent protection of EPHB2 from fragmentation might have improved the function of the neurovascular unit. Stereological evaluation of cerebral blood vessel branching in the frontal cortex and basal ganglia (hereafter referred to as cerebral) revealed a pro-angiogenic effect of KLK8 inhibition, only in wildtype mice but not in the transgenics (Fig. 6a, b). Next, we determined the impact of KLK8 inhibition on the cerebral expression pattern of the BBB-located Aβ efflux-conducting LRP1 and MDR1 (also known as P-glycoprotein 1), and the Aβ influx receptor RAGE. RAGE didn't change in transgenics but in wildtypes, presumably due to the general increase of vessel density. Cerebral LRP1 and MDR1 protein levels increased (the first one in transgenics by trend) following anti-KLK8 antibody delivery (Fig. 6c, d), suggesting facilitated elimination of cerebral Aβ via BBB-mediated clearance. To directly test this hypothesis, we determined the kinetics of Aβ efflux from the brain to blood in transgenics, utilizing a modified protocol from Castellano et al. (Castellano, J.M., Deane, R., Gottesdiener, A.J., Verghese, P.B., Stewart, F.R., West, T., Paoletti, A.C., Kasper, T.R., DeMattos, R.B., Zlokovic, B.V., et al. 2012. Low-density lipoprotein receptor overexpression enhances the rate of brain-to-blood Abeta clearance in a mouse model of beta-amyloidosis. Proc Natl Acad Sci U S A 109:15502-15507). 10 and 40 min (=*t₁₀* and *t₄₀*) after intravenous injection of an Aβ stabilizing anti-Aβ antibody (HJ5.1, enters brain parenchyma only marginally, does not affect the Aβ-brain-to-blood-equilibrium, and protects Aβ from enzymatic digestion), blood plasma Aβ₄₀ and Aβ₄₂ levels were determined. Anti-KLK8 antibody treatment increased plasma Aβ₄₀ at *t₁₀* (and at *t₄₀* by trend) as well as Aβ₄₂ at *t₄₀,* indicating improved Aβ clearance across the BBB (Fig. 6e).

### KLK8 blockade induces autophagy and Aβ phagocytosis

Autophagy-controlled intracellular clearance of organelles, proteins and peptides is impaired in the brains of AD patients and transgenic mice. EPHB2 induction appears to promote autophagy under neoplastic conditions. We therefore asked whether KLK8 inhibition and the resulting EPHB2 protection are able to counteract autophagy deficits in the AD affected brain. First, we corroborated an AD-related cerebral perturbation of autophagy modulators, i.e. beclin-1, involved in the initiation of autophagosome assemblies, and STX17, a protein that triggers fusion of autophagosomes with lysosomes in both mouse (Fig. 7a, b) and man (Fig. 7c, d).

The next step was to assess the protein levels of beclin-1, ATG5 (essential for the autophagosome assembly) and STX17 in anti-KLK8 antibody versus IgG-treated mice. In transgenics (and to a lesser extend in wildtypes), KLK8 inhibition elevated the levels of beclin-1 and ATG5 in the frontal cortex and basal ganglia (Fig. 8a-d). By rescuing the autophagy machinery, anti-KLK8 antibody treatment also reversed the cortical accumulation of intraneuronal cathepsin D (Fig. 8e-g) - a lysosomal enzyme, which is present in excess in murine and human AD affected brain. The levels of cathepsin D were lower in the basal ganglia *per se,* and remained unaffected by treatment (Fig. 8e, f).

Impaired Aβ phagocytosis and dysfunctional beclin-1-associated autophagy in the AD affected brain is tightly linked to reduced microglial activity. Accordingly, we examined the effect of anti-KLK8 antibody treatment on primary transgenic and wildtype microglial/astroglial co-cultures (for experimental design see Fig. 9a) after demonstrating KLK8 secretion and EPHB2 expression (but virtually no Aβ generation) in these naïve cells (Fig. 9b). While incubation with Aβ₄₂ (at d1) knocked down the expression levels of glial autophagy molecules beclin-1, ATG5, and STX17 and weakened fluorescence emission in the autophagy assay, simultaneous co-treatment with anti-KLK8 antibody protected the autophagy machinery in both transgenic and wildtype glial cells (Fig. 9c-f). Of note, anti-KLK8 antibody treatment doubled intramicroglial Aβ₄₂ levels (co-localizing with microglial marker AIF1, Fig. 9g, i), while Aβ₄₂ levels in the supernatant were reduced when compared to IgG control (Fig. 9h, i), pinpointing an enhanced clearance of extracellular Aβ₄₂ via microglial phagocytosis.

To test whether the positive effects of KLK8 inhibition on autophagy protection and Aβ clearance were transduced by EPHB2 receptor, we co-incubated anti-KLK8 antibody-treated glial cells with an EPHB2 inhibitory antibody (Attwood, B.K. et al., supra). In spite of anti-KLK8-antibody presence, inhibition of EPHB2 abolished autophagy and reduced microglial Aβ clearance to levels indistinguishable from or even, below IgG control treated cells, underlining the decisive role of EPHB2 in this context (Fig. 10a-f). Prolonged cell viability monitoring for up to 11 days of treatment revealed that neither anti-KLK8 antibody nor anti-EPHB2 antibody affected glial survival or proliferation (Fig. 10g). A genotype-specific difference in basal autophagy and Aβ phagocytosis efficacy could not be detected in primary glia (data not shown), supporting the data of previous publications that microglial functional impairment coincides with amyloid deposition and does not precede it.

Next, we searched for evidence of an enhanced microglial Aβ phagocytosis triggered by KLK8 inhibition *in vivo.* Stereological quantification revealed an increase in the total number of activated AlF1-positive microglia in the basal ganglia (but not frontal cortex) of transgenic (but not wildtype) mice (Fig. 11a, b). Additionally, the average number of plaques surrounding microglia was elevated in the basal ganglia (but not in frontal cortex) in verum-treated transgenics (Fig. 11c, d). As the expression of the pro-inflammatory prostaglandin E receptor 2 (PTGER2) was not affected by anti-KLK8 antibody treatment (Fig. 11e-h), the utilisation of anti-KLK8 antibody seems to promote the proliferation of phagocytic rather than cytotoxic microglia, plaque approximation and subsequent Aβ uptake also *in vivo.* Together, our *in vitro* and *in vivo* data strongly support that KLK8 inhibition induces autophagy and Aβ clearance via microglial phagocytosis.

### KLK8 blockade counteracts tau pathology

Although TgCRND8 mice are not a model of primary tau pathology, they display abnormal tau processing as a consequence of Aβ pathology (A. Bellucci, M.C. Rosi, C. Grossi, A. Fiorentini, I. Luccarini, and F. Casamenti, Abnormal processing of tau in the brain of aged TgCRND8 mice. Neurobiol Dis 2007;27:328-338). As it has been recently shown that ligand-triggered stimulation of EPHB2 attenuates tau hyperphosphorylation in tau-transgenic mice (Jiang J, Wang ZH, Qu M, Gao D, Liu XP, Zhu LQ, et al. (2015): Stimulation of EphB2 attenuates tau phosphorylation through PI3K/Akt-mediated inactivation of glycogen synthase kinase-3beta. Sci Rep 5: 11765), we next examined whether KLK8 inhibition and subsequent EPHB2 restoration might reverse Aβ-related tau hyperphosphorylation in TgCRND8 mice as well. We found that four weeks of anti-KLK8 antibody administration reduced the ratio of neuritic plaques (containing phospho-tau positive dystrophic neurites) in relationship to total number of Aβ plaques (Fig. 12a, b) and lessened tau phosphorylation at amino acids S202/T205, S396 and S212/214 (Fig. 12c, d). The neuroprotective effect of anti-KLK8 therapy against tau hyperphosphorylation was mediated by activation of PI3K (indicated by increased phosphorylation of PI3K at T199/T458) as well as Akt (indicated by increased phosphorylation of Akt at S473), and thus down-stream inhibition of GSK3β (indicated by increased phosphorylation of GSK3β at S9) (Fig. 12e, f). Taken together, KLK8 inhibition and resulting EPHB2 protection induces PI3K/Akt signaling and suppresses GSK3β kinase activity, thereby diminishing AD-associated tau pathology (Fig. 12 g).

### Discussion:

We demonstrate here an AD-related KLK8 increase and EPHB2 depletion in both murine and human hippocampus. We surprisingly show a sustained rise in KLK8 mRNA and protein levels in different brain regions, prior to Aβ pathology onset in mice and at a preclinical stage in humans, before the depletion of its proteolytic target EPHB2 occurs. Further, we could assess increased KLK8 levels in CSF and serum of AD patients. This exceedingly early and multifocal event suggests a key role for KLK8 in the pathogenesis of AD. Accordingly, we provide evidence that inhibition of KLK8 reduces amyloidogenic APP processing and Aβ load, counteracts tau pathology, in particular reduces tau hyperphosphorylation and reduces the proportion of neuritic plaques, improves neurovascular function and Aβ clearance across the BBB, enhances autophagy and microglial Aβ phagocytosis, and further diminishes fear and spatial memory deficits (by intervening with their molecular/structural signatures), thereby qualifying KLK8 as a promising therapeutic target for AD treatment.

Our results indicate that KLK8 inhibition interferes with early steps of the amyloid cascade, suggesting possible prophylactic properties in the early disease stages. Conversely, four weeks of antibody application was sufficient to exert therapeutic effects in mice with moderate AD pathology, hinting at potential curative benefits in later disease stages. Further work should also investigate which of the molecular, structural and behavioural improvements are immediate consequences of KLK8 blockade, secondary effects of EPHB2 protection, or tertiary signs of integral amelioration. For example, the effects on the autophagy machinery and microglial Aβ clearance appear to be directly attributable to anti-KLK8 treatment and EPHB2-dependent. Treatment-induced anxiolytic effects are probably also the immediate consequence of reduced KLK8 activity and diminished EPHB2 fragmentation, resulting in FKBP5 and glucocorticoid receptor suppression. Contrastingly, the effects of anti-KLK8 treatment on Aβ generation/Aβ aggregation/neurovascular dysfunction could arise from impacting only one member of this loop, and subsequently affecting other components by breaking the ongoing "vicious circle". If this is the case, the question remains as to which component is the initial target. Furthermore, EPHB2-dependent and EPHB2-independent effects of KLK8 blockade should be distinguished, in particular, because KLK8 has additional substrates with possible AD implications besides EPHB2. A BLAST analysis of the KLK8 target recognition sequence YGRY identified eight putative and one assured KLK8 substrates. Two of these proteins, i.e. fibronectin and steroid 5 alpha-reductase 1 play a role in AD and are therefore candidates for further testing in the context of anti-KLK8 therapy.

Ultimately, the most intriguing issue remains to verify the bench-to-bedside translational potential of this novel therapeutic approach.

### Summary:

Memory loss and increased anxiety are the clinical hallmarks of Alzheimer's disease (AD). Kallikrein-8 (KLK8) is a serine protease, implicated in synaptic plasticity and memory acquisition as well as in anxiety-related behavior.

Here, we surprisingly demonstrate a drastic up-regulation of KLK8 mRNA and protein in human and murine brain at incipient stages of Alzheimer's pathology, long before any behavioral signs of disease appear. In transgenic mice, KLK8 inhibition enhanced structural plasticity and reversed the molecular signatures of anxiety, along with improving memory and reducing fear. Moreover, it intervened in amyloid β (Aβ) metabolism by impeding amyloidogenic amyloid precursor protein (APP) processing, facilitating Aβ clearance across the blood-brain-barrier and boosting the autophagy machinery, and thereby reducing cerebral Aβ load. Additionally, KLK8 blockade mitigated tau phosphorylation signaling, abnormal tau hyperphosphorylation as well as neuritic plaque burden. At least partially, these effects were transduced by restoration of the ephrin receptor B2 (EPHB2) - a substrate of KLK8, as *in vitro* blockade of EPHB2 abolished the positive effects of KLK8 inhibition on microglial amyloid clearance. These results surprisingly identify KLK8 as a promising new therapeutic target against AD.

Our results indicate that KLK8 inhibition interferes with early steps of the amyloid cascade, suggesting possible prophylactic properties in the early disease stages. Conversely, 4 weeks of antibody application was sufficient to exert therapeutic effects in mice with moderate AD pathology, hinting at potential curative benefits also in later disease stages. Moreover, KLK8 should be evaluated as an antecedent blood and/or cerebrospinal fluid (CSF) biomarker in mild cognitive impairment (MCI) patients, as its cerebral expression is already increased in preclinical stages of AD along with increased CSF and serum levels in later stages of the disease.

## Claims

1. An antibody or functionally active part thereof comprising at least one antigen binding fragment of the antibody which specifically binds to Kallikrein-8 and inhibits the proteolytic activity of Kallikrein-8 for use in the treatment or prevention of Alzheimer's disease.

2. The antibody or functionally active part thereof for use of claim 1, wherein the antibody or functionally active part thereof is selected from a monoclonal antibody, chimeric antibody, human antibody, humanized antibody, Fab, a Fab', a F(ab')2, a Fv, a disulfide-linked Fv, a scFv, a (scFv)2, a bivalent antibody, a bispecific antibody, a multispecific antibody, a diabody, a triabody, a tetrabody and a minibody.

3. The antibody or functionally active part thereof for use according to claim 1 or 2,
(a) wherein the antibody or functionally active part thereof is bound to a compound which is able to transport the antibody or functionally active part thereof across the blood-brain barrier,
preferably wherein the antibody or functionally active part thereof is bound to the compound covalently or non-covalently, and/or in from of a fusion protein, conjugate, complex, liposome or nanoparticle,
more preferably wherein the antibody or functionally active part thereof is bound to an antibody which specifically binds to the Transferrin receptor (Tfr) or a functionally active part thereof comprising at least one antigen binding fragment of the antibody,
and/or
(b) wherein the antibody or functionally active part thereof is administered intravenously, nasally, orally, by implantation into the brain or by intraventricular delivery,
and/or
(c) wherein the patient exhibits an increased level of Kallikrein-8 and/or kallikrein-8 mRNA in brain tissue and/or in cerebrospinal fluid and/or in the blood,
and/or
(d) wherein the disease symptoms increased anxiety and/or cognitive impairment are prevented or treated.

4. An antibody or functionally active part thereof for use according to any of claims 1 to 3, wherein the antibody or functionally active part thereof is administered to a patient
(a) who has Alzheimer's disease and/or a preclinical stage of Alzheimer's disease,
and/or
(b) is diagnosed to have Alzheimer's disease, or a preclinical stage of Alzheimer's disease,
and/or
(c) is identified to be responsive to treatment with an antibody or functionally active part thereof comprising at least one antigen binding fragment of the antibody which specifically binds to Kallikrein-8 and inhibits the proteolytic activity of Kallikrein-8, preferably wherein the patient is identified to have an increased level of Kallikrein 8 and/or kallikrein 8 mRNA in at least one bodily sample and/or in at least one bodily tissue.

5. The antibody or functionally active part thereof for use according to claim 4,
wherein the patient has Alzheimer's disease, or a preclinical stage of Alzheimer's disease at the start of treatment, and/or
is diagnosed to have Alzheimer's disease, or a preclinical stage of Alzheimer's disease at the start of treatment.

6. The antibody or functionally active part thereof for use according to any of claims 1 to 5,
(1) wherein administration to a patient results in reduced amyloidogenic APP processing and/or reduced Aβ load and/or reduced Tau hyperphosphorylation and/or a decreased proportion of neuritic plaques and/or improved neurovascular function and/or improved Aβ clearance across the blood-brain-barrier, and/or enhanced autophagy and/or enhanced microglial Aβ phagocytosis,
and/or
(2) wherein prevention of Alzheimer's disease is attenuating the severity or delaying the onset of at least one clinical symptom of the Alzheimer's disease, in particular increased anxiety and/or cognitive impairment,
and/or
(3) wherein treatment of Alzheimer's disease is mitigation of at least one symptom of Alzheimer's disease, in particular mitigation of increased anxiety and/or cognitive impairment,
and/or
(4) wherein the antibody or functionally active part thereof inhibits proteolytic fragmentation by Kallikrein-8 of at least one protein comprising the sequence YGRY (SEQ ID No: 1), preferably wherein the at least one protein is selected from EPHB2, steroid 5 alpha-reductase 1, casein, fibronectin, collagen type IV, fibrinogen, kininogen, neuregulin-1, CAM-L1, single-chain tPA, PAR2, pro-KLK1 and pro-KLK11.

7. An antibody or functionally active part thereof for use according to any of claims 1 to 6, wherein the antibody or functionally active part thereof is comprised in a pharmaceutical composition which further comprises at least one pharmaceutically acceptable excipient, preferably the antibody or functionally active part thereof is comprised in a pharmaceutical solution, in particular a pharmaceutical saline solution, in a pharmaceutical solution suitable for intraventricular administration or in a pharmaceutical composition suitable for intravenous, nasal or oral administration or for administration by implantation into the brain.

## Patentansprüche

1. Antikörper oder funktionell aktiver Teil davon, umfassend mindestens ein Antigen-bindendes Fragment des Antikörpers, der spezifisch an Kallikrein-8 bindet und die proteolytische Aktivität von Kallikrein-8 inhibiert, zur Verwendung bei der Behandlung oder Vorbeugung der Alzheimer-Erkrankung.

2. Antikörper oder funktionell aktiver Teil davon zur Verwendung nach Anspruch 1, wobei der Antikörper oder der funktionell aktive Teil davon ausgewählt ist aus einem monoklonalen Antikörper, chimären Antikörper, humanen Antikörper, humanisierten Antikörper, Fab, einem Fab', einem F(ab')2, einem Fv, einem disulfid-verknüpften Fv, einem scFv, einem (scFv)2, einem bivalenten Antikörper, einem bispezifischen Antikörper, einem multispezifischen Antikörper, einem Diabody, einem Triabody, einem Tetrabody und einem Minibody.

3. Antikörper oder funktionell aktiver Teil davon zur Verwendung gemäß Anspruch 1 oder 2,
(a) wobei der Antikörper oder der funktionell aktive Teil davon an eine Verbindung gebunden ist, die in der Lage ist, den Antikörper oder den funktionell aktiven Teil davon über die Blut-Hirn-Schranke zu transportieren,
vorzugsweise wobei der Antikörper oder der funktionell aktive Teil davon kovalent oder nicht-kovalent an die Verbindung gebunden ist, und/oder in Form eines Fusionsproteins, Konjugats, Komplexes, Liposoms oder Nanopartikels,
stärker bevorzugt wobei der Antikörper oder der funktionell aktive Teil davon an einen Antikörper gebunden ist, der spezifisch an den Transferrin-Rezeptor (Tfr) bindet, oder an einen funktionell aktiven Teil davon, der mindestens ein Antigen-bindendes Fragment des Antikörpers umfasst,
und/oder
(b) wobei der Antikörper oder der funktionell aktive Teil davon intravenös, nasal, oral, durch Implantation in das Gehirn oder durch intraventrikuläre Zuführung verabreicht wird,
und/oder
(c) wobei der Patient einen erhöhten Spiegel von Kallikrein-8 und/oder Kallikrein-8 mRNA im Hirngewebe und/oder in der Zerebrospinalflüssigkeit und/oder im Blut zeigt,
und/oder
(d) wobei die Erkrankungssymptome erhöhte Angst und/oder kognitive Beeinträchtigung vorgebeugt oder behandelt werden.

4. Antikörper oder funktionell aktiver Teil davon zur Verwendung nach einem beliebigen der Ansprüche 1 bis 3, wobei der Antikörper oder der funktionell aktive Teil davon einem Patienten verabreicht wird
(a) der die Alzheimer Erkrankung und/oder ein präklinisches Stadium der Alzheimer Erkrankung hat,
und/oder
(b) der diagnostiziert ist, die Alzheimer Erkrankung oder ein präklinisches Stadium der Alzheimer Erkrankung zu haben,
und/oder
(c) der identifiziert ist/wird, auf eine Behandlung mit einem Antikörper oder einem funktionell aktiven Teil davon anzusprechen, der mindestens ein Antigen-bindendes Fragment des Antikörpers umfasst, der spezifisch an Kallikrein-8 bindet und die proteolytische Aktivität von Kallikrein-8 inhibiert, vorzugsweise wobei der Patient identifiziert ist/wird, einen erhöhten Spiegel von Kallikrein 8 und/oder Kallikrein-8 mRNA in mindestens einer Körperprobe und/oder in mindestens einem Körpergewebe aufzuweisen.

5. Antikörper oder funktionell aktiver Teil davon zur Verwendung gemäß Anspruch 4,
wobei der Patient zu Beginn der Behandlung die Alzheimer Erkrankung oder ein präklinisches Stadium der Alzheimer Erkrankung hat, und/oder eine Alzheimer Erkrankung oder ein präklinisches Stadium der Alzheimer Erkrankung zu Beginn der Behandlung diagnostiziert ist/wird.

6. Antikörper oder funktionell aktiver Teil davon zur Verwendung gemäß einem beliebigen der Ansprüche 1 bis 5,
(1) wobei die Verabreichung an einen Patienten zu einer reduzierten amyloidogenen APP-Prozessierung (*amyloidogenic APP processing)* und/oder einer reduzierten Aß-Last und/oder einer reduzierten Tau-Hyperphosphorylierung und/oder einem verringerten Anteil an neuritischen Plaques und/oder einer verbesserten neurovaskulären Funktion und/oder einer verbesserten Aβ-Clearance über die Blut-Hirn-Schranke und/oder einer verstärkten Autophagie und/oder einer verstärkten mikroglialen Aβ-Phagozytose führt,
und/oder
(2) wobei die Vorbeugung der Alzheimer Erkrankung das Abschwächen der Schwere oder das Verzögen des Auftretens von mindestens einem klinischen Symptom der Alzheimer Erkrankung ist, insbesondere erhöhte Angst und/oder kognitive Beeinträchtigung,
und/oder
(3) wobei die Behandlung der Alzheimer Erkrankung die Linderung mindestens eines Symptoms der Alzheimer Erkrankung ist, insbesondere die Linderung von erhöhter Angst und/oder kognitiver Beeinträchtigung,
und/oder
(4) wobei der Antikörper oder der funktionell aktive Teil davon die proteolytische Fragmentierung durch Kallikrein-8 von mindestens einem Protein inhibiert, das die Sequenz YGRY (SEQ ID Nr. 1) umfasst, wobei das mindestens eine Protein vorzugsweise ausgewählt ist aus EPHB2, Steroid-5-alpha-Reduktase 1, Casein, Fibronektin, Kollagen Typ IV, Fibrinogen, Kininogen, Neuregulin-1, CAM-L1, einzelkettigem (*single-chain*) tPA, PAR2, pro-KLK1 und pro-KLK11.

7. Antikörper oder funktionell aktiver Teil davon zur Verwendung gemäß einem beliebigen der Ansprüche 1 bis 6, wobei der Antikörper oder der funktionell aktive Teil davon in einer pharmazeutischen Zusammensetzung umfasst ist, die weiterhin mindestens einen pharmazeutisch verträglichen Hilfsstoff umfasst, vorzugsweise ist der Antikörper oder der funktionell aktive Teil davon in einer pharmazeutischen Lösung, insbesondere einer pharmazeutischen Salzlösung, in einer pharmazeutischen Lösung, die für eine intraventrikuläre Verabreichung geeignet ist, oder in einer pharmazeutischen Zusammensetzung enthalten, die für eine intravenöse, nasale oder orale Verabreichung oder für eine Verabreichung durch Implantation in das Gehirn geeignet ist.

## Revendications

1. Anticorps ou partie fonctionnellement active de celui-ci comprenant au moins un fragment de liaison à l'antigène de l'anticorps qui se lie spécifiquement à la kallikréine-8 et inhibe l'activité protéolytique de la kallikréine-8 pour une utilisation dans le traitement ou la prévention de la maladie d'Alzheimer.

2. Anticorps ou partie fonctionnellement active de celui-ci pour une utilisation selon la revendication 1, l'anticorps ou la partie fonctionnellement active de celui-ci étant choisi parmi un anticorps monoclonal, un anticorps chimérique, un anticorps humain, un anticorps humanisé, Fab, un Fab', un F(ab')2, un Fv, un Fv à liaison disulfure, un scFv, un (scFv)2, un anticorps bivalent, un anticorps bispécifique, un anticorps multispécifique, un diabody, un triabody, un tétrabody et un minibody.

3. Anticorps ou partie fonctionnellement active de celui-ci pour une utilisation selon la revendication 1 ou 2,
(a) l'anticorps ou la partie fonctionnellement active de celui-ci étant lié à un composé qui est capable de transporter l'anticorps ou la partie fonctionnellement active de celui-ci à travers la barrière hématoencéphalique,
de préférence l'anticorps ou la partie fonctionnellement active de celui-ci étant lié au composé d'une manière covalente ou non covalente, et/ou sous forme d'une protéine de fusion, d'un conjugué, d'un complexe, d'un liposome ou d'une nanoparticule,
plus particulièrement l'anticorps ou la partie fonctionnellement active de celui-ci étant lié à un anticorps qui se lie spécifiquement au récepteur de la transferrine (Tfr) ou à une partie fonctionnellement active de celui-ci comprenant au moins un fragment de liaison à l'antigène de l'anticorps,
et/ou
(b) l'anticorps ou la partie fonctionnellement active de celui-ci étant administré par voie intraveineuse, nasale, orale, par implantation dans le cerveau ou par administration intraventriculaire,
et/ou
(c) le patient présentant un niveau accru de kallikréine-8 et/ou d'ARNm de kallikréine-8 dans le tissu cérébral et/ou dans le liquide céphalorachidien et/ou dans le sang,
et/ou
(d) les symptômes de la maladie anxiété accrue et/ou du trouble cognitif accru sont prévenus ou traités.

4. Anticorps ou partie fonctionnellement active de celui-ci pour une utilisation selon l'une quelconque des revendications 1 à 3, l'anticorps ou la partie fonctionnellement active de celui-ci étant administré à un patient
(a) qui a une maladie d'Alzheimer et/ou un stade préclinique de la maladie d'Alzheimer,
et/ou
(b) est diagnostiqué comme ayant la maladie d'Alzheimer ou un stade préclinique de la maladie d'Alzheimer,
et/ou
(c) est identifié comme répondant à un traitement avec un anticorps ou une partie fonctionnellement active de celui-ci comprenant au moins un fragment de liaison à l'antigène de l'anticorps qui se lie spécifiquement à la kallikréine-8 et inhibe l'activité protéolytique de la kallikréine-8, de préférence le patient étant identifié comme ayant un niveau accru de kallikréine-8 et/ou d'ARNm de la kallikréine-8 dans au moins un échantillon corporel et/ou dans au moins un tissu corporel.

5. Anticorps ou partie fonctionnellement active de celui-ci pour une utilisation selon la revendication 4, le patient ayant la maladie d'Alzheimer ou un stade préclinique de la maladie d'Alzheimer au début du traitement, et/ou
étant diagnostiqué comme ayant la maladie d'Alzheimer ou un stade préclinique de la maladie d'Alzheimer au début du traitement.

6. Anticorps ou partie fonctionnellement active de celui-ci pour une utilisation selon l'une quelconque des revendication 1 à 5,
(1) l'administration à un patient conduisant à une réduction du traitement de l'APP amyloïdogène et/ou une réduction de la charge Aβ et/ou une réduction de l'hyperphosphorylation de Tau et/ou une diminution de la proportion de plaques neuritiques et/ou une amélioration de la fonction neurovasculaire et/ou une clairance améliorée de l'Aβ à travers la barrière hématoencéphalique, et/ou une amélioration de l'autophagie et/ou une amélioration de la phagocytose microgliale d'Aβ,
et/ou
(2) la prévention de la maladie d'Alzheimer atténuant la gravité et retardant l'apparition d'au moins un symptôme clinique de la maladie d'Alzheimer, en particulier une augmentation de l'anxiété et/ou du trouble cognitif,
et/ou
(3) le traitement de la maladie d'Alzheimer étant une mitigation d'au moins un symptôme de la maladie d'Alzheimer, en particulier une mitigation de l'augmentation de l'anxiété et/ou du trouble cognitif,
et/ou
(4) l'anticorps ou la partie fonctionnellement active de celui-ci inhibant la fragmentation protéolytique par la kallikréine-8 d'au moins une protéine comprenant la séquence YGRY (SEQ ID NO: 1), de préférence l'au moins une protéine étant choisie parmi EPHB2, la stéroïde 5 alpha-réductase 1, la caséine, la fibronectine, le collagène de type IV, le fibrinogène, le kininogène, la neuréguline-1, CAM-L1, le tPA monocaténaire, PAR2, pro-KLK1 et pro-KLK11.

7. Anticorps ou partie fonctionnellement active de celui-ci pour une utilisation selon l'une quelconque des revendications 1 à 6, l'anticorps ou la partie fonctionnellement active de celui-ci étant compris dans une composition pharmaceutique qui comprend en outre au moins un excipient pharmaceutiquement acceptable, de préférence l'anticorps ou la partie fonctionnellement active de celui-ci étant compris dans une solution pharmaceutique, en particulier une solution salée pharmaceutique, dans une solution pharmaceutique convenant à une administration intraventriculaire ou dans une composition pharmaceutique convenant à une administration intraveineuse, nasale ou orale ou pour administration par implantation dans le cerveau.


### SEQUENCE LISTING

<110> Universität Duisburg-Essen
<120> Agents inhibiting Kallikrein-8 for use in the prevention or treatment of Alzheimer's disease
<130> P69914PC
<150> EP 15002769.6
   <151> 2015-09-25
<150> EP 15003657.2
   <151> 2015-12-22
<160> 13
<170> PatentIn version 3.5
<210> 1
   <211> 4
   <212> PRT
   <213> Artificial Sequence
<220>
   <223> substrate motif
<400> 1
   Tyr Gly Arg Tyr
   1
<210> 2
   <211> 260
   <212> PRT
   <213> Mus musculus
<220>
   <221> MISC_FEATURE
   <222> (1)..(260)
   <223> pro-form sequence
<400> 2
   Met Gly Arg Pro Pro Pro Cys Ala Ile Gln Pro Trp Ile Leu Leu Leu
   1               5                   10                  15
   Leu Phe Met Gly Ala Trp Ala Gly Leu Thr Arg Ala Gln Gly Ser Lys
               20                  25                  30
   Ile Leu Glu Gly Arg Glu Cys Ile Pro His Ser Gln Pro Trp Gln Ala
           35                  40                  45
   Ala Leu Phe Gln Gly Glu Arg Leu Ile Cys Gly Gly Val Leu Val Gly
       50                  55                  60
   Asp Arg Trp Val Leu Thr Ala Ala His Cys Lys Lys Gln Lys Tyr Ser
   65                  70                  75                  80
   Val Arg Leu Gly Asp His Ser Leu Gln Ser Arg Asp Gln Pro Glu Gln
                   85                  90                  95
   Glu Ile Gln Val Ala Gln Ser Ile Gln His Pro Cys Tyr Asn Asn Ser
               100                 105                 110
   Asn Pro Glu Asp His Ser His Asp Ile Met Leu Ile Arg Leu Gln Asn
           115                 120                 125
   Ser Ala Asn Leu Gly Asp Lys Val Lys Pro Val Gln Leu Ala Asn Leu
       130                 135                 140
   Cys Pro Lys Val Gly Gln Lys Cys Ile Ile Ser Gly Trp Gly Thr Val
   145                 150                 155                 160
   Thr Ser Pro Gln Glu Asn Phe Pro Asn Thr Leu Asn Cys Ala Glu Val
                   165                 170                 175
   Lys Ile Tyr Ser Gln Asn Lys Cys Glu Arg Ala Tyr Pro Gly Lys Ile
               180                 185                 190
   Thr Glu Gly Met Val Cys Ala Gly Ser Ser Asn Gly Ala Asp Thr Cys
           195                 200                 205
   Gln Gly Asp Ser Gly Gly Pro Leu Val Cys Asp Gly Met Leu Gln Gly
       210                 215                 220
   Ile Thr Ser Trp Gly Ser Asp Pro Cys Gly Lys Pro Glu Lys Pro Gly
   225                 230                 235                 240
   Val Tyr Thr Lys Ile Cys Arg Tyr Thr Thr Trp Ile Lys Lys Thr Met
                   245                 250                 255
   Asp Asn Arg Asp
               260
<210> 3
   <211> 260
   <212> PRT
   <213> Homo sapiens
<220>
   <221> MISC_FEATURE
   <222> (1)..(260)
   <223> pro-form sequence
<400> 3
   Met Gly Arg Pro Arg Pro Arg Ala Ala Lys Thr Trp Met Phe Leu Leu
   1               5                   10                  15
   Leu Leu Gly Gly Ala Trp Ala Gly His Ser Arg Ala Gln Glu Asp Lys
               20                  25                  30
   Val Leu Gly Gly His Glu Cys Gln Pro His Ser Gln Pro Trp Gln Ala
           35                  40                  45
   Ala Leu Phe Gln Gly Gln Gln Leu Leu Cys Gly Gly Val Leu Val Gly
       50                  55                  60
   Gly Asn Trp Val Leu Thr Ala Ala His Cys Lys Lys Pro Lys Tyr Thr
   65                  70                  75                  80
   Val Arg Leu Gly Asp His Ser Leu Gln Asn Lys Asp Gly Pro Glu Gln
                   85                  90                  95
   Glu Ile Pro Val Val Gln Ser Ile Pro His Pro Cys Tyr Asn Ser Ser
               100                 105                 110
   Asp Val Glu Asp His Asn His Asp Leu Met Leu Leu Gln Leu Arg Asp
           115                 120                 125
   Gln Ala Ser Leu Gly Ser Lys Val Lys Pro Ile Ser Leu Ala Asp His
       130                 135                 140
   Cys Thr Gln Pro Gly Gln Lys Cys Thr Val Ser Gly Trp Gly Thr Val
   145                 150                 155                 160
   Thr Ser Pro Arg Glu Asn Phe Pro Asp Thr Leu Asn Cys Ala Glu Val
                   165                 170                 175
   Lys Ile Phe Pro Gln Lys Lys Cys Glu Asp Ala Tyr Pro Gly Gln Ile
               180                 185                 190
   Thr Asp Gly Met Val Cys Ala Gly Ser Ser Lys Gly Ala Asp Thr Cys
           195                 200                 205
   Gln Gly Asp Ser Gly Gly Pro Leu Val Cys Asp Gly Ala Leu Gln Gly
       210                 215                 220
   Ile Thr Ser Trp Gly Ser Asp Pro Cys Gly Arg Ser Asp Lys Pro Gly
   225                 230                 235                 240
   Val Tyr Thr Asn Ile Cys Arg Tyr Leu Asp Trp Ile Lys Lys Ile Ile
                   245                 250                 255
   Gly Ser Lys Gly
   260
<210> 4
   <211> 987
   <212> PRT
   <213> Mus musculus
<400> 4
   Met Ala Val Arg Arg Leu Gly Ala Ala Leu Leu Leu Leu Pro Leu Leu
   1 5 10 15
   Ala Ala Val Glu Glu Thr Leu Met Asp Ser Thr Thr Ala Thr Ala Glu
   20 25 30
   Leu Gly Trp Met Val His Pro Pro Ser Gly Trp Glu Glu Val Ser Gly
   35 40 45
   Tyr Asp Glu Asn Met Asn Thr Ile Arg Thr Tyr Gln Val Cys Asn Val
   50 55 60
   Phe Glu Ser Ser Gln Asn Asn Trp Leu Arg Thr Lys Phe Ile Arg Arg
   65 70 75 80
   Arg Gly Ala His Arg Ile His Val Glu Met Lys Phe Ser Val Arg Asp
   85 90 95
   Cys Ser Ser Ile Pro Ser Val Pro Gly Ser Cys Lys Glu Thr Phe Asn
   100 105 110
   Leu Tyr Tyr Tyr Glu Ala Asp Phe Asp Leu Ala Thr Lys Thr Phe Pro
   115 120 125
   Asn Trp Met Glu Asn Pro Trp Val Lys Val Asp Thr Ile Ala Ala Asp
   130 135 140
   Glu Ser Phe Ser Gln Val Asp Leu Gly Gly Arg Val Met Lys Ile Asn
   145 150 155 160
   Thr Glu Val Arg Ser Phe Gly Pro Val Ser Arg Asn Gly Phe Tyr Leu
   165 170 175
   Ala Phe Gln Asp Tyr Gly Gly Cys Met Ser Leu Ile Ala Val Arg Val
   180 185 190
   Phe Tyr Arg Lys Cys Pro Arg Ile Ile Gln Asn Gly Ala Ile Phe Gln
   195 200 205
   Glu Thr Leu Ser Gly Ala Glu Ser Thr Ser Leu Val Ala Ala Arg Gly
   210 215 220
   Ser Cys Ile Ala Asn Ala Glu Glu Val Asp Val Pro Ile Lys Leu Tyr
   225 230 235 240
   Cys Asn Gly Asp Gly Glu Trp Leu Val Pro Ile Gly Arg Cys Met Cys
   245 250 255
   Lys Ala Gly Phe Glu Ala Val Glu Asn Gly Thr Val Cys Arg Gly Cys
   260 265 270
   Pro Ser Gly Thr Phe Lys Ala Asn Gln Gly Asp Glu Ala Cys Thr His
   275 280 285
   Cys Pro Ile Asn Ser Arg Thr Thr Ser Glu Gly Ala Thr Asn Cys Val
   290 295 300
   Cys Arg Asn Gly Tyr Tyr Arg Ala Asp Leu Asp Pro Leu Asp Met Pro
   305 310 315 320
   Cys Thr Thr Ile Pro Ser Ala Pro Gln Ala Val Ile Ser Ser Val Asn
   325 330 335
   Glu Thr Ser Leu Met Leu Glu Trp Thr Pro Pro Arg Asp Ser Gly Gly
   340 345 350
   Arg Glu Asp Leu Val Tyr Asn Ile Ile Cys Lys Ser Cys Gly Ser Gly
   355 360 365
   Arg Gly Ala Cys Thr Arg Cys Gly Asp Asn Val Gln Tyr Ala Pro Arg
   370 375 380
   Gln Leu Gly Leu Thr Glu Pro Arg Ile Tyr Ile Ser Asp Leu Leu Ala
   385 390 395 400
   His Thr Gln Tyr Thr Phe Glu Ile Gln Ala Val Asn Gly Val Thr Asp
   405 410 415
   Gln Ser Pro Phe Ser Pro Gln Phe Ala Ser Val Asn Ile Thr Thr Asn
   420 425 430
   Gln Ala Ala Pro Ser Ala Val Ser Ile Met His Gln Val Ser Arg Thr
   435 440 445
   Val Asp Ser Ile Thr Leu Ser Trp Ser Gln Pro Asp Gln Pro Asn Gly
       450                 455                 460
   Val Ile Leu Asp Tyr Glu Leu Gln Tyr Tyr Glu Lys Gln Glu Leu Ser
   465                 470                 475                 480
   Glu Tyr Asn Ala Thr Ala Ile Lys Ser Pro Thr Asn Thr Val Thr Val
                   485                 490                 495
   Gln Gly Leu Lys Ala Gly Ala Ile Tyr Val Phe Gln Val Arg Ala Arg
               500                 505                 510
   Thr Val Ala Gly Tyr Gly Arg Tyr Ser Gly Lys Met Tyr Phe Gln Thr
           515                 520                 525
   Met Thr Glu Ala Glu Tyr Gln Thr Ser Ile Lys Glu Lys Leu Pro Leu
       530                 535                 540
   Ile Val Gly Ser Ser Ala Ala Gly Leu Val Phe Leu Ile Ala Val Val
   545                 550                 555                 560
   Val Ile Ala Ile Val Cys Asn Arg Arg Gly Phe Glu Arg Ala Asp Ser
                   565                 570                 575
   Glu Tyr Thr Asp Lys Leu Gln His Tyr Thr Ser Gly His Met Thr Pro
               580                 585                 590
   Gly Met Lys Ile Tyr Ile Asp Pro Phe Thr Tyr Glu Asp Pro Asn Glu
           595                 600                 605
   Ala Val Arg Glu Phe Ala Lys Glu Ile Asp Ile Ser Cys Val Lys Ile
       610                 615                 620
   Glu Gln Val Ile Gly Ala Gly Glu Phe Gly Glu Val Cys Ser Gly His
   625                 630                 635                 640
   Leu Lys Leu Pro Gly Lys Arg Glu Ile Phe Val Ala Ile Lys Thr Leu
                   645                 650                 655
   Lys Ser Gly Tyr Thr Glu Lys Gln Arg Arg Asp Phe Leu Ser Glu Ala
               660                 665                 670
   Ser Ile Met Gly Gln Phe Asp His Pro Asn Val Ile His Leu Glu Gly
           675                 680                 685
   Val Val Thr Lys Ser Thr Pro Val Met Ile Ile Thr Glu Phe Met Glu
       690                 695                 700
   Asn Gly Ser Leu Asp Ser Phe Leu Arg Gln Asn Asp Gly Gln Phe Thr
   705                 710                 715                 720
   Val Ile Gln Leu Val Gly Met Leu Arg Gly Ile Ala Ala Gly Met Lys
                   725                 730                 735
   Tyr Leu Ala Asp Met Asn Tyr Val His Arg Asp Leu Ala Ala Arg Asn
               740                 745                 750
   Ile Leu Val Asn Ser Asn Leu Val Cys Lys Val Ser Asp Phe Gly Leu
           755                 760                 765
   Ser Arg Phe Leu Glu Asp Asp Thr Ser Asp Pro Thr Tyr Thr Ser Ala
       770                 775                 780
   Leu Gly Gly Lys Ile Pro Ile Arg Trp Thr Ala Pro Glu Ala Ile Gln
   785                 790                 795                 800
   Tyr Arg Lys Phe Thr Ser Ala Ser Asp Val Trp Ser Tyr Gly Ile Val
                   805                 810                 815
   Met Trp Glu Val Met Ser Tyr Gly Glu Arg Pro Tyr Trp Asp Met Thr
               820                 825                 830
   Asn Gln Asp Val Ile Asn Ala Ile Glu Gln Asp Tyr Arg Leu Pro Pro
           835                 840                 845
   Pro Met Asp Cys Pro Ser Ala Leu His Gln Leu Met Leu Asp Cys Trp
       850                 855                 860
   Gln Lys Asp Arg Asn His Arg Pro Lys Phe Gly Gln Ile Val Asn Thr
   865                 870                 875                 880
   Leu Asp Lys Met Ile Arg Asn Pro Asn Ser Leu Lys Ala Met Ala Pro
                   885                 890                 895
   Leu Ser Ser Gly Ile Asn Leu Pro Leu Leu Asp Arg Thr Ile Pro Asp
               900                 905                 910
   Tyr Thr Ser Phe Asn Thr Val Asp Glu Trp Leu Glu Ala Ile Lys Met
           915                 920                 925
   Gly Gln Tyr Lys Glu Ser Phe Ala Asn Ala Gly Phe Thr Ser Phe Asp
       930                 935                 940
   Val Val Ser Gln Met Met Met Glu Asp Ile Leu Arg Val Gly Val Thr
   945                 950                 955                 960
   Leu Ala Gly His Gln Lys Lys Ile Leu Asn Ser Ile Gln Val Met Arg
                   965                 970                 975
   Ala Gln Met Asn Gln Ile Gln Ser Val Glu Val
               980                 985
<210> 5
   <211> 986
   <212> PRT
   <213> Mus musculus
<400> 5
   Met Ala Val Arg Arg Leu Gly Ala Ala Leu Leu Leu Leu Pro Leu Leu
   1               5                   10                  15
   Ala Ala Val Glu Glu Thr Leu Met Asp Ser Thr Thr Ala Thr Ala Glu
               20                  25                  30
   Leu Gly Trp Met Val His Pro Pro Ser Gly Trp Glu Glu Val Ser Gly
           35                  40                  45
   Tyr Asp Glu Asn Met Asn Thr Ile Arg Thr Tyr Gln Val Cys Asn Val
       50                  55                  60
   Phe Glu Ser Ser Gln Asn Asn Trp Leu Arg Thr Lys Phe Ile Arg Arg
   65                  70                  75                  80
   Arg Gly Ala His Arg Ile His Val Glu Met Lys Phe Ser Val Arg Asp
                   85                  90                  95
   Cys Ser Ser Ile Pro Ser Val Pro Gly Ser Cys Lys Glu Thr Phe Asn
               100                 105                 110
   Leu Tyr Tyr Tyr Glu Ala Asp Phe Asp Leu Ala Thr Lys Thr Phe Pro
           115                 120                 125
   Asn Trp Met Glu Asn Pro Trp Val Lys Val Asp Thr Ile Ala Ala Asp
       130                 135                 140
   Glu Ser Phe Ser Gln Val Asp Leu Gly Gly Arg Val Met Lys Ile Asn
   145                 150                 155                 160
   Thr Glu Val Arg Ser Phe Gly Pro Val Ser Arg Asn Gly Phe Tyr Leu
                   165                 170                 175
   Ala Phe Gln Asp Tyr Gly Gly Cys Met Ser Leu Ile Ala Val Arg Val
               180                 185                 190
   Phe Tyr Arg Lys Cys Pro Arg Ile Ile Gln Asn Gly Ala Ile Phe Gln
   195 200 205
   Glu Thr Leu Ser Gly Ala Glu Ser Thr Ser Leu Val Ala Ala Arg Gly
   210 215 220
   Ser Cys Ile Ala Asn Ala Glu Glu Val Asp Val Pro Ile Lys Leu Tyr
   225 230 235 240
   Cys Asn Gly Asp Gly Glu Trp Leu Val Pro Ile Gly Arg Cys Met Cys
   245 250 255
   Lys Ala Gly Phe Glu Ala Val Glu Asn Gly Thr Val Cys Arg Gly Cys
   260 265 270
   Pro Ser Gly Thr Phe Lys Ala Asn Gln Gly Asp Glu Ala Cys Thr His
   275 280 285
   Cys Pro Ile Asn Ser Arg Thr Thr Ser Glu Gly Ala Thr Asn Cys Val
   290 295 300
   Cys Arg Asn Gly Tyr Tyr Arg Ala Asp Leu Asp Pro Leu Asp Met Pro
   305 310 315 320
   Cys Thr Thr Ile Pro Ser Ala Pro Gln Ala Val Ile Ser Ser Val Asn
   325 330 335
   Glu Thr Ser Leu Met Leu Glu Trp Thr Pro Pro Arg Asp Ser Gly Gly
   340 345 350
   Arg Glu Asp Leu Val Tyr Asn Ile Ile Cys Lys Ser Cys Gly Ser Gly
   355 360 365
   Arg Gly Ala Cys Thr Arg Cys Gly Asp Asn Val Gln Tyr Ala Pro Arg
   370 375 380
   Gln Leu Gly Leu Thr Glu Pro Arg Ile Tyr Ile Ser Asp Leu Leu Ala
   385 390 395 400
   His Thr Gln Tyr Thr Phe Glu Ile Gln Ala Val Asn Gly Val Thr Asp
   405 410 415
   Gln Ser Pro Phe Ser Pro Gln Phe Ala Ser Val Asn Ile Thr Thr Asn
   420 425 430
   Gln Ala Ala Pro Ser Ala Val Ser Ile Met His Gln Val Ser Arg Thr
   435 440 445
   Val Asp Ser Ile Thr Leu Ser Trp Ser Gln Pro Asp Gln Pro Asn Gly
   450 455 460
   Val Ile Leu Asp Tyr Glu Leu Gln Tyr Tyr Glu Lys Glu Leu Ser Glu
   465 470 475 480
   Tyr Asn Ala Thr Ala Ile Lys Ser Pro Thr Asn Thr Val Thr Val Gln
   485 490 495
   Gly Leu Lys Ala Gly Ala Ile Tyr Val Phe Gln Val Arg Ala Arg Thr
   500 505 510
   Val Ala Gly Tyr Gly Arg Tyr Ser Gly Lys Met Tyr Phe Gln Thr Met
   515 520 525
   Thr Glu Ala Glu Tyr Gln Thr Ser Ile Lys Glu Lys Leu Pro Leu Ile
   530 535 540
   Val Gly Ser Ser Ala Ala Gly Leu Val Phe Leu Ile Ala Val Val Val
   545 550 555 560
   Ile Ala Ile Val Cys Asn Arg Arg Gly Phe Glu Arg Ala Asp Ser Glu
   565 570 575
   Tyr Thr Asp Lys Leu Gln His Tyr Thr Ser Gly His Met Thr Pro Gly
   580 585 590
   Met Lys Ile Tyr Ile Asp Pro Phe Thr Tyr Glu Asp Pro Asn Glu Ala
   595 600 605
   Val Arg Glu Phe Ala Lys Glu Ile Asp Ile Ser Cys Val Lys Ile Glu
   610 615 620
   Gln Val Ile Gly Ala Gly Glu Phe Gly Glu Val Cys Ser Gly His Leu
   625 630 635 640
   Lys Leu Pro Gly Lys Arg Glu Ile Phe Val Ala Ile Lys Thr Leu Lys
   645 650 655
   Ser Gly Tyr Thr Glu Lys Gln Arg Arg Asp Phe Leu Ser Glu Ala Ser
   660 665 670
   Ile Met Gly Gln Phe Asp His Pro Asn Val Ile His Leu Glu Gly Val
   675 680 685
   Val Thr Lys Ser Thr Pro Val Met Ile Ile Thr Glu Phe Met Glu Asn
       690                 695                 700
   Gly Ser Leu Asp Ser Phe Leu Arg Gln Asn Asp Gly Gln Phe Thr Val
   705                 710                 715                 720
   Ile Gln Leu Val Gly Met Leu Arg Gly Ile Ala Ala Gly Met Lys Tyr
                   725                 730                 735
   Leu Ala Asp Met Asn Tyr Val His Arg Asp Leu Ala Ala Arg Asn Ile
               740                 745                 750
   Leu Val Asn Ser Asn Leu Val Cys Lys Val Ser Asp Phe Gly Leu Ser
           755                 760                 765
   Arg Phe Leu Glu Asp Asp Thr Ser Asp Pro Thr Tyr Thr Ser Ala Leu
       770                 775                 780
   Gly Gly Lys Ile Pro Ile Arg Trp Thr Ala Pro Glu Ala Ile Gln Tyr
   785                 790                 795                 800
   Arg Lys Phe Thr Ser Ala Ser Asp Val Trp Ser Tyr Gly Ile Val Met
                   805                 810                 815
   Trp Glu Val Met Ser Tyr Gly Glu Arg Pro Tyr Trp Asp Met Thr Asn
               820                 825                 830
   Gln Asp Val Ile Asn Ala Ile Glu Gln Asp Tyr Arg Leu Pro Pro Pro
           835                 840                 845
   Met Asp Cys Pro Ser Ala Leu His Gln Leu Met Leu Asp Cys Trp Gln
       850                 855                 860
   Lys Asp Arg Asn His Arg Pro Lys Phe Gly Gln Ile Val Asn Thr Leu
   865                 870                 875                 880
   Asp Lys Met Ile Arg Asn Pro Asn Ser Leu Lys Ala Met Ala Pro Leu
                   885                 890                 895
   Ser Ser Gly Ile Asn Leu Pro Leu Leu Asp Arg Thr Ile Pro Asp Tyr
               900                 905                 910
   Thr Ser Phe Asn Thr Val Asp Glu Trp Leu Glu Ala Ile Lys Met Gly
           915                 920                 925
   Gln Tyr Lys Glu Ser Phe Ala Asn Ala Gly Phe Thr Ser Phe Asp Val
       930                 935                 940
   Val Ser Gln Met Met Met Glu Asp Ile Leu Arg Val Gly Val Thr Leu
   945                 950                 955                 960
   Ala Gly His Gln Lys Lys Ile Leu Asn Ser Ile Gln Val Met Arg Ala
                   965                 970                 975
   Gln Met Asn Gln Ile Gln Ser Val Glu Val
               980                 985
<210> 6
   <211> 986
   <212> PRT
   <213> Homo sapiens
<400> 6
   Met Ala Leu Arg Arg Leu Gly Ala Ala Leu Leu Leu Leu Pro Leu Leu
   1               5                   10                  15
   Ala Ala Val Glu Glu Thr Leu Met Asp Ser Thr Thr Ala Thr Ala Glu
               20                  25                  30
   Leu Gly Trp Met Val His Pro Pro Ser Gly Trp Glu Glu Val Ser Gly
           35                  40                  45
   Tyr Asp Glu Asn Met Asn Thr Ile Arg Thr Tyr Gln Val Cys Asn Val
       50                  55                  60
   Phe Glu Ser Ser Gln Asn Asn Trp Leu Arg Thr Lys Phe Ile Arg Arg
   65                  70                  75                  80
   Arg Gly Ala His Arg Ile His Val Glu Met Lys Phe Ser Val Arg Asp
                   85                  90                  95
   Cys Ser Ser Ile Pro Ser Val Pro Gly Ser Cys Lys Glu Thr Phe Asn
               100                 105                 110
   Leu Tyr Tyr Tyr Glu Ala Asp Phe Asp Ser Ala Thr Lys Thr Phe Pro
           115                 120                 125
   Asn Trp Met Glu Asn Pro Trp Val Lys Val Asp Thr Ile Ala Ala Asp
       130                 135                 140
   Glu Ser Phe Ser Gln Val Asp Leu Gly Gly Arg Val Met Lys Ile Asn
   145                 150                 155                 160
   Thr Glu Val Arg Ser Phe Gly Pro Val Ser Arg Ser Gly Phe Tyr Leu
                   165                 170                 175
   Ala Phe Gln Asp Tyr Gly Gly Cys Met Ser Leu Ile Ala Val Arg Val
               180                 185                 190
   Phe Tyr Arg Lys Cys Pro Arg Ile Ile Gln Asn Gly Ala Ile Phe Gln
           195                 200                 205
   Glu Thr Leu Ser Gly Ala Glu Ser Thr Ser Leu Val Ala Ala Arg Gly
       210                 215                 220
   Ser Cys Ile Ala Asn Ala Glu Glu Val Asp Val Pro Ile Lys Leu Tyr
   225                 230                 235                 240
   Cys Asn Gly Asp Gly Glu Trp Leu Val Pro Ile Gly Arg Cys Met Cys
                   245                 250                 255
   Lys Ala Gly Phe Glu Ala Val Glu Asn Gly Thr Val Cys Arg Gly Cys
               260                 265                 270
   Pro Ser Gly Thr Phe Lys Ala Asn Gln Gly Asp Glu Ala Cys Thr His
           275                 280                 285
   Cys Pro Ile Asn Ser Arg Thr Thr Ser Glu Gly Ala Thr Asn Cys Val
       290                 295                 300
   Cys Arg Asn Gly Tyr Tyr Arg Ala Asp Leu Asp Pro Leu Asp Met Pro
   305                 310                 315                 320
   Cys Thr Thr Ile Pro Ser Ala Pro Gln Ala Val Ile Ser Ser Val Asn
                   325                 330                 335
   Glu Thr Ser Leu Met Leu Glu Trp Thr Pro Pro Arg Asp Ser Gly Gly
               340                 345                 350
   Arg Glu Asp Leu Val Tyr Asn Ile Ile Cys Lys Ser Cys Gly Ser Gly
           355                 360                 365
   Arg Gly Ala Cys Thr Arg Cys Gly Asp Asn Val Gln Tyr Ala Pro Arg
       370                 375                 380
   Gln Leu Gly Leu Thr Glu Pro Arg Ile Tyr Ile Ser Asp Leu Leu Ala
   385                 390                 395                 400
   His Thr Gln Tyr Thr Phe Glu Ile Gln Ala Val Asn Gly Val Thr Asp
                   405                 410                 415
   Gln Ser Pro Phe Ser Pro Gln Phe Ala Ser Val Asn Ile Thr Thr Asn
               420                 425                 430
   Gln Ala Ala Pro Ser Ala Val Ser Ile Met His Gln Val Ser Arg Thr
   435 440 445
   Val Asp Ser Ile Thr Leu Ser Trp Ser Gln Pro Asp Gln Pro Asn Gly
   450 455 460
   Val Ile Leu Asp Tyr Glu Leu Gln Tyr Tyr Glu Lys Glu Leu Ser Glu
   465 470 475 480
   Tyr Asn Ala Thr Ala Ile Lys Ser Pro Thr Asn Thr Val Thr Val Gln
   485 490 495
   Gly Leu Lys Ala Gly Ala Ile Tyr Val Phe Gln Val Arg Ala Arg Thr
   500 505 510
   Val Ala Gly Tyr Gly Arg Tyr Ser Gly Lys Met Tyr Phe Gln Thr Met
   515 520 525
   Thr Glu Ala Glu Tyr Gln Thr Ser Ile Gln Glu Lys Leu Pro Leu Ile
   530 535 540
   Ile Gly Ser Ser Ala Ala Gly Leu Val Phe Leu Ile Ala Val Val Val
   545 550 555 560
   Ile Ala Ile Val Cys Asn Arg Arg Gly Phe Glu Arg Ala Asp Ser Glu
   565 570 575
   Tyr Thr Asp Lys Leu Gln His Tyr Thr Ser Gly His Met Thr Pro Gly
   580 585 590
   Met Lys Ile Tyr Ile Asp Pro Phe Thr Tyr Glu Asp Pro Asn Glu Ala
   595 600 605
   Val Arg Glu Phe Ala Lys Glu Ile Asp Ile Ser Cys Val Lys Ile Glu
   610 615 620
   Gln Val Ile Gly Ala Gly Glu Phe Gly Glu Val Cys Ser Gly His Leu
   625 630 635 640
   Lys Leu Pro Gly Lys Arg Glu Ile Phe Val Ala Ile Lys Thr Leu Lys
   645 650 655
   Ser Gly Tyr Thr Glu Lys Gln Arg Arg Asp Phe Leu Ser Glu Ala Ser
   660 665 670
   Ile Met Gly Gln Phe Asp His Pro Asn Val Ile His Leu Glu Gly Val
   675 680 685
   Val Thr Lys Ser Thr Pro Val Met Ile Ile Thr Glu Phe Met Glu Asn
       690                 695                 700
   Gly Ser Leu Asp Ser Phe Leu Arg Gln Asn Asp Gly Gln Phe Thr Val
   705                 710                 715                 720
   Ile Gln Leu Val Gly Met Leu Arg Gly Ile Ala Ala Gly Met Lys Tyr
                   725                 730                 735
   Leu Ala Asp Met Asn Tyr Val His Arg Asp Leu Ala Ala Arg Asn Ile
               740                 745                 750
   Leu Val Asn Ser Asn Leu Val Cys Lys Val Ser Asp Phe Gly Leu Ser
           755                 760                 765
   Arg Phe Leu Glu Asp Asp Thr Ser Asp Pro Thr Tyr Thr Ser Ala Leu
       770                 775                 780
   Gly Gly Lys Ile Pro Ile Arg Trp Thr Ala Pro Glu Ala Ile Gln Tyr
   785                 790                 795                 800
   Arg Lys Phe Thr Ser Ala Ser Asp Val Trp Ser Tyr Gly Ile Val Met
                   805                 810                 815
   Trp Glu Val Met Ser Tyr Gly Glu Arg Pro Tyr Trp Asp Met Thr Asn
               820                 825                 830
   Gln Asp Val Ile Asn Ala Ile Glu Gln Asp Tyr Arg Leu Pro Pro Pro
           835                 840                 845
   Met Asp Cys Pro Ser Ala Leu His Gln Leu Met Leu Asp Cys Trp Gln
       850                 855                 860
   Lys Asp Arg Asn His Arg Pro Lys Phe Gly Gln Ile Val Asn Thr Leu
   865                 870                 875                 880
   Asp Lys Met Ile Arg Asn Pro Asn Ser Leu Lys Ala Met Ala Pro Leu
                   885                 890                 895
   Ser Ser Gly Ile Asn Leu Pro Leu Leu Asp Arg Thr Ile Pro Asp Tyr
               900                 905                 910
   Thr Ser Phe Asn Thr Val Asp Glu Trp Leu Glu Ala Ile Lys Met Gly
           915                 920                 925
   Gln Tyr Lys Glu Ser Phe Ala Asn Ala Gly Phe Thr Ser Phe Asp Val
   930 935 940
   Val Ser Gln Met Met Met Glu Asp Ile Leu Arg Val Gly Val Thr Leu
   945 950 955 960
   Ala Gly His Gln Lys Lys Ile Leu Asn Ser Ile Gln Val Met Arg Ala
   965 970 975
   Gln Met Asn Gln Ile Gln Ser Val Glu Val
   980 985
<210> 7
   <211> 987
   <212> PRT
   <213> Homo sapiens
<400> 7
   Met Ala Leu Arg Arg Leu Gly Ala Ala Leu Leu Leu Leu Pro Leu Leu
   1               5                   10                  15
   Ala Ala Val Glu Glu Thr Leu Met Asp Ser Thr Thr Ala Thr Ala Glu
               20                  25                  30
   Leu Gly Trp Met Val His Pro Pro Ser Gly Trp Glu Glu Val Ser Gly
           35                  40                  45
   Tyr Asp Glu Asn Met Asn Thr Ile Arg Thr Tyr Gln Val Cys Asn Val
       50                  55                  60
   Phe Glu Ser Ser Gln Asn Asn Trp Leu Arg Thr Lys Phe Ile Arg Arg
   65                  70                  75                  80
   Arg Gly Ala His Arg Ile His Val Glu Met Lys Phe Ser Val Arg Asp
                   85                  90                  95
   Cys Ser Ser Ile Pro Ser Val Pro Gly Ser Cys Lys Glu Thr Phe Asn
               100                 105                 110
   Leu Tyr Tyr Tyr Glu Ala Asp Phe Asp Ser Ala Thr Lys Thr Phe Pro
           115                 120                 125
   Asn Trp Met Glu Asn Pro Trp Val Lys Val Asp Thr Ile Ala Ala Asp
       130                 135                 140
   Glu Ser Phe Ser Gln Val Asp Leu Gly Gly Arg Val Met Lys Ile Asn
   145                 150                 155                 160
   Thr Glu Val Arg Ser Phe Gly Pro Val Ser Arg Ser Gly Phe Tyr Leu
                   165                 170                 175
   Ala Phe Gln Asp Tyr Gly Gly Cys Met Ser Leu Ile Ala Val Arg Val
               180                 185                 190
   Phe Tyr Arg Lys Cys Pro Arg Ile Ile Gln Asn Gly Ala Ile Phe Gln
           195                 200                 205
   Glu Thr Leu Ser Gly Ala Glu Ser Thr Ser Leu Val Ala Ala Arg Gly
       210                 215                 220
   Ser Cys Ile Ala Asn Ala Glu Glu Val Asp Val Pro Ile Lys Leu Tyr
   225                 230                 235                 240
   Cys Asn Gly Asp Gly Glu Trp Leu Val Pro Ile Gly Arg Cys Met Cys
                   245                 250                 255
   Lys Ala Gly Phe Glu Ala Val Glu Asn Gly Thr Val Cys Arg Gly Cys
               260                 265                 270
   Pro Ser Gly Thr Phe Lys Ala Asn Gln Gly Asp Glu Ala Cys Thr His
           275                 280                 285
   Cys Pro Ile Asn Ser Arg Thr Thr Ser Glu Gly Ala Thr Asn Cys Val
       290                 295                 300
   Cys Arg Asn Gly Tyr Tyr Arg Ala Asp Leu Asp Pro Leu Asp Met Pro
   305                 310                 315                 320
   Cys Thr Thr Ile Pro Ser Ala Pro Gln Ala Val Ile Ser Ser Val Asn
                   325                 330                 335
   Glu Thr Ser Leu Met Leu Glu Trp Thr Pro Pro Arg Asp Ser Gly Gly
               340                 345                 350
   Arg Glu Asp Leu Val Tyr Asn Ile Ile Cys Lys Ser Cys Gly Ser Gly
           355                 360                 365
   Arg Gly Ala Cys Thr Arg Cys Gly Asp Asn Val Gln Tyr Ala Pro Arg
       370                 375                 380
   Gln Leu Gly Leu Thr Glu Pro Arg Ile Tyr Ile Ser Asp Leu Leu Ala
   385                 390                 395                 400
   His Thr Gln Tyr Thr Phe Glu Ile Gln Ala Val Asn Gly Val Thr Asp
                   405                 410                 415
   Gln Ser Pro Phe Ser Pro Gln Phe Ala Ser Val Asn Ile Thr Thr Asn
               420                 425                 430
   Gln Ala Ala Pro Ser Ala Val Ser Ile Met His Gln Val Ser Arg Thr
           435                 440                 445
   Val Asp Ser Ile Thr Leu Ser Trp Ser Gln Pro Asp Gln Pro Asn Gly
       450                 455                 460
   Val Ile Leu Asp Tyr Glu Leu Gln Tyr Tyr Glu Lys Glu Leu Ser Glu
   465                 470                 475                 480
   Tyr Asn Ala Thr Ala Ile Lys Ser Pro Thr Asn Thr Val Thr Val Gln
                   485                 490                 495
   Gly Leu Lys Ala Gly Ala Ile Tyr Val Phe Gln Val Arg Ala Arg Thr
               500                 505                 510
   Val Ala Gly Tyr Gly Arg Tyr Ser Gly Lys Met Tyr Phe Gln Thr Met
           515                 520                 525
   Thr Glu Ala Glu Tyr Gln Thr Ser Ile Gln Glu Lys Leu Pro Leu Ile
       530                 535                 540
   Ile Gly Ser Ser Ala Ala Gly Leu Val Phe Leu Ile Ala Val Val Val
   545                 550                 555                 560
   Ile Ala Ile Val Cys Asn Arg Arg Arg Gly Phe Glu Arg Ala Asp Ser
                   565                 570                 575
   Glu Tyr Thr Asp Lys Leu Gln His Tyr Thr Ser Gly His Met Thr Pro
               580                 585                 590
   Gly Met Lys Ile Tyr Ile Asp Pro Phe Thr Tyr Glu Asp Pro Asn Glu
           595                 600                 605
   Ala Val Arg Glu Phe Ala Lys Glu Ile Asp Ile Ser Cys Val Lys Ile
       610                 615                 620
   Glu Gln Val Ile Gly Ala Gly Glu Phe Gly Glu Val Cys Ser Gly His
   625                 630                 635                 640
   Leu Lys Leu Pro Gly Lys Arg Glu Ile Phe Val Ala Ile Lys Thr Leu
                   645                 650                 655
   Lys Ser Gly Tyr Thr Glu Lys Gln Arg Arg Asp Phe Leu Ser Glu Ala
               660                 665                 670
   Ser Ile Met Gly Gln Phe Asp His Pro Asn Val Ile His Leu Glu Gly
           675                 680                 685
   Val Val Thr Lys Ser Thr Pro Val Met Ile Ile Thr Glu Phe Met Glu
       690                 695                 700
   Asn Gly Ser Leu Asp Ser Phe Leu Arg Gln Asn Asp Gly Gln Phe Thr
   705                 710                 715                 720
   Val Ile Gln Leu Val Gly Met Leu Arg Gly Ile Ala Ala Gly Met Lys
                   725                 730                 735
   Tyr Leu Ala Asp Met Asn Tyr Val His Arg Asp Leu Ala Ala Arg Asn
               740                 745                 750
   Ile Leu Val Asn Ser Asn Leu Val Cys Lys Val Ser Asp Phe Gly Leu
           755                 760                 765
   Ser Arg Phe Leu Glu Asp Asp Thr Ser Asp Pro Thr Tyr Thr Ser Ala
       770                 775                 780
   Leu Gly Gly Lys Ile Pro Ile Arg Trp Thr Ala Pro Glu Ala Ile Gln
   785                 790                 795                 800
   Tyr Arg Lys Phe Thr Ser Ala Ser Asp Val Trp Ser Tyr Gly Ile Val
                   805                 810                 815
   Met Trp Glu Val Met Ser Tyr Gly Glu Arg Pro Tyr Trp Asp Met Thr
               820                 825                 830
   Asn Gln Asp Val Ile Asn Ala Ile Glu Gln Asp Tyr Arg Leu Pro Pro
           835                 840                 845
   Pro Met Asp Cys Pro Ser Ala Leu His Gln Leu Met Leu Asp Cys Trp
       850                 855                 860
   Gln Lys Asp Arg Asn His Arg Pro Lys Phe Gly Gln Ile Val Asn Thr
   865                 870                 875                 880
   Leu Asp Lys Met Ile Arg Asn Pro Asn Ser Leu Lys Ala Met Ala Pro
                   885                 890                 895
   Leu Ser Ser Gly Ile Asn Leu Pro Leu Leu Asp Arg Thr Ile Pro Asp
               900                 905                 910
   Tyr Thr Ser Phe Asn Thr Val Asp Glu Trp Leu Glu Ala Ile Lys Met
           915                 920                 925
   Gly Gln Tyr Lys Glu Ser Phe Ala Asn Ala Gly Phe Thr Ser Phe Asp
       930                 935                 940
   Val Val Ser Gln Met Met Met Glu Asp Ile Leu Arg Val Gly Val Thr
   945                 950                 955                 960
   Leu Ala Gly His Gln Lys Lys Ile Leu Asn Ser Ile Gln Val Met Arg
                   965                 970                 975
   Ala Gln Met Asn Gln Ile Gln Ser Val Glu Val
               980                 985
<210> 8
   <211> 1322
   <212> DNA
   <213> Mus musculus
<400> 8
   gacacaccga agggaagtcc gggggcctct tccaccgagt ccgagtgacc ccgccccttg      60
   cattctggaa ggtgaggcgc agaggtcccc agacacggac ctcaggcgca gggaggtccc     120
   ctttctctga gcccaggacc ctcccacccc caggctcaca ttctttctct caggatcttc     180
   aagcgggtct cttaagctcc ctcttcccca ggacgttgga gtcacagcct cagatctttc     240
   tctccaatct cacaagtggg ccagaactcc tttataatgt ctggatcccc aacagcaagc     300
   tctcccccac actaaaattc ggggatctag agctctgccc tagctttctc agcccctagc     360
   tccatcctcc agcaagactc aagacagctc cggaaacacc tccttccccc agttccccag     420
   acaacaagat ctcaggctcc tccctcggac ttcctcttag ttccaccctc ttcctcagag     480
   gccaccatgg gacgcccccc accctgtgca atccagccgt ggatccttct gcttctgttc     540
   atgggagcgt gggcagggct caccagagct cagggctcca agatcctgga aggtcgagag     600
   tgtatacccc actcccagcc ttggcaggca gccttgttcc agggcgagag actgatctgt     660
   gggggtgtcc tggttggaga cagatgggtc ctcacggcag cccactgcaa aaaacagaag     720
   tactccgtgc gtctgggtga tcatagcctc cagagcagag atcagccgga gcaggagatc     780
   caggtggctc agtctatcca gcatccttgc tacaacaaca gcaacccaga agatcacagt     840
   cacgatataa tgctcattcg actgcagaac tcagcaaacc tcggggacaa ggtgaagccg     900
   gtccaactgg ccaatctgtg tcccaaagtt ggccagaagt gcatcatatc aggctggggc     960
   actgtcacca gccctcaaga gaactttcca aacaccctca actgtgcgga agtgaaaatc    1020
   tattcccaga acaagtgtga gagagcctat ccagggaaga tcaccgaggg catggtctgt    1080
   gctggcagca gcaatggagc tgacacgtgc cagggtgact caggaggccc tctggtgtgc    1140
   gacgggatgc tccagggcat cacctcatgg ggctcagacc cctgtgggaa acccgagaaa    1200
   cctggagtct acaccaaaat ctgccgctac actacctgga tcaagaagac catggacaac    1260
   agggactgat cctggtgtgt gtgtgtgggg ggggttgtca ataaacacca ccattggctg    1320
   gc                                                                   1322
<210> 9
   <211> 1023
   <212> DNA
   <213> Homo sapiens
<400> 9
   gttcccagaa gctccccagg ctctagtgca ggaggagaag gaggaggagc aggaggtgga 60
   gattcccagt taaaaggctc cagaatcgtg taccaggcag agaactgaag tactggggcc 120
   tcctccactg ggtccgaatc agtaggtgac cccgcccctg gattctggaa gacctcacca 180
   tgggacgccc ccgacctcgt gcggccaaga cgtggatgtt cctgctcttg ctggggggag 240
   cctgggcagg acactccagg gcacaggagg acaaggtgct ggggggtcat gagtgccaac 300
   cccattcgca gccttggcag gcggccttgt tccagggcca gcaactactc tgtggcggtg 360
   tccttgtagg tggcaactgg gtccttacag ctgcccactg taaaaaaccg aaatacacag 420
   tacgcctggg agaccacagc ctacagaata aagatggccc agagcaagaa atacctgtgg 480
   ttcagtccat cccacacccc tgctacaaca gcagcgatgt ggaggaccac aaccatgatc 540
   tgatgcttct tcaactgcgt gaccaggcat ccctggggtc caaagtgaag cccatcagcc 600
   tggcagatca ttgcacccag cctggccaga agtgcaccgt ctcaggctgg ggcactgtca 660
   ccagtccccg agagaatttt cctgacactc tcaactgtgc agaagtaaaa atctttcccc 720
   agaagaagtg tgaggatgct tacccggggc agatcacaga tggcatggtc tgtgcaggca 780
   gcagcaaagg ggctgacacg tgccagggcg attctggagg ccccctggtg tgtgatggtg 840
   cactccaggg catcacatcc tggggctcag acccctgtgg gaggtccgac aaacctggcg 900
   tctataccaa catctgccgc tacctggact ggatcaagaa gatcataggc agcaagggct 960
   gattctagga taagcactag atctccctta ataaactcac aactctctgg ttcaaaaaaa 1020
   aaa 1023
<210> 10
   <211> 1158
   <212> DNA
   <213> Homo sapiens
<400> 10
   gttcccagaa gctccccagg ctctagtgca ggaggagaag gaggaggagc aggaggtgga 60
   gattcccagt taaaaggctc cagaatcgtg taccaggcag agaactgaag tactggggcc 120
   tcctccactg ggtccgaatc agtaggtgac cccgcccctg gattctggaa gacctcacca 180
   tgggacgccc ccgacctcgt gcggccaaga cgtggatgtt cctgctcttg ctggggggag 240
   cctgggcagc gtgtggaagc ctggacctcc tcactaagtt gtatgcggag aacttgccgt 300
   gtgtccattt gaacccacag tggccttccc agccctcgca ctgccccaga gggtggcgat 360
   ccaaccctct ccctcctgct gcaggacact ccagggcaca ggaggacaag gtgctggggg 420
   gtcatgagtg ccaaccccat tcgcagcctt ggcaggcggc cttgttccag ggccagcaac 480
   tactctgtgg cggtgtcctt gtaggtggca actgggtcct tacagctgcc cactgtaaaa 540
   aaccgaaata cacagtacgc ctgggagacc acagcctaca gaataaagat ggcccagagc 600
   aagaaatacc tgtggttcag tccatcccac acccctgcta caacagcagc gatgtggagg 660
   accacaacca tgatctgatg cttcttcaac tgcgtgacca ggcatccctg gggtccaaag 720
   tgaagcccat cagcctggca gatcattgca cccagcctgg ccagaagtgc accgtctcag 780
   gctggggcac tgtcaccagt ccccgagaga attttcctga cactctcaac tgtgcagaag 840
   taaaaatctt tccccagaag aagtgtgagg atgcttaccc ggggcagatc acagatggca 900
   tggtctgtgc aggcagcagc aaaggggctg acacgtgcca gggcgattct ggaggccccc 960
   tggtgtgtga tggtgcactc cagggcatca catcctgggg ctcagacccc tgtgggaggt 1020
   ccgacaaacc tggcgtctat accaacatct gccgctacct ggactggatc aagaagatca 1080
   taggcagcaa gggctgattc taggataagc actagatctc ccttaataaa ctcacaactc 1140
   tctggttcaa aaaaaaaa 1158
<210> 11
   <211> 600
   <212> DNA
   <213> Homo sapiens
<400> 11
   gttcccagaa gctccccagg ctctagtgca ggaggagaag gaggaggagc aggaggtgga 60
   gattcccagt taaaaggctc cagaatcgtg taccaggcag agaactgaag tactggggcc 120
   tcctccactg ggtccgaatc agtaggtgac cccgcccctg gattctggaa gacctcacca 180
   tgggacgccc ccgacctcgt gcggccaaga cgtggatgtt cctgctcttg ctggggggag 240
   cctgggcaga gaattttcct gacactctca actgtgcaga agtaaaaatc tttccccaga 300
   agaagtgtga ggatgcttac ccggggcaga tcacagatgg catggtctgt gcaggcagca 360
   gcaaaggggc tgacacgtgc cagggcgatt ctggaggccc cctggtgtgt gatggtgcac 420
   tccagggcat cacatcctgg ggctcagacc cctgtgggag gtccgacaaa cctggcgtct 480
   ataccaacat ctgccgctac ctggactgga tcaagaagat cataggcagc aagggctgat 540
   tctaggataa gcactagatc tcccttaata aactcacaac tctctggttc aaaaaaaaaa 600
<210> 12
   <211> 466
   <212> DNA
   <213> Homo sapiens
<400> 12
   gttcccagaa gctccccagg ctctagtgca ggaggagaag gaggaggagc aggaggtgga       60
   gattcccagt taaaaggctc cagaatcgtg taccaggcag agaactgaag tactggggcc      120
   tcctccactg ggtccgaatc agtaggtgac cccgcccctg gattctggaa gacctcacca      180
   tgggacgccc ccgacctcgt gcggccaaga cgtggatgtt cctgctcttg ctggggggag      240
   cctgggcagg gcgattctgg aggccccctg gtgtgtgatg gtgcactcca gggcatcaca      300
   tcctggggct cagacccctg tgggaggtcc gacaaacctg gcgtctatac caacatctgc      360
   cgctacctgg actggatcaa gaagatcata ggcagcaagg gctgattcta ggataagcac      420
   tagatctccc ttaataaact cacaactctc tggttcaaaa aaaaaa                      466
<210> 13
   <211> 863
   <212> DNA
   <213> Homo sapiens
<400> 13
   gttcccagaa gctccccagg ctctagtgca ggaggagaag gaggaggagc aggaggtgga       60
   gattcccagt taaaaggctc cagaatcgtg taccaggcag agaactgaag tactggggcc      120
   tcctccactg ggtccgaatc agtaggtgac cccgcccctg gattctggaa gacctcacca      180
   tgggacgccc ccgacctcgt gcggccaaga cgtggatgtt cctgctcttg ctggggggag      240
   cctgggcagg aaatacacag tacgcctggg agaccacagc ctacagaata aagatggccc      300
   agagcaagaa atacctgtgg ttcagtccat cccacacccc tgctacaaca gcagcgatgt      360
   ggaggaccac aaccatgatc tgatgcttct tcaactgcgt gaccaggcat ccctggggtc      420
   caaagtgaag cccatcagcc tggcagatca ttgcacccag cctggccaga agtgcaccgt      480
   ctcaggctgg ggcactgtca ccagtccccg agagaatttt cctgacactc tcaactgtgc      540
   agaagtaaaa atctttcccc agaagaagtg tgaggatgct tacccggggc agatcacaga      600
   tggcatggtc tgtgcaggca gcagcaaagg ggctgacacg tgccagggcg attctggagg      660
   ccccctggtg tgtgatggtg cactccaggg catcacatcc tggggctcag acccctgtgg      720
   gaggtccgac aaacctggcg tctataccaa catctgccgc tacctggact ggatcaagaa      780
   gatcataggc agcaagggct gattctagga taagcactag atctccctta ataaactcac      840
   aactctctgg ttcaaaaaaa aaa                                               863